# EUROPEAN PATENT SPECIFICATION

(11) **EP 1 060 191 B1**
(45) Date of publication and mention of the grant of the patent: **28.04.2010**
(21) Application number: 99906076.7
(22) Date of filing: 25.02.1999
(51) Int. Cl.: C07K 14/605, A61K 38/26

(54) **DERIVATIVES OF GLP-1 ANALOGS**
ABKÖMMLINGE VON GLP-1 ANALOGEN
DERIVES D'ANALOGUES DE GLP-1

(30) Priority: 27.02.1998 DK 26898
(43) Date of publication of application: 20.12.2000
(73) Proprietor: NOVO NORDISK A/S, 2880 Bagsværd (DK)
(72) Inventor: KNUDSEN, Liselotte, Bjerre, DK-2500 Valby (DK); HUUSFELDT, Per, Olaf, DK-2100 København Ø (DK); NIELSEN, Per, Franklin, DK-3500 Værløse (DK); PEDERSEN, Freddy, Zimmerdahl, DK-3500 Værløse (DK)
(74) Representative: Kjerrumgaard, Lars Bo
(86) International application number: PCT/DK1999/000082
(87) International publication number: WO 1999/043706

(56) References cited:
- EP-A2- 0 708 179
- WO-A1-87/06941
- WO-A1-90/11296
- WO-A1-95/31214
- WO-A1-96/29342
- WO-A1-98/08871
- US-A- 5 545 618
- US-A- 5 614 492
- US-A- 5 631 224

## Description

### FIELD OF THE INVENTION

The present invention relates to novel derivatives of human glucagon-like peptide-1 (GLP-1) and fragments thereof and analogues of such fragments which have a protracted profile of action and to methods of making and using them.

### BACKGROUND OF THE INVENTION

Peptides are widely used in medical practice, and since they can be produced by recombinant DNA technology it can be expected that their importance will increase also in the years to come. When native peptides or analogues thereof are used in therapy is generally found that they have a high clearance. A high clearance of a therapeutic agent is inconvenient in cases where it is desired to maintain a high blood level thereof over a prolonged period of time since repeated administrations will then be necessary. Examples of peptides which have a high clearance are: ACTH, corticotropin-releasing factor, angiotensin, calcitonin, insulin, glucagon, glucagon-like peptide-1, glucagon-like peptide-2, insulin-like growth factor-1, insulin-like growth factor-2, gastric inhibitory peptide, growth hormone-releasing factor, pituitary adenylate cyclase activating peptide, secretin, enterogastrin, somatostatin, somatotropin, somatomedin, parathyroid hormone, thrombopoietin, erythropoietin, hypothalamic releasing factors, prolactin, thyroid stimulating hormones, endorphins, enkephalins, vasopressin, oxytocin, opiods and analogues thereof, superoxide dismutase, interferon, asparaginase, arginase, arginine deaminase, adenosine deaminase and ribonuclease. In some cases it is possible to influence the release profile of peptides by applying suitable pharmaceutical compositions, but this approach has various shortcomings and is not generally applicable.

The hormones regulating insulin secretion belong to the so-called enteroinsular axis, designating a group of hormones, released from the gastrointestinal mucosa in response to the presence and absorption of nutrients in the gut, which promote an early and potentiated release of insulin. The enhancing effect on insulin secretion, the so-called incretin effect, is probably essential for a normal glucose tolerance. Many of the gastrointestinal hormones, including gastrin and secretin (cholecystokinin is not insulinotropic in man), are insulinotropic, but the only physiologically important ones, those that are responsible for the incretin effect, are the glucose-dependent insulinotropic polypeptide, GIP, and glucagon-like peptide-1 (GLP-1). Because of its insulinotropic effect, GIP, isolated in 1973 (1) immediately attracted considerable interest among diabetologists. However, numerous investigations carried out during the following years clearly indicated that a defective secretion of GIP was not involved in the pathogenesis of insulin dependent diabetes mellitus (IDDM) or non insulin-dependent diabetes mellitus (NIDDM) (2). Furthermore, as an insulinotropic hormone, GIP was found to be almost ineffective in NIDDM (2). The other incretin hormone, GLP-1 is the most potent insulinotropic substance known (3). Unlike GIP, it is surprisingly effective in stimulating insulin secretion in NIDDM patients. In addition, and in contrast to the other insulinotropic hormones (perhaps with the exception of secretin) it also potently inhibits glucagon secretion. Because of these actions it has pronounced blood glucose lowering effects particularly in patients with NIDDM.

GLP-1, a product of the proglucagon (4), is one of the youngest members of the secretin-VIP family of peptides, but is already established as an important gut hormone with regulatory function in glucose metabolism and gastrointestinal secretion and metabolism (5). The glucagon gene is processed differently in the pancreas and in the intestine. In the pancreas (9), the processing leads to the formation and parallel secretion of 1) glucagon itself, occupying positions 33-61 of proglucagon (PG); 2) an N-terminal peptide of 30 amino acids (PG (1-30)) often called glicentin-related pancreatic peptide, GRPP (10, 11); 3) a hexapeptide corresponding to PG (64-69); 4) and, finally, the so-called major proglucagon fragment (PG (72-158)), in which the two glucagon-like sequences are buried (9). Glucagon seems to be the only biologically active product. In contrast, in the intestinal mucosa, it is glucagon that is buried in a larger molecule, while the two glucagon-like peptides are formed separately (8). The following products are formed and secreted in parallel: 1) glicentin, corresponding to PG (1-69), with the glucagon sequence occupying residues Nos. 33-61 (12); 2) GLP-1(7-36)amide (PG (78-107))amide (13), not as originally believed PG (72-107)amide or 108, which is inactive). Small amounts of C-terminally glycine-extended but equally bioactive GLP-1 (7-37), (PG (78-108)) are also formed (14); 3) intervening peptide-2 (PG (111-122)amide) (15); and 4) GLP-2 (PG (126-158)) (15, 16). A fraction of glicentin is cleaved further into GRPP (PG (1-30)) and oxyntomodulin (PG (33-69)) (17, 18). Of these peptides, GLP-1, has the most conspicuous biological activities.

Being secreted in parallel with glicentin/enteroglucagon, it follows that the many studies of enteroglucagon secretion (6, 7) to some extent also apply to GLP-1 secretion, but GLP-1 is metabolised more quickly with a plasma half-life in humans of 2 min (19). Carbohydrate or fat-rich meals stimulate secretion (20), presumably as a result of direct interaction of yet unabsorbed nutrients with the microvilli of the open-type L-cells of the gut mucosa. Endocrine or neural mechanisms promoting GLP-1 secretion may exist but have not yet been demonstrated in humans.

The incretin function of GLP-1 (29-31) has been clearly illustrated in experiments with the GLP-1 receptor antagonist, exendin 9-39, which dramatically reduces the incretin effect elicited by oral glucose in rats (21, 22). The hormone interacts directly with the β-cells via the GLP-1 receptor (23) which belongs to the glucagon/VIP/calcitonin family of G-protein-coupled 7-transmembrane spanning receptors. The importance of the GLP-1 receptor in regulating insulin secretion was illustrated in recent experiments in which a targeted disruption of the GLP-1 receptor gene was carried out in mice. Animals homozygous for the disruption had greatly deteriorated glucose tolerance and fasting hyperglycaemia, and even heterozygous animals were glucose intolerant (24). The signal transduction mechanism (25) primarily involves activation of adenylate cyclase, but elevations of intracellular Ca²⁺ are also essential (25, 26). The action of the hormone is best described as a potentiation of glucose stimulated insulin release (25), but the mechanism that couples glucose and GLP-1 stimulation is not known. It may involve a calcium-induced calcium release (26, 27). As already mentioned, the insulinotropic action of GLP-1 is preserved in diabetic β-cells. The relation of the latter to its ability to convey "glucose competence" to isolated insulin-secreting cells (26, 28), which respond poorly to glucose or GLP-1 alone, but fully to a combination of the two, is also not known. Equally importantly, however, the hormone also potently inhibits glucagon secretion (29). The mechanism is not known, but seems to be paracrine, via neighbouring insulin or somatostatin cells (25). Also the glucagonostatic action is glucose-dependent, so that the inhibitory effect decreases as blood glucose decreases. Because of this dual effect, if the plasma GLP-1 concentrations increase either by increased secretion or by exogenous infusion the molar ratio of insulin to glucagon in the blood that reaches the liver via the portal circulation is greatly increased, whereby hepatic glucose production decreases (30). As a result blood glucose concentrations decrease. Because of the glucose dependency of the insulinotropic and glucagonostatic actions, the glucose lowering effect is self-limiting, and the hormone, therefore, does not cause hypoglycaemia regardless of dose (31). The effects are preserved in patients with diabetes mellitus (32), in whom infusions of slightly supraphysiological doses of GLP-1 may completely normalise blood glucose values in spite of poor metabolic control and secondary failure to sulphonylurea (33). The importance of the glucagonostatic effect is illustrated by the finding that GLP-1 also lowers blood glucose in type-1 diabetic patients without residual β-cell secretory capacity (34).

In addition to its effects on the pancreatic islets, GLP-1 has powerful actions on the gastrointestinal tract. Infused in physiological amounts, GLP-1 potently inhibits pentagastrin-induced as well as meal-induced gastric acid secretion (35, 36). It also inhibits gastric emptying rate and pancreatic enzyme secretion (36). Similar inhibitory effects on gastric and pancreatic secretion and motility may be elicited in humans upon perfusion of the ileum with carbohydrate- or lipid-containing solutions (37, 38). Concomitantly, GLP-1 secretion is greatly stimulated, and it has been speculated that GLP-1 may be at least partly responsible for this so-called "ileal-brake" effect (38). In fact, recent studies suggest that, physiologically, the ileal-brake effects of GLP-1 may be more important than its effects on the pancreatic islets. Thus, in dose response studies GLP-1 influences gastric emptying rate at infusion rates at least as low as those required to influence islet secretion (39).

GLP-1 seems to have an effect on food intake. Intraventricular administration of GLP-1 profoundly inhibits food intake in rats (40, 42). This effect seems to be highly specific. Thus, N-terminally extended GLP-1 (PG 72-107)amide is inactive and appropriate doses of the GLP-1 antagonist, exendin 9-39, abolish the effects of GLP-1 (41). Acute, peripheral administration of GLP-1 does not inhibit food intake acutely in rats (41, 42). However, it remains possible that GLP-1 secreted from the intestinal L-cells may also act as a satiety signal.

Not only the insulinotropic effects but also the effects of GLP-1 on the gastrointestinal tract are preserved in diabetic patients (43), and may help curtailing meal-induced glucose excursions, but, more importantly, may also influence food intake. Administered intravenously, continuously for one week, GLP-1 at 4 ng/kg/min has been demonstrated to dramatically improve glycaemic control in NIDDM patients without significant side effects (44). The peptide is fully active after subcutaneous administration (45), but is rapidly degraded mainly due to degradation by dipeptidyl peptidase IV-like enzymes (46, 47).

The amino acid sequence of GLP-1 is given *i.a.* by Schmidt et al. (Diabetologia 28 704-707 (1985). Human GLP-1 is a 37 amino acid residue peptide originating from preproglucagon which is synthesised, *i.a.* in the L-cells in the distal ileum, in the pancreas and in the brain. Processing of preproglucagon to GLP-1(7-36)amide, GLP-1(7-37) and GLP-2 occurs mainly in the L-cells. Although the interesting pharmacological properties of GLP-1 (7-37) and analogues thereof have attracted much attention in recent years only little is known about the structure of these molecules. The secondary structure of GLP-1 in micelles has been described by Thorton et al. (Biochemistry 33 3532-3539 (1994)), but in normal solution, GLP-1 is considered a very flexible molecule. Surprisingly, we found that derivatisation of this relatively small and very flexible molecule resulted in compounds whose plasma profile were highly protracted and still had retained activity.

GLP-1 and analogues of GLP-1 and fragments thereof are useful *i.a.* in the treatment of Type 1 and Type 2 diabetes and obesity.

WO 87/06941 discloses GLP-1 fragments, including GLP-1(7-37), and functional derivatives thereof and to their use as an insulinotropic agent.

WO. 90/11296 discloses GLP-1 fragments, including GLP-1 (7-36), and functional derivatives thereof which have an insulinotropic activity which exceeds the insulinotropic activity of GLP-1 (1-36) or GLP-1 (1-37) and to their use as insulinotropic agents.

The amino acid sequence of GLP-1 (7-36) and GLP-1 (7-37) is: 
7 8 9 10 11 12 13 14 15 16 17
His-Ala-Glu-Gly-Thr-Phe-Thr-Ser-Asp-Val-Ser-

18 19 20 21 22 23 24 25 26 27 28
Ser-Tyr-Leu-Glu-Gly-Gln-Ala-Ala-Lys-Glu-Phe-

29 30 31 32 33 34 35 36
Ile-Ala-Trp-Leu-Val-Lys-Gly-Arg-X
(I)
 wherein X is NH₂ for GLP-1 (7-36) and X is Gly for GLP-1 (7-37).

WO 91/11457 discloses analogues of the active GLP-1 peptides 7-34, 7-35, 7-36, and 7-37 which can also be useful as GLP-1 moieties.

WO 98/08871 (corresponding to EP 944648 which is prior art under Art. 54(3) EPC) discloses among others the following GLP-1-derivatives:
Glu^{22,23,30}Arg^{26,34}Lys³⁸(N^{ε}-(γ-glutamyl(N^{α}-tetradecanoyl)))-GLP-1(7-38)-OH,
Glu^{23,26}Arg³⁴Lys³⁸(N^{ε}-(γ-glutamyl(N^{α}-tetradecanoyl)))-GLP-1(7-38)-OH,
Glu^{23,34}Arg³⁴Lys³⁸(N^{ε}-(γ-glutamyl(N^{α}-tetradecanoyl)))-GLP-1(7-38)-OH,
Glu^{26,34}Arg³⁸Lys³⁸(N^{ε}-(γ-glutamyl(N^{α}-tetradecanoyl)))-GLP-1(7-38)-OH,
Glu^{26,34}Arg³⁴Lys³⁸(N^{ε}-(γ-glutamyl(N^{α}-tetradecanoyl)))-GLP-1(7-38)-OH.

Unfortunately, the high clearance limits the usefulness of these compounds. Thus there still is a need for improvements in this field. Accordingly, it is an object of the present invention to provide derivatives of GLP-1 and analogues thereof which have a protracted profile of action relative to GLP-1(7-37). It is a further object of the invention to provide derivatives of GLP-1 and analogues thereof which have a lower clearance than GLP-1(7-37). It is a further object of the invention to provide a pharmaceutical composition comprising a compound of the invention and to use a compound of the invention to provide such a composition. Also, it is an object of the present invention to provide a method of treating insulin dependent and non-insulin dependent diabetes mellitus.

### References.

1. Pederson RA. Gastric Inhibitory Polypeptide. In Walsh JH, Dockray GJ (eds) Gut peptides: Biochemistry and Physiology. Raven Press, New York 1994, pp. 217259.
2. Krarup T. Immunoreactive gastric inhibitory polypeptide. Endocr Rev 1988;9:122-134.
3. Ørskov C. Glucagon-like peptide-1, a new hormone of the enteroinsular axis. Diabetologia 1992; 35:701-711.
4. Bell Gl, Sanchez-Pescador R, Laybourn PJ, Najarian RC. Exon duplication and divergence in the human preproglucagon gene. Nature 1983; 304: 368-371.
5. Holst JJ. Glucagon-like peptide-1 (GLP-1) - a newly discovered Gl hormone. Gastroenterology 1994; 107: 1848-1855.
6. Holst JJ. Gut glucagon, enteroglucagon, gut GLI, glicentin - current status. Gastroenterology 1983;84:1602-1613.
7. Holst JJ, Ørskov C. Glucagon and other proglucagon-derived peptides. In Walsh JH, Dockray GJ, eds. Gut peptides: Biochemistry and Physiology. Raven Press, New York, pp. 305-340, 1993.
8. Ørskov C, Holst JJ, Knuhtsen S, Baldissera FGA, Poulsen SS, Nielsen OV. Glucagon-like peptides GLP-1 and GLP-2, predicted products of the glucagon gene, are secreted separately from the pig small intestine, but not pancreas. Endocrinology 1986;119:1467-1475.
9. Holst JJ, Bersani M, Johnsen AH, Kofod H, Hartmann B, Ørskov C. Proglucagon processing in porcine and human pancreas. J Biol Chem, 1994; 269: 18827-1883.
10. Moody AJ, Holst JJ, Thim L, Jensen SL. Relationship of glicentin to proglucagon and glucagon in the porcine pancreas. Nature 1981; 289: 514-516.
11. Thim L, Moody AJ, Purification and chemical characterisation of a glicentin-related pancreatic peptide (proglucagon fragment) from porcine pancreas. Biochim Biophys Acta 1982;703:134-141.
12. Thim L, Moody AJ. The primary structure of glicentin (proglucagon). Regul Pept 1981;2:139-151.
13. Ørskov C, Bersani M, Johnsen AH, Højrup P, Holst JJ. Complete sequences of glucagon-like peptide-1 (GLP-1) from human and pig small intestine. J. Biol. Chem. 1989;264:12826-12829.
14. Ørskov C, Rabenhøj L, Kofod H, Wettergren A, Holst JJ. Production and secretion of amidated and glycine-extended glucagon-like peptide-1 (GLP-1) in man. Diabetes 1991; 43: 535-539.
15. Buhl T, Thim L, Kofod H, Ørskov C, Harling H, & Holst JJ: Naturally occurring products of proglucagon 111-160 in the porcine and human small intestine. J. Biol. Chem. 1988;263:8621-8624.
16. Ørskov C, Buhl T, Rabenhøj L, Kofod H, Holst JJ: Carboxypeptidase-B-like processing of the C-terminus of glucagon-like peptide-2 in pig and human small intestine. FEBS letters, 1989;247:193-106.
17. Holst JJ. Evidence that enteroglucagon (II) is identical with the C-terminal sequence (residues 33-69) of glicentin. Biochem J. 1980;187:337-343.
18. Bataille D, Tatemoto K, Gespach C, Jörnvall H, Rosselin G, Mutt V. Isolation of glucagon-37 (bioactive enteroglucagon/oxyntomodulin) from porcine jejuno-ileum. Characterisation of the peptide. FEBS Lett 1982; 146:79-86.
19. Ørskov C, Wettergren A, Holst JJ. The metabolic rate and the biological effects of GLP-1 7-36amide and GLP-1 7-37 in healthy volunteers are identical. Diabetes 1993;42:658-661.
20. Elliott RM, Morgan LM, Tredger JA, Deacon S, Wright J, Marks V. Glucagon-like peptide-1 (7-36)amide and glucose-dependent insulinotropic polypeptide secretion in response to nutrient ingestion in man: acute post-prandial and 24-h secretion patterns. J Endocrinol 1993; 138: 159-166.
21. Kolligs F, Fehmann HC, Göke R, Göke B. Reduction of the incretin effect in rats by the glucagon-like peptide-1 receptor antagonist exendin (9-39)amide. Diabetes 1995; 44: 16-19.
22. Wang Z, Wang RM, Owji AA, Smith DM, Ghatei M, Bloom SR. Glucagon-like peptide-1 is a physiological incretin in rat. J. Clin. Invest. 1995; 95: 417-421.
23. Thorens B. Expression cloning of the pancreatic b cell receptor for the gluco-incretin hormone glucagon-like peptide 1. Proc Natl Acad Sci 1992;89:8641-4645.
24. Scrocchi L, Auerbach AB, Joyner AL, Drucker DJ. Diabetes in mice with targeted disruption of the GLP-1 receptor gene. Diabetes 1996; 45: 21A.
25. Fehmann HC, Göke R, Göke B. Cell and molecular biology of the incretin hormones glucagon-like peptide-I (GLP-1) and glucose-dependent insulin releasing polypeptide (GIP). Endocrine Reviews, 1995; 16: 390-410.
26. Gromada J, Dissing S, Bokvist K, Renström E, Frøkjær-Jensen J, Wulff BS, Rorsman P. Glucagon-like peptide I increases cytoplasmic calcium in insulin-secreting bTC3-cells by enhancement of intracellular calcium mobilisation. Diabetes 1995; 44: 767-774.
27. Holz GG, Leech CA, Habener JF. Activation of a cAMP-regulated Ca2+-signaling pathway in pancreatic β-cells by the insulinotropic hormone glucagon-like peptide-1. J Biol Chem, 1996; 270: 17749-17759.
28. Holz GG, Kühltreiber WM, Habener JF. Pancreatic beta-cells are rendered glucose competent by the insulinotropic hormone glucagon-like peptide-1(7-37). Nature 1993;361:362-365.
29. Ørskov C, Holst JJ, Nielsen OV: Effect of truncated glucagon-like peptide-1 (proglucagon 78-107 amide) on endocrine secretion from pig pancreas, antrum and stomach. Endocrinology 1988;123:2009-2013.
30. Hvidberg A, Toft Nielsen M, Hilsted J, Øsrskov C, Holst JJ. Effect of glucagon-like peptide-1 (proglucagon 78-107amide) on hepatic glucose production in healthy man. Metabolism 1994;43:104-108.
31. Qualmann C, Nauck M, Holst JJ, Ørskov C, Creutzfeldt W. Insulinotropic actions of intravenous glucagon-like peptide-1 [7-36 amide] in the fasting state in healthy subjects. Acta Diabetologica, 1995; 32: 13-16.
32. Nauck MA, Heimesaat MM, Ørskov C, Holst JJ, Ebert R, Creutzfeldt W. Preserved incretin activity of GLP-1(7-36amide) but not of synthetic human GIP in patients with type 2-diabetes mellitus. J Clin Invest 1993;91:301-307.
33. Nauck MA, Kleine N, Ørskov C, Holst JJ, Willms B, Creutzfeldt W. Normalisation of fasting hyperglycaemia by exogenous GLP-1 (7-36amide) in type 2-diabetic patients. Diabetologia 1993;36:741-744.
34. Creutzfeldt W, Kleine N, Willms B, Ørskov C, Holst JJ, Nauck MA. Glucagonostatic actions and reduction of fasting hyperglycaemia by exogenous glucagon-liem, peptide-1 (7-36amide) in type I diabetic patients. Diabetes Care 1996; 19: 580-586.
35. Schjoldager BTG, Mortensen PE, Christiansen J, Ørskov C, Holst JJ. GLP-1 (glucagon-like peptide-1) and truncated GLP-1, fragments of human proglucagon, inhibit gastric acid secretion in man. Dig. Dis. Sci. 1989; 35:703-708.
36. Wettergren A, Schjoldager B, Mortensen PE, Myhre J, Christiansen J, Holst JJ. Truncated GLP-1 (proglucagon 72-107amide) inhibits gastric and pancreatic functions in man. Dig Dis Sci 1993;38:665-673.
37. Layer P, Holst JJ, Grandt D, Goebell H: Ileal release of glucagon-like peptide-1 (GLP-1): association with inhibition of gastric acid in humans. Dig Dis Sci 1995; 40: 1074-1082.
38. Layer P, Holst JJ. GLP-1: A humoral mediator of the ileal brake in humans? Digestion 1993; 54: 385-386.
39. Nauck M, Ettler R, Niedereichholz U, Ørskov C, Holst JJ, Schmiegel W. Inhibition of gastric emptying by GLP-1 (7-36 amide) or (7-37): effects on postprandial glycaemia and insulin secretion. Abstract. Gut 1995; 37 (suppl. 2): A124.
40. Schick RR, vorm Walde T, Zimmermann JP, Schusdziarra V, Classen M. Glucagon-like peptide 1 - a novel brain peptide involved in feeding regulation. in Ditschuneit H, Gries FA, Hauner H, Schusdziarra V, Wechsler JG (eds.) Obesity in Europe. John Libbey & Company ltd, 1994; pp. 363-367.
41. Tang-Christensen M, Larsen PJ, Göke R, Fink-Jensen A, Jessop DS, Møller M, Sheikh S. Brain GLP-1 (7-36) amide receptors play a major role in regulation of food and water intake. Am. J. Physiol., 1996, in press.
42. Turton MD, O'Shea D, Gunn I, Beak SA, Edwards CMB, Meeran K, et al. A role for glucagon-like peptide-1 in the regulation of feeding. Nature 1996; 379: 69-72.
43. Willms B, Werner J, Creutzfeldt W, Ørskov C, Holst JJ, Nauck M. Inhibition of gastric emptying by glucagon-like peptide-1 (7-36 amide) in patients with type-2-diabetes mellitus. Diabetologia 1994; 37, suppl.1: A118.
44. Larsen J, Jallad N, Damsbo P. One-week continuous infusion of GLP-1 (7-37) improves glycaemic control in NIDDM. Diabetes 1996; 45, suppl. 2: 233A.
45. Ritzel R, Ørskov C, Holst JJ, Nauck MA. Pharmacokinetic, insulinotropic, and glucagonostatic properties of GLP-1 [7-36 amide] after subcutaneous injection in healthy volunteers. Dose-response relationships. Diabetologia 1995; 38: 720-725.
46. Deacon CF, Johnsen AH, Holst JJ. Degradation of glucagon-like peptide-1 by human plasma in vitro yields an N-terminally truncated peptide that is a major endogenous metabolite in vivo. J Clin Endocrinol Metab 1995; 80: 952-957.
47. Deacon CF, Nauck MA, Toft-Nielsen M, Pridal L, Willms B, Holst JJ. 1995. Both subcutaneous and intravenously administered glucagon-like peptide-1 are rapidly degraded from the amino terminus in type II diabetic patients and in healthy subjects. Diabetes 44: 1126-1131.

### SUMMARY OF THE INVENTION

The present invention relates to derivatives of a GLP-1 analog, which derivative has a protracted profile of action relative to GLP-1 (7-37), which GLP-1 analog is of formula I: 
 7   8   9  10  11  12  13  14  15  16  17
His-Xaa-Xaa-Gly-Xaa-Phe-Thr-Xaa-Asp-Xaa-Xaa-

18  19  20  21  22  23  24  25  26  27  28
Xaa-Xaa-Xaa-Xaa-Xaa-Xaa-Xaa-Xaa-Xaa-Xaa-Phe-

29  30  31  32  33  34  35  36  37  38
Ile-Xaa-Xaa-Xaa-Xaa-Xaa-Xaa-Xaa-Xaa-Xaa

39  40  41  42  43  44  45
Xaa-Xaa-Xaa-Xaa-Xaa-Xaa-Xaa
 wherein
Xaa at position 8 is Ala, Gly, Ser, Thr, Leu, Ile, Val, Glu or Asp,
Xaa at position 9 is Glu or Asp,
Xaa at position 11 is Thr, Ala, Gly, Ser, Leu, Ile, Val, Glu or Asp,
Xaa at position 14 is Ser, Ala, Gly, Thr, Leu, Ile, Val, Glu or Asp,
Xaa at position 16 is Val, Ala, Gly, Ser, Thr, Leu, Ile, Tyr, Glu or Asp,
Xaa at position 17 is Ser, Ala, Gly, Thr, Leu, Ile, Val, Glu or Asp,
Xaa at position 18 is Ser, Ala, Gly, Thr, Leu, Ile, Val, Glu or Asp,
Xaa at position 19 is Tyr, Phe, Trp, Glu or Asp,
Xaa at position 20 is Leu, Ala, Gly, Ser, Thr, Leu, Ile, Val, Glu or Asp,
Xaa at position 21 is Glu or Asp,
Xaa at position 22 is Gly, Ala, Ser, Thr, Leu, Ile, Val, Glu or Asp,
Xaa at position 23 is Gln, Asn, Arg, Glu or Asp,
Xaa at position 24 is Ala, Gly, Ser, Thr, Leu, Ile, Val, Arg, Glu or Asp,
Xaa at position 25 is Ala, Gly, Ser, Thr, Leu, Ile, Val, Glu or Asp,
Xaa at position 26 is Arg, Gln, Glu, Asp, or His,
Xaa at position 27 is Glu or Asp,
Xaa at position 30 is Ala, Gly, Ser, Thr, Leu, Ile, Val, Glu or Asp,
Xaa at position 31 is Trp, Phe, Tyr, Glu or Asp,
Xaa at position 32 is Leu, Gly, Ala, Ser, Thr, Ile, Val, Glu or Asp,
Xaa at position 33 is Val, Gly, Ala, Ser, Thr, Leu, Ile, Glu or Asp,
Xaa at position 34 is Arg, Glu, Asp, or His,
Xaa at position 35 is Gly, Ala, Ser, Thr, Leu, Ile, Val, Glu or Asp,
Xaa at position 36 is Arg, Lys, Glu, Asp, or His,
Xaa at position 37 is Gly, Ala, Ser, Thr, Leu, Ile, Val, Glu, Asp, or Lys, or is deleted,
Xaa at position 38 is Arg, Lys, Glu, Asp, or His, or is deleted,
Xaa at position 39 is Arg, Lys, Glu, Asp, or His, or is deleted,
Xaa at position 40 is Asp, Glu, or Lys, or is deleted,
Xaa at position 41 is Phe, Trp, Tyr, Glu, Asp, or Lys, or is deleted,
Xaa at position 42 is Pro, Lys, Glu, or Asp, or is deleted,
Xaa at position 43 is Glu, Asp, or Lys, or is deleted,
Xaa at position 44 is Glu, Asp, or Lys, or is deleted, and
Xaa at position 45 is Val, Glu, Asp, or Lys, or is deleted, or
(a) a C-1-6-ester thereof, (b) amide, C-1-6-alkylamide, or C-1-6-dialkylamide thereof and/or (c) a pharmaceutically acceptable salt thereof,
provided that
A. when the amino acid at position 37, 38, 39, 40, 41, 42, 43 or 44 is deleted, then each amino acid downstream of the amino acid is also deleted,
B. the derivative of the GLP-1 analog contains only one Lys at the carboxy terminus, and Glu or Asp is adjacent to Lys,
C. the ε-amino group of Lys is substituted with a lipophilic substituent via a spacer,
D. the total number of different amino acids between the derivative of the GLP-1 analog and the corresponding native form of GLP-1 does not exceed six,
E. the derivative of GLP-1 analog of formula I is not selected from:
   Glu^{22,23,30}Arg^{26,34}Lys³⁸(N^{ε}-(γ-glutamyl(N^{α}-tetradecanoyl)))-GLP-1(7-38)-OH,
   Glu^{23,26}Arg³⁴Lys³⁸(N^{ε}-(γ-glutamyl(N^{α}-tetradecanoyl)))-GLP-1(7-38)-OH,
   Glu^{26,34}Lys³⁸(N^{ε}-(γ-glutamyl(N^{α}-tetradecanoyl)))-GLP-1(7-38)-OH,
   Glu^{26,34}Lγs³⁸(N^{ε}-(γ-glutamyl(N^{α}-tetradecanoyl)))-GLP-1(7-38)-OH,
   Glu^{26,34}Lys³⁸(N^{ε}-(γ-glutamyl(N^{α}-tetradecanoyl)))-GLP-1(7-38)-OH.

### DETAILED DESCRIPTION OF THE INVENTION

A simple system is used to describe fragments and analogues of GLP-1. For example, Gly⁸-GLP-1(7-37) designates a fragment of GLP-1 formally derived from GLP-1 by deleting the amino acid residues Nos. 1 to 6 and substituting the naturally occurring amino acid residue in position 8 (Ala) by Gly. Similarly, Lys³⁴(N^{ε}-tetradecanoyl)-GLP-1(7-37) designates GLP-1(7-37) wherein the ε-amino group of the Lys residue in position 34 has been tetradecanoylated. Where reference in this text is made to C-terminally extended GLP-1 analogues, the amino acid residue in position 38 is Arg unless otherwise indicated, the optional amino acid residue in position 39 is also Arg unless otherwise indicated and the optional amino acid residue in position 40 is Asp unless otherwise indicated. Also, if a C-terminally extended analogue extends to position 41, 42, 43, 44 or 45, the amino acid sequence of this extension is as in the corresponding sequence in human preproglucagon unless otherwise indicated.

### GLP-1 Analogs

The present invention relates to derivatives of GLP-1 analogues. The derivatives of the invention have interesting pharmacological properties, in particular they have a more protracted profile of action than the parent peptides.

In the present text, the designation "an analogue" is used to designate a peptide wherein one or more amino acid residues of the parent peptide have been substituted by another amino acid residue.

The total number of different amino acids between the derivative of the GLP-1 analog and the corresponding native form of GLP-1 does not exceed six. Preferably, the number of different amino acids is five. More preferably, the number of different amino acids is four. Even more preferably, the number of different amino acids is three. Even more preferably, the number of different amino acids is two. Most preferably, the number of different amino acids is one. In order to determine the number of different amino acids, one should compare the amino acid sequence of the derivative of the GLP-1 analog of the present invention with the corresponding native GLP-1. For example, there are two different amino acids between the derivative Gly⁸Arg²⁶Lys³⁴(N^{ε}-(7-deoxycholoyl))-GLP-1(7-40) and the correspondiing native GLP-1 (i.e., GLP-1 (7-40)). The differences are located at positions 8 and 26. Similarly, there is only different amino acid between the derivative Lys²⁶(N^{ε}-(7-deoxycholoyl))Arg³⁴-GLP-1(7-40) and the corresponding native GLP-1. The difference is located at position 34.

The derivatives of the GLP-1 analogs of the present invention have only one Lys. The ε-amino group of Lys is substituted with a lipophilic substituent. The one Lys is located at the carboxy terminus of the derivative of the GLP-1 analogs. The derivatives of the GLP-1 analogs of the present invention have only one Lys and Glu or Asp is adjacent to Lys.

In a preferred embodiment, the amino acids at positions 37-45 are absent.

In another preferred embodiment, the amino acids at positions 38-45 are absent.

In another preferred embodiment, the amino acids at positions 39-45 are absent.

In another preferred embodiment, Xaa at position 8 is Ala, Gly, Ser, Thr, or Val.

In another preferred embodiment, Xaa at position 9 is Glu.

In another preferred embodiment, Xaa at position 11 is Thr.

In another preferred embodiment, Xaa at position 14 is Ser.

In another preferred embodiment, Xaa at position 16 is Val.

In another preferred embodiment, Xaa at position 17 is Ser.

In another preferred embodiment, Xaa at position 18 is Ser, Glu, or Asp.

In another preferred embodiment, Xaa at position 19 is Tyr, Glu, or Asp.

In another preferred embodiment, Xaa at position 20 is Leu, Glu, or Asp.

In another preferred embodiment, Xaa at position 21 is Glu, or Asp.

In another preferred embodiment, Xaa at position 22 is Gly, Glu, or Asp.

In another preferred embodiment, Xaa at position 23 is Gln, Glu, or Asp.

In another preferred embodiment, Xaa at position 24 is Ala, Glu, or Asp.

In another preferred embodiment, Xaa at position 25 is Ala, Glu, or Asp.

In another preferred embodiment, Xaa at position 26 is Glu, Asp, or Arg.

In another preferred embodiment, Xaa at position 27 is Glu, or Asp.

In another preferred embodiment, Xaa at position 30 is Ala, Glu, or Asp.

In another preferred embodiment, Xaa at position 31 is Trp, Glu, or Asp.

In another preferred embodiment, Xaa at position 32 is Leu, Glu, or Asp.

In another preferred embodiment, Xaa at position 33 is Val, Glu, or Asp.

In another preferred embodiment, Xaa at position 34 is Arg, Glu, or Asp.

In another preferred embodiment, Xaa at position 35 is Gly, Glu, or Asp.

In another preferred embodiment, Xaa at position 36 is Arg, Lys, Glu, or Asp.

In another preferred embodiment, Xaa at position 37 is Gly, Glu, Asp, or Lys.

In another preferred embodiment, Xaa at position 38 is Arg, or Lys, or is deleted.

In another preferred embodiment, Xaa at position 39 is deleted.

In another preferred embodiment, Xaa at position 40 is deleted.

In another preferred embodiment, Xaa at position 41 is deleted.

In another preferred embodiment, Xaa at position 42 is deleted.

In another preferred embodiment, Xaa at position 43 is deleted.

In another preferred embodiment, Xaa at position 44 is deleted.

In another preferred embodiment, Xaa at position 45 is deleted.

In another preferred embodiment, Xaa at position 26 is Arg, each of Xaa at positions 37-45 is deleted, and each of the other Xaa is the amino acid in native GLP-1 (7-36).

In another preferred embodiment, Xaa at position 26 is Arg, each of Xaa at positions 38-45 is deleted, and each of the other Xaa is the amino acid in native GLP-1 (7-37).

In another preferred embodiment, Xaa at position 26 is Arg, each of Xaa at positions 39-45 is deleted, and each of the other Xaa is the amino acid in native GLP-1 (7-38).

In another preferred embodiment, Xaa at position 34 is Arg, each of Xaa at positions 37-45 is deleted, and each of the other Xaa is the amino acid in native GLP-1 (7-36).

In another preferred embodiment, Xaa at position 34 is Arg, each of Xaa at positions 38-45 is deleted, and each of the other Xaa is the amino acid in native GLP-1 (7-37).

In another preferred embodiment, Xaa at position 34 is Arg, each of Xaa at positions 39-45 is deleted, and each of the other Xaa is the amino acid in native GLP-1 (7-38).

In another preferred embodiment, Xaa at positions 26 and 34 is Arg, Xaa at position 36 is Lys, each of Xaa at positions 37-45 is deleted, and each of the other Xaa is the amino acid in native GLP-1(7-36).

In another preferred embodiment, Xaa at positions 26 and 34 is Arg, Xaa at position 36 is Lys, each of Xaa at positions 38-45 is deleted, and each of the other Xaa is the amino acid in native GLP-1 (7-37).

In another preferred embodiment, Xaa at positions 26 and 34 is Arg, Xaa at position 36 is Lys, each of Xaa at positions 39-45 is deleted, and each of the other Xaa is the amino acid in native GLP-1(7-38).

In another preferred embodiment, Xaa at positions 26 and 34 is Arg, Xaa at position 38 is Lys, each of Xaa at positions 39-45 is deleted, and each of the other Xaa is the amino acid in native GLP-1 (7-38).

### Derivatives

The term "derivative" is defined as a modification of one or more amino acid residues of a peptide by chemical means, either with or without an enzyme, e.g., by alkylation, acylation, ester formation, or amide formation.

### Lipophilic Substituents

To obtain a satisfactory protracted profile of action of the GLP-1 derivative, the lipophilic substituent attached to the GLP-1 moiety preferably comprises 4-40 carbon atoms, in particular 8-25 carbon atoms. The lipophilic substituent may be attached to an amino group of the GLP-1 moiety by means of a carboxyl group of the lipophilic substituent which forms an amide bond with an amino group of the amino acid residue to which it is attached.

The lipophilic substituent is attached to the GLP-1 moiety by means of a spacer. The spacer is Glu or Asp, the carboxyl group of which may form an amide bond with an amino group of the amino acid residue, and the amino group thereof may form an amide bond with a carboxyl group of the lipophilic substituent. In another preferred embodiment Glu or Asp forms an amide bond with the ε-amino group of Lys and another amide bond with a carboxyl group present in the lipophilic substituent, that is, the lipophilic substituent is a N^{ε}-acylated lysine residue. Other preferred spacers are N^{ε}-(γ-L-glutamyl), and N^{ε}-(β-L-asparagyl).

In another preferred embodiment of the present invention, the lipophilic substituent has a group which can be negatively charged. One preferred group which can be negatively charged is a carboxylic acid group.

In a further preferred embodiment, the lipophilic substituent comprises from 4 to 40 carbon atoms, more preferred from 8 to 25 carbon atoms.

In a further preferred embodiment, the present invention relates to a GLP-1 derivative having a lipophilic substituent which comprises a partially or completely hydrogenated cyclopentanophenathrene skeleton.

In a further preferred embodiment, the present invention relates to a GLP-1 derivative having a lipophilic substituent which is a straight-chain or branched alkyl group.

In a further preferred embodiment, the present invention relates to a GLP-1 derivative having a lipophilic substituent which is the acyl group of a straight-chain or branched fatty acid.

In a further preferred embodiment, the present invention relates to a GLP-1 derivative having a lipophilic substituent which is an acyl group selected from the group comprising CH₃(CH₂)ₙCO-, wherein n is an integer from 4 to 38, preferably an integer from 4 to 24, more preferred selected from the group comprising CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.

In a further preferred embodiment, the present invention relates to a GLP-1 derivative having a lipophilic substituent which is an acyl group of a straight-chain or branched alkane α,ω-dicarboxylic acid.

In a further preferred embodiment, the present invention relates to a GLP-1 derivative having a lipophilic substituent which is an acyl group selected from the group comprising HOOC(CH₂)ₘCO-, wherein m is an integer from 4 to 38, preferably an integer from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

In a further preferred embodiment, the present invention relates to a GLP-1 derivative having a lipophilic substituent which is a group of the formula CH₃(CH₂)ₚ((CH₂)_{q}COOH)CHNH-CO(CH₂)₂CO-, wherein p and q are integers and p+q is an integer of from 8 to 33, preferably from 12 to 28.

In a further preferred embodiment, the present invention relates to a GLP-1 derivative having a lipophilic substituent which is a group of the formula CH₃(CH₂)ᵣCO-NHCH(COOH)(CH₂)₂CO-, wherein r is an integer of from 10 to 24.

In a further preferred embodiment, the present invention relates to a GLP-1 derivative having a lipophilic substituent which is a group of the formula CH₃(CH₂)ₛCO-NHCH((CH₂)₂COOH)CO-, wherein s is an integer of from 8 to 24.

In a further preferred embodiment, the present invention relates to a GLP-1 derivative having a lipophilic substituent which is a group of the formula COOH(CH₂)ₜCO- wherein t is an integer of from 8 to 24.

In a further preferred embodiment, the present invention relates to a GLP-1 derivative having a lipophilic substituent which is a group of the formula -NHCH(COOH)(CH₂)₄NH-CO(CH₂)ᵤCH₃, wherein u is an integer of from 8 to 18.

In a further preferred embodiment, the present invention relates to a GLP-1 derivative having a lipophilic substituent which is a group of the formula CH₃(CH₂)ᵥCO-NH-(CH₂)_{z}-CO,
wherein n is an integer of from 8 to 24 and z is an integer of from 1 to 6.

In a further preferred embodiment, the present invention relates to a GLP-1 derivative having a lipophilic substituent which is a group of the formula -NHCH(COOH)(CH₂)₄NH-COCH((CH₂)₂COOH)NH-CO(CH₂)_{w}CH₃, wherein w is an integer of from 10 to 16.

In a further preferred embodiment, the present invention relates to a GLP-1 derivative having a lipophilic substituent which is a group of the formula -NHCH(COOH)(CH₂)₄NH-CO(CH₂)₂CH(COOH)NH-CO(CH₂)ₓCH₃, wherein x is an integer of from 10 to 16.

In a further preferred embodiment, the present invention relates to a GLP-1 derivative having a lipophilic substituent which is a group of the formula -NHCH(COOH)(CH₂)₄NH-CO(CH₂)₂CH(COOH)NHCO(CH₂)_{y}CH₃, wherein y is zero or an integer of from 1 to 22.

In a further preferred embodiment, the present invention relates to a GLP-1 derivative having a lipophilic substituent which can be negatively charged. Such a lipophilic substituent can for example be a substituent which has a carboxyl group.

In a further preferred embodiment the present invention relates to a GLP-1 derivative of formula I provided that
the GLP-1 derivative of formula I is not selected from:
Glu^{22,23,30}Arg^{26,34}Lys³⁸(N^{ε}-(γ-glutamyl(N^{α}-tetradecanoyl)))-GLP-1(7-38)-OH,
Glu^{23,26}Arg³⁴Lys³⁸(N^{ε}-(γ-glutamyl(N^{α}-tetradecanoyl)))-GLP-1(7-38)-OH,
Arg^{26,34}Lys³⁸(N^{ε}-(γ-glutamyl(N^{α}-tetradecanoyl)))-GLP-1(7-38)-OH,
Arg^{26,34}Lys³⁸(N^{ε}-(γ-glutamyl(N^{α}-hexadecanoyl)))-GLP-1(7-38)-OH,
Arg^{26,34}Lys³⁸(N^{ε}-(γ-glutamyl(N^{α}-octadecanoyl)))-GLP-1(7-38)OH.

### Other Derivatives

The derivatives of GLP-1 analogues of the present invention may be in the form one or more of (a) a C-1-6-ester, (b) an amide, C-1-6-alkylamide, or C-1-6-dialkylamide, and (c) a pharmaceutical salt. In a preferred embodiment, the derivatives of GLP-1 analogues are in the form of an acid addition salt or a carboxylate salt, most preferably in the form of an acid addition salt.

### Preferred Derivatives of GLP-1 Analogues of the Present Invention

In a further preferred embodiment, a parent peptide for a derivative of the invention is Arg²⁶-GLP-1(7-37); Arg³⁴-GLP-1(7-37);Lys³⁶-GLP-1(7-37); Arg^{26,34}Lys³⁶-GLP-1(7-37); Arg^{26,34}Lys³⁶-GLP-1(7-38); Arg^{26,34}Lys³⁹-GLP-1(7-39); Arg^{26,34}Lys⁴⁰-GLP-1(7-40); Arg²⁶Lys³⁶-GLP-1(7-37); Arg³⁴Lys³⁶-GLP-1(7-37); Arg²⁶Lys³⁹-GLP-1 (7-39); Arg³⁴Lys⁴⁰-GLP-1(7-40); Arg^{26,34}Lys^{36,39}-GLP-1 (7-39); Arg^{26,34}Lys^{36,40}-GLP-1 (7-40); Gly⁸Arg²⁶-GLP-1(7-37); Gly⁸Arg³⁴-GLP-1(7-37);Glys³⁶-GLP-1(7-37); Gly⁸Arg^{26,34}Lys³⁶-GLP-1(7-37); Gly⁸Arg^{26,34}Lys³⁹-GLP-1(7-39);Gly⁸Arg^{26,34}Lys⁴⁰-GLP-1(7-40); Gly⁸Arg²⁶Lys³⁶-GLP-1(7-37); Gly⁸Arg³⁴Lys³⁶-GLP-1(7-37); Gly⁸Arg²⁶Lys³⁹-GLP-1(7-39); Gly⁸Arg³⁴Lys⁴⁰-GLP-1(7-40); Gly⁸Arg^{26,34}Lys^{36,39}-GLP-1(7-39); or Gly⁸Arg^{26,3}Lys^{36,40}-GLP-1 (7-40).

In a further preferred embodiment, a parent peptide for a derivative of the invention is:
Arg^{26,34}Lys³⁸GLP-1 (7-38);
Arg^{26,34}Lys³⁹GLP-1(7-39);
Arg^{26,34}Lys⁴⁰GLP-1(7-40);
Arg^{26,34}Lys⁴¹GLP-1(7-41);
Arg^{26,34}Lys⁴²GLP-1(7-42);
Arg^{26,3}Lys⁴³GLP-1(7-43);
Arg^{26,34}Lys⁴⁴GLP-1(7-44);
Arg^{26,34}Lys⁴⁵GLP-1(7-45);
Arg²⁶Lys³⁸GLP-1(7-38);
Arg³⁴Lys³⁸GLP-1(7-38);
Arg^{26,34}Lys^{36,38}GLP-1(7-38);
Arg^{26,34}-Lys³⁸GLP-1(7-38);
Arg²⁶Lys³⁹GLP-1(1-39);
Arg³⁴Lys³⁹GLP-1(1-39);
Arg^{26,34}Lys^{36,39}GLP-1(1-39);
Arg²⁶Lys³⁹GLP-1(7-39);
Arg³⁴Lys³⁹GLP-1(7-39);
Arg^{26,34}Lys^{36,39}GLP-1(7-39);

In a further preferred embodiment, the present invention relates to a GLP-1 derivative wherein the parent peptide is selected from the group comprising Arg²⁶-GLP-1(7-37), Arg³⁴-GLP-1 (7-37), Lys³⁶-GLP-1(7-37), Arg^{26,34}Lys³⁶-GLP-1 (7-37), Arg²⁶Lys³⁶-GLP-1(7-37), Arg³⁴Lys³⁶-GLP-1(7-37), Gly⁸Arg²⁶-GLP-1(7-37), Gly⁸Arg³⁴-GLP-1(7-37), Gly⁸Lys³⁶-GLP-1(7-37), Gly⁸Arg^{26,34}Lys³⁶-GLP-1(7-37), Gly⁸Arg²⁶Lys³⁶-GLP-1(7-37) and Gly⁸Arg³⁴Lys³⁶-GLP-1(7-37).

In a further preferred embodiment, the present invention relates to a GLP-1 derivative wherein the parent peptide is selected from the group comprising Arg²⁶Lys³⁸-GLP-1(7-38), Arg^{26,34}Lys³⁸-GLP-1(7-38), Arg^{26,34}Lys^{36,38}-GLP-1 (7-38), Gly⁸Arg²⁶Lys³⁸-GLP-1 (7-38) and Gly⁸Arg^{26,34}Lys^{36,38}-GLP-1(7-38).

In a further preferred embodiment, the present invention relates to a GLP-1 derivative wherein the parent peptide is selected from the group comprising Arg²⁶Lys³⁹-GLP-1(7-39), Arg^{26,34}Lys^{36,39}-GLP-1 (7-39), Gly⁸Arg²⁶Lys³⁹-GLP-1 (7-39) and Gly⁸Arg^{26,34}Lys^{36,39}-GLP-1 (7-39).

In a further preferred embodiment, the present invention relates to a GLP-1 derivative wherein the parent peptide is selected from the group comprising Arg³⁴Lys⁴⁰-GLP-1(7-40), Arg^{26,34}Lys^{36,40}-GLP-1 (7-40), Gly⁸Arg³⁴Lys⁴⁰-GLP-1 (7-40) and Gly⁸Arg^{26,34}Lys^{36,40}-GLP-1 (7-40).

In a further preferred embodiment, the present invention relates to a GLP-1 derivative wherein the parent peptide is:
Arg²⁶-GLP-1(7-36); Arg³⁴-GLP-1(7-36); Arg^{26,34}Lys³⁶-GLP-1(7-36); Arg²⁶-GLP-1(7-36)amide; Arg³⁴-GLP-1(7-36)amide; Arg^{26,34}Lys³⁶-GLP-1(7-36)amide; Arg²⁶-GLP-1(7-37); Arg³⁴-GLP-1(7-37); Arg^{26,34}Lys³⁶-GLP-1(7-37); Arg²⁶-GLP-1(7-38); Arg³⁴-GLP-1(7-38) ; Arg^{26,34}Lys³⁸GLP-1(7-38); Arg²⁶-GLP-1 (7-39); Arg³⁴-GLP-1 (7-39); Arg^{26,34}Lys³⁹-GLP-1 (7-39);
Gly⁸Arg²⁶-GLP-1(7-36); Gly⁸Arg³⁴-GLP-1(7-36); Gly⁸Arg^{26,34}Lys³⁶-GLP-1(7-36); Gly⁸Arg²⁶-GLP-1(7-36)amide; Gly⁸Arg³⁴-GLP-1(7-36)amide; Gly⁸Arg^{26,34}Lys³⁶-GLP-1(7-36)amide; Gly⁸Arg²⁶-GLP-1(7-37); Gly⁸Arg³⁴-GLP-1(7-37); Gly⁸Arg^{26,34}Lys³⁶-GLP-1(7-37); Gly⁸Arg²⁶-GLP-1(7-38);
Gly⁸Arg³⁴-GLP-1(7-38) ; Gly⁸Arg^{26,34}Lys³⁸GLP-1(7-38); Gly⁸Arg²⁶-GLP-1(7-39); Gly⁸Arg³⁴-GLP-1 (7-39); Gly⁸Arg^{26,34}Lys³⁹-GLP-1(7-39);
Val⁸Arg²⁶-GLP-1(7-36); Val⁸Arg³⁴-GLP-1(7-36); Val⁸Arg^{26,34}Lys³⁶-GLP-1(7-36); Val⁸Arg²⁶-GLP-1 (7-36)amide; Val⁸Arg³⁴-GLP-1(7-36)amide; Val⁸Arg^{26,34}Lys³⁶-GLP-1(7-36)amide; Val⁸Arg²⁶-GLP-1(7-37); Val⁸Arg³⁴-GLP-1(7-37); Val⁸Arg^{26,34}Lys³⁶-GLP-1(7-37); Val⁸Arg²⁶-GLP-1(7-38); Val⁸Arg³⁴-GLP-1(7-38); Val⁸Arg^{26,34}Lys³⁸GLP-1(7-38); Val⁸Arg²⁶-GLP-1(7-39); Val⁸Arg³⁴-GLP-1 (7-39); Val⁸Arg^{26,34}Lys³⁹-GLP-1 (7-39);
Ser⁸Arg²⁶-GLP-1(7-36); Ser⁸Arg³⁴-GLP-1(7-36); Ser⁸Arg^{26,34}Lys³⁶-GLP-1(7-36); Ser⁸Arg²⁶-GLP-1(7-36)amide; Ser⁸Arg³⁴-GLP-1(7-36)amide; Ser⁸Arg^{26,34}Lys³⁶-GLP-1(7-36)amide; Ser⁸Arg²⁶-GLP-1(7-37); Ser⁸Arg³⁴-GLP-1(7-37); Ser⁸Arg^{26,34}Lys³⁶-GLP-1(7-37); Ser⁸Arg²⁶-GLP-1(7-38); Ser⁸Arg³⁴-GLP-1(7-38); Ser⁸Arg^{26,34}Lys³⁸GLP-1(7-38); Ser⁸Arg²⁶-GLP-1(7-39); Ser⁸Arg³⁴-GLP-1 (7-39); Ser⁸Arg^{26,34}Lys³⁹-GLP-1 (7-39);
Thr⁸Arg²⁶-GLP-1(7-36); Thr⁸Arg³⁴-GLP-1(7-36); Thr⁸Arg^{26,34}Lys³⁶-GLP-1(7-36); Thr⁸Arg²⁶-GLP-1 (7-36)amide; Thr⁸Arg³⁴-GLP-1(7-36)amide; Thr⁸Arg^{26,34}Lys³⁶-GLP-1(7-36)amide; Thr⁸Arg²⁶-GLP-1(7-37); Thr⁸Arg³⁴-GLP-1(7-37); Thr⁸Arg^{26,34}Lys³⁶-GLP-1(7-37); Thr⁸Arg²⁶-GLP-1(7-38); Thr⁸Arg³⁴-GLP-1(7-38) ; Thr⁸Arg^{26,34}Lys³⁸GLP-1(7-38); Thr⁸Arg²⁶-GLP-1(7-39); Thr⁸Arg³⁴-GLP-1(7-39); TheArg^{26,34}Lys-³⁹-GLP- (7-39);
Val⁸Glu³⁵Arg^{26,34}Lys³⁶-GLP-1 (7-36); Val⁸Glu³⁵Arg^{26,34}Lys³⁶-GLP-1 (7-36)amide; Val⁸Glu³⁶Arg^{26,34}Lys³⁷GLP-1(7-37); Val⁸Glu³⁷Arg^{26,34}Lys-1(7-38); Val⁸Glu³⁸Arg^{26,34}Lys³⁹-GLP-1 (7-39); Val⁸Glu³⁵Arg^{26,34}Lys³⁶-GLP-1(7-36); Val⁸Glu³⁵Arg^{26,34}Lys³⁶-GLP-1(7-36)amide; Val⁸Glu³⁶Arg^{26,34}-Lys³⁷GLP-1(7-37); Val⁸Glu³⁷Arg^{26,34}Lys³⁸GLP-1(7-38); Val⁸Glu³⁸Arg^{26,34}Lys³⁹-GLP-1 (7-39);
Val⁸Asp³⁵Arg^{26,34}Lys³⁶-GLP-1(7-36); Val⁸Asp³⁵Arg^{26,34}Lys³⁶-GLP-1(7-36)amide; Val⁸Asp³⁶Arg^{26,34}Lys³⁷GLP-1(7-37); Val⁸Asp³⁷Arg^{26,34}Lys³⁸GLP-1(7-38); Val⁸Asp³⁸Arg^{26,34}Lys³⁹-GLP-1 (7-39); Val⁸Asp³⁵Arg^{26,34}Lys³⁶-GLP-1(7-36); Val⁸Asp³⁵Arg^{26,34} Lys³⁶-GLP-1(7-36)amide; Val⁸Asp³⁶Arg^{26,34}Lys³⁷GLP-1(7-37); Val⁸Asp³⁷Arg^{26,34} Lys³⁸GLP-1 (7-38); Val⁸Asp³⁸Arg^{26,34}Lys³⁹-GLP-1(7-39);
Ser⁸Glu³⁵Arg^{26,34}Lys³⁶-GLP-1(7-36); Ser⁸Glu³⁵Arg^{26,34}Lys³⁶-GLP-1(7-36)amide; Ser⁸Glu³⁶Arg^{26,34}Lys³⁷GLP-1(7-37); Ser⁸Glu³⁷Arg^{26,34}Lys³⁸GLP-1(7-38); Ser⁸Glu³⁸Arg^{26,34}Lys³⁹-GLP-1 (7-39); Ser⁸Glu³⁵Arg^{26,34}Lys-GLP-1(7-36); Ser⁸Glu³⁵Arg^{26,34}Lys³⁶-GLP-1(7-36)amide;
4Ser⁸Glu³⁶Arg^{26,34}Lys³⁷GLP-1(7-37); Ser⁸Glu³⁷Arg^{26,34}Lys³⁸GLP-1(7-38); Ser⁸Glu³⁸Arg^{26,34}Lys³⁹-GLP-1 (7-39);
Ser⁸Asp³⁵Arg^{26,34}Lys³⁶-GLP-1(7-36); Ser⁸Asp³⁵Arg^{26,34}Lys³⁶-GLP-1(7-36)amid; Ser⁸Asp³⁶Arg^{26,34}Lys³⁷GLP-1(7-37); Ser⁸Asp³⁷Arg^{26,34}Lys³⁸GLP-1(7-38); Ser⁸Asp³⁸Arg^{26,34}Lys³⁹-GLP-1 (7-39); Ser⁸Asp³⁵Arg^{26,34}Lys³⁶-GLP-1(7-36); Ser⁸Asp³⁵Arg^{26,34}Lys³⁶-GLP-1(7-36)amide; Ser⁸Asp³⁶Arg^{26,34}Lys³⁷GLP-1(7-37); Ser⁸Asp³⁷Arg^{26,34}Lys³⁸GLP-1(7-38); Ser⁸Asp³⁸Arg^{26,34}Lys³⁹-GLP-1(7-39);
Thr⁸GlU³⁵Arg^{26,34}Lys³⁶-GLP-1(7-36); Thr⁸Glu³⁵Arg^{26,34}Lys³⁶-GLP-1(7-36)amide; Thr⁸Glu³⁶Arg^{26,34}Lys³⁷GLP-1(7-37); Thr⁸Glu³⁷Arg^{26,34}Lys³⁸GLP-1(7-38); Thr⁸Glu³⁸Arg^{26,34}Lys³⁹-GLP-1 (7-39); Thr⁸Glu³⁵Arg^{26,34}Lys³⁶-GLP-1(7-36); Thr⁸Glu³⁵Arg^{26,34}Lys³⁶-GLP-1(7-36)amide; Thr⁸Glu³⁶Arg^{26,34}Lys³⁷GLP-1(7-37); Thr⁸Glu³⁷Arg^{26,34}Lys³⁸GLP-1(7-38); Thr⁸Glu³⁸Arg^{26,34}Lys³⁹-GLP-1(7-39);
Thr⁸Asp³⁵Arg^{26,34}Lys³⁶-GLP-1(7-36); Thr⁸Asp³⁵Arg^{26,34}Lys³⁶-GLP-1(7-36)amide; Thr⁸Asp³⁶Arg^{26,34}Lys³⁷GLP-1(7-37); Thr⁸Asp³⁷Arg^{26,34}Lys³⁸GLP-1(7-38); Thr⁸Asp³⁸Arg^{26,34}Lys³⁹-GLP-1 (7-39); Thr⁸Asp³⁵Arg^{26,34}Lys³⁶-GLP-1(7-36); Thr⁸Asp³⁵Arg^{26,34}Lys³⁶-GLP-1(7-36)amide; Thr⁸Asp³⁶Arg^{26,34}Lys³⁷GLP-1(7-37); Thr⁸Asp³⁷Arg^{26,34}Lys³⁸GLP-1(7-38); Thr⁸Asp³⁸Arg^{26,34}Lys³⁹-GLP-1(7-39);
Gly⁸Glu³⁵Arg^{26,34}Lys³⁶-GLP-1(7-36); Gly⁸Glu³⁵Arg^{26,34}Lys³⁶-GLP-1 (7-36)amide; Gly⁸Glu³⁶Arg^{26,34}Lys³⁷GLP-1(7-37); Gly⁸Glu³⁷Arg^{26,34}Lys³⁸GLP-1(7-38); Gly⁸Glu³⁸Arg^{26,34}Lys³⁹-GLP-1 (7-39); Gly⁸Glu³⁵Arg^{26,34}Lys³⁶-GLP-1(7-36); Gly⁸Glu³⁵Arg^{26,34}Lys³⁶-GLP-1(7-36)amide; Gly⁸Glu³⁶Arg^{26,34}Lys³⁷GLP-1(7-37); Gly⁸Glu³⁷Arg^{26,34}Lys³⁸GLP-1(7-38); Gly⁸Glu³⁸Arg^{26,34}Lys³⁹-GLP-1(7-39);
Gly⁸Asp³⁵Arg^{36,34}Lys³⁶-GLP -1(7-36); Gly⁸Asp³⁵Arg^{26,34}Lys³⁶-GLP-1 (7-36)amide; Gly⁸Asp³⁶Arg^{26,34}Lys³⁷GLP-1(7-37); Gly⁸Asp³⁷Arg^{26,34}Lys³⁸GLP-1(7-38); Gly⁸Asp³⁸Arg^{26,34}Lys³⁹-GLP-1 (7-39); Gly⁸Asp³⁵Arg^{26,34}Lys³⁶-GLP-1(7-36); Gly⁸Asp³⁵Arg^{26,34}Lys³⁶-GLP-1(7-36)amide; Gly⁸Asp³⁶Arg^{26,34}Lys³⁷GLP-1(7-37); Gly⁸Asp³⁷Arg^{26,34}Lys³⁸GLP-1(7-38); Gly⁸Asp³⁸Arg^{26,34}Lys³⁹-GLP-1(7-39);
Arg^{26,34}Lys¹⁸-GLP-1(7-36); Arg^{26,34}Lys¹⁸-GLP-1(7-36)amide; Arg^{26,34}Lys¹⁸GLP-1(7-37); Arg^{26,34}Lys¹⁸GLP-1(7-38); Gly⁸Asp¹⁹Arg^{26,34}Lys¹⁸-GLP-1(7-36); Gly⁸Asp¹⁷Arg^{26,34}Lys¹⁸-GLP-1(7-36); Gly⁸Asp¹⁹Arg^{26,34}Lys¹⁸-GLP-1(7-36)amide; Gly⁸Asp¹⁷Arg^{26,34}Lys¹⁸-GLP-1 (7-36)amide; Gly⁸Asp¹⁹Arg^{26,34}Lys¹⁸GLP-1(7-37); Gly⁸Asp¹⁹Arg^{26,34}Lys¹⁸GLP-1(7-38); Gly⁸Asp¹⁷Arg^{26,34}Lys¹⁸GLP-1(7-38);
Arg^{26,34}Lys²³-GLP-1 (7-36); Arg^{26,34}Lys²³-GLP-1(7-36)amide; Arg^{26,34}Lys²³GLP-1(7-37); Arg^{26,34}Lys²³GLP-1(7-38); Gly⁸Asp²⁴Arg^{26,34}Lys²³-GLP-1(7-36); Gly⁸Asp²²Arg^{26,34}Lys²³-GLP-1(7-36); Gly⁸Asp²⁴Arg^{26,34}Lys²³-GLP-1(7-36)amide; Gly⁸Asp²²Arg^{26,34}Lys²³-GLP-1(7-36)amide; Gly⁸Asp²⁴Arg^{26,34}Lys²³GLP-1(7-37); Gly⁸Asp²⁴Arg^{26,34}Lys²³GLP-1(7-38); Gly⁸Asp²²Arg^{26,34}Lys²³GLP-1(7-38);
Arg^{26,34}Lys²⁷-GLP-1(7-36); Arg^{26,34}Lys²⁷-GLP-1(7-36)amide; Arg^{26,34}Lys²⁷GLP-1(7-37); Arg^{26,34}Lys²⁷GLP-1(7-38); Gly⁸Asp²⁸Arg^{26,34}Lys²⁷-GLP-1(7-36); Gly⁸Asp²⁶Arg^{26,34}Lys²⁷-GLP-1 (7-36); Gly⁸Asp²⁸Arg^{26,34}Lys²⁷-GLP-1 (7-36)amide; Gly⁸Asp²⁶Arg^{26,34}Lys²⁷-GLP-1(7-36)amide; Gly⁸Asp²⁸Arg^{26,34}Lys²⁷GLP-1(7-37); Gly⁸Asp²⁸Arg^{26,34}Lys²⁷GLP-1(7-38); Gly⁸Asp²⁶Arg^{26,34}Lys²⁷GLP-1(7-38);
Arg^{26,34}Lys¹⁸-GLP-1(7-36); Arg^{26,34}Lys¹⁸-GLP-1 (7-36)amide; Arg^{26,34}Lys¹⁸GLP-1(7-37); Arg^{26,34}Lys¹⁸GLP-1(7-38); Val⁸Asp¹⁹Arg^{26,34}Lys¹⁸-GLP-1(7-36); Val⁸Asp¹⁷Arg^{26,34}Lys¹⁸-GLP-1 (7-36); Val⁸Asp¹⁹Arg^{26,34}Lys¹⁸-GLP-1(7-36)amide; Val⁸Asp¹⁷Arg^{26,34}Lys¹8-GLP-1(7-36)amide; Val⁸Asp¹⁹Arg^{26,34}Lys¹⁸GLP-1(7-37); Val⁸Asp¹⁹Arg^{26,34}Lys¹⁸GLP-1(7-38); Val⁸Asp¹⁷Ar^{26,34}Lys¹⁸GLP-1(7-38);
Arg^{26,34}Lys²³-GLP-1(7-36); Arg^{26,34}Lys²³-GLP-1 (7-36)amide; Arg^{26,34}Lys²³GLP-1(7-37); Arg^{26,34}Lys²³GLP-1 (7-38); Val⁸Asp²⁴Arg^{26,34}Lys²³-GLP-1 (7-36); Val⁸Asp²²Arg^{26,34}Lys²³-GLP-1 (7-36); Val⁸Asp²⁴Arg^{26,34}Lys²³-GLP-1(7-36)amide; Val⁸Asp²²Arg^{26,34}Lys^{26,34}-GLP-1(7-36)amide; Val⁸Asp²⁴Arg^{26,34}Lys²³GLP-1(7-37); Val⁸Asp²⁴Arg^{26,34}Lys²³GLP-1(7-38); Val⁸Asp²²Arg^{26,34}Lys²³GLP-1(7-38);
Arg^{26,34}Lys²⁷-GLP-1(7-36); Arg^{26,34}Lys²⁷-GLP-1 (7-36)amide; Arg^{26,34}Lys²⁷GLP-1(7-37); Arg^{26,34}Lys²⁷GLP-1(7-38); Val⁸Asp²⁸Arg^{26,34}Lys²⁷-GLP-1(7-36); Val⁸Asp²⁶Arg^{26,34}Lys²⁷-GLP-1 (7-36); Val⁸Asp²⁸Arg^{26,34}Lys²⁷-GLP-1(7-36)amide; Val⁸Asp²⁶Arg^{26,34}Lys²⁷-GLP-1(7-36)amide; Val⁸Asp²⁸Arg^{26,34}Lys²⁷GLP-1(7-37); Val⁸Asp²⁸Arg^{26,34}Lys²⁷GLP-1(7-38); Val⁸Asp²⁶Arg^{26,34}Lys²⁷GLP-1(7-38);
Arg^{26,34}Lys¹⁸-GLP-1 (7-36); Arg^{26,34}Lys¹⁸-GLP-1(7-36)amide; Arg^{26,34}Lys¹⁸GLP-1(7-37); Arg^{26,34}Lys¹⁸GLP-1 (7-38); Ser⁸Asp¹⁹Arg^{26,34}Lys¹⁸-GLP-1(7-36); Ser⁸Asp¹⁷Arg^{26,34}Lys¹⁸-GLP-1 (7-36); Ser⁸Asp¹⁹Arg^{26,34}Lys¹⁸-GLP-1(7-36)amide; Ser⁸Asp¹⁷Arg^{26,34}Lys¹⁸-GLP-1 (7-36)amide; Ser⁸Asp¹⁹Arg^{26,34}Lys¹⁸GLP-1(7-37); Ser⁸Asp¹⁹Arg^{26,34}Lys¹⁸GLP-1 (7-38); Ser⁸Asp¹⁷Arg^{26,34}Lys¹⁸GLP-1(7-38);
Arg^{26,34}Lys²³-GLP-1(7-36); Arg^{26,34}Lys²³-GLP-1(7-36)amide; Arg^{26,34}Lys²³GLP-1(7-37); Arg^{26,34}Lys²³GLP-1(7-38); Ser⁸Asp²⁴Arg^{26,34}Lys²³-GLP-1 (7-36); Ser⁸Asp²²Arg^{26,34}Lys²³-GLP-1 (7-36); Ser⁸Asp²⁴Arg^{26,34}Lys²³-GLP-1(7-36)amide; Ser⁸Asp²²Arg^{26,34}Lys²³-GLP-1(7-36)amide; Ser⁸Asp²⁴Arg^{26,34}Lys²³GLP-1(7-37); Ser⁸Asp²⁴Arg^{26, 34}Lys²³GLP-1(7-38); Ser⁸Asp²²Arg^{26,34}Lys²³GLP-1(7-38);
Arg^{26,34}Lys²⁷-GLP-1(7-36); Arg^{26,34}Lys²⁷-GLP-1(7-36)amide; Arg^{26,34}Lys²⁷GLP-1(7-37); Arg^{26,34}Lys²⁷GLP-1(7-38); Ser⁸Asp²⁸Arg^{26,34}Lys²⁷-GLP-1(7-36); Ser⁸Asp²⁶Arg^{26,34}Lys²⁷-GLP-1(7-36); Ser⁸Asp²⁸Arg^{26,34}Lys²⁷-GLP-1(7-36)amide; Ser⁸Asp²⁶Arg^{26,34}Lys²⁷-GLP-1(7-36)amide; Ser⁸Asp²⁸Arg²6^{,34}Lys²⁷GLP-1(7-37); Ser⁸Asp²⁸Arg^{26,34}Lys²⁷GLP-1(7-38); Ser⁸Asp²⁶Arg^{26,34}Lys²⁷GLP-1(7-38);
Arg^{26,34}Lys¹⁸-GLP-1(7-36); Arg^{26,34}Lys¹⁸-GLP-1 (7-36)amide; Arg^{26,34}Lys¹⁸GLP-1(7-37); Arg^{26,34}Lys¹⁸GLP-1(7-38); Thr⁸Asp¹⁹Arg^{26,34}Lys¹⁸-GLP-1(7-36); Thr⁸Asp¹⁷Arg^{26,34}Lys¹⁸-GLP-1(7-36); Thr⁸Asp¹⁹Arg^{26,34}Lys¹⁸-GLP-1 (7-36)amide; Thr⁸Asp¹⁷Arg^{26,34}Lys¹⁸-GLP-1(7-36)amide; Thr⁸Asp¹⁹Arg^{26,34}Lys¹⁸GLP-1(7-37); Thr⁸Asp¹⁹Arg^{26,34}Lys¹⁸GLP-1(7-38); Thr⁸Asp¹⁷Arg^{26,34}Lys¹⁸GLP-1(7-38);
Arg^{26,34}Lys²³-GLP-1(7-36); Arg^{26,34}Lys²³-GLP-1 (7-36)amide; Arg^{26,34}Lys²³GLP-1(7-37); Arg^{26,34}Lys²³GLP-1(7-38); Thr⁸ Asp²⁴Arg^{26,34}Lys²³-GLP-1(7-36); Thr⁸Asp²²Arg^{26,34}Lys²³-GLP-1 (7-36); Thr⁸Asp²⁴Arg^{26,34}Lys²³-GLP-1(7-36)amide; Thr⁸Asp²²Arg^{26,34}Lys²³-GLP-1 (7-36)amide; Thr⁸Asp²⁴Arg^{26,34}Lys²³GLP-1(7-37); Thr⁸Asp²⁴Arg^{26,34}Lys²³GLP-1(7-38); Thr⁸Asp²²Arg^{26,34}Lys²³GLP-1(7-38);
Arg^{26,34}Lys²⁷-GLP-1(7-36); Arg^{26,34}Lys²⁷-GLP-1(7-36)amide; Arg^{26, 34}Lys²⁷GLP-1(7-37); Arg^{26,34}Lys²⁷GLP-1(7-38); Thr⁸Asp²⁸Arg^{26,34}Lys²⁷-GLP-1 (7-36); Thr⁸Asp²⁶Arg^{26,34}Lys²⁷-GLP-1(7-36); Thr⁸Asp²⁸Arg^{26,34}Lys²⁷-GLP-1 (7-36)amide; Thr⁸Asp²⁶Arg^{26,34}Lys²⁷-GLP-1(7-36)amide; Thr⁸Asp²⁸Arg^{26,34}Lys²⁷GLP-1(7-37); Thr⁸Asp²⁸Arg^{26,34}Lys²⁷GLP-1(7-38); Thr⁸Asp²⁶Arg^{26,34}Lys²⁷GLP-1(7-38).

In a further preferred embodiment, the present invention relates to a GLP-1 derivative wherein the parent peptide is:
Arg²⁶Lys³⁶-GLP-1(7-36); Arg³⁴Lys³⁶-GLP-1(7-36); Arg²⁶Lys³⁶-GLP-1(7-37); Arg³⁴Lys³⁶-GLP-1(7-37); Arg²⁶Lys³⁷-GLP-1(7-37); Arg³⁴Lys³⁷-GLP-1(7-37); Arg²⁶Lys³⁹-GLP-1(7-39); Arg³⁴Lys³⁹-GLP-1 (7-39); Arg^{26,34}Lys^{36,39}-GLP-1(7-39);
Arg²⁶Lys¹⁸-GLP-1(7-36); Arg³⁴Lys¹⁸-GLP-1(7-36); Arg²⁶Lys¹⁸GLP-1(7-37); Arg³⁴Lys¹⁸GLP-1(7-37); Arg²⁶Lys¹⁸GLP-1(7-38); Arg³⁴Lys¹⁸GLP-1(7-38); Arg²⁶Lys¹⁸GLP-1(7-39); Arg³⁴Lys¹⁸GLP-1(7-39);
Arg²⁶Lys²³-GLP-1(7-36); Arg³⁴Lys²³-GLP-1(7-36); Arg²⁶Lys²³GLP-1(7-37); Arg³⁴Lys²³GLP-1(7-37); Arg²⁶Lys²³GLP-1(7-38); Arg³⁴Lys²³GLP-1(7-38); Arg²⁶Lys²³GLP-1(7-39); Arg³⁴Lys²³GLP-1 (7-39);
Arg²⁶Lys²⁷-GLP-1(7-36); Arg³⁴Lys²⁷-GLP-1(7-36); Arg²⁶Lys²⁷GLP-1(7-37); Arg³⁴Lys²⁷GLP-1(7-37); Arg²⁶Lys²⁷GLP-1(7-38); Arg³⁴Lys²⁷GLP-1(7-38); Arg²⁶Lys²⁷GLP-1(7-39); Arg³⁴Lys²⁷GLP-1 (7-39);
Arg^{26,34}Lys^{18,36}-GLP-1(7-36); Arg^{26,34}Lys¹⁸GLP-1 (7-37); Arg^{26,34}Lys^{18,37}GLP-1(7-37); Arg^{26,34}Lys^{18,38}GLP-1(7-38); Arg^{26,34}Lys^{18,39}GLP-1(7-39); Arg^{26,34}Lys^{23,36}-GLP-1(7-36); Arg^{26,34}Lys²³GLP-1(7-37); Arg^{26,34}Lys^{23,37}GLP-1 (7-37); Arg^{26,34}Lys^{23,38}GLP-1(7-38); Arg^{26,34}Lys^{23,39}GLP-1(7-39); Arg^{26,34}Lys^{27,36}-GLP-1(7-36); Arg^{26,34}Lys²⁷GLP-1(7-37); Arg^{26,34}Lys^{27,37}GLP-1(7-37); Arg^{26,34}Lys^{27,38}GLP-1(7-38); Arg^{26,34}Lys^{27,39}GLP-1(7-39);
Gly⁸GLP-1 (7-36); Gly⁸GLP-1(7-37); Gly⁸GLP-1 (7-38); Gly⁸GLP-1(7-39) Gly⁸Arg²⁶Lys³⁶-GLP-1(7-36); Gly⁸Arg³⁴Lys³⁶-GLP-1(7-36); Gly⁸Arg²⁶Lys³⁶-GLP-1(7-37); Gly⁸Arg³⁴Lys³⁶-GLP-1(7-37); Gly⁸Arg²⁶Lys³⁷-GLP-1(7-37); Gly⁸Arg³⁴Lys³⁷-GLP-1(7-37); Gly⁸Arg²⁶Lys³⁹-GLP-1(7-39); Gly⁸Arg³⁴Lys³⁹-GLP-1(7-39); Gly⁸Arg^{26,34}Lys^{36,39}-GLP-1(7-39); Gly⁸Arg²⁶Lys¹⁸-GLP-1(7-36); Gly⁸Arg³⁴Lys¹⁸-GLP-1(7-36); Gly⁸Arg²⁶Lys¹⁸GLP-1(7-37); Gly⁸Arg³⁴Lys¹⁸GLP-1(7-37); Gly⁸Arg²⁶Lys¹⁸GLP-1(7-38); Gly⁸Arg³⁴Lys¹⁸GLP-1(7-38); Gly⁸Arg²⁶Lys¹⁸GLP-1(7-39); Gly⁸Arg³⁴Lys¹⁸GLP-1(7-39);
Gly⁸Arg²⁶Lys²³-GLP-1(7-36); Gly⁸Arg³⁴Lys²³-GLP-1 (7-36); Gly⁸Arg²⁶Lys²³GLP-1(7-37); Gly⁸Arg³⁴Lys²³GLP-1(7-37); Gly⁸Arg²⁶Lys²³GLP-1(7-38); Gly⁸Arg³⁴Lys²³GLP-1(7-38); Gly⁸Arg²⁶Lys²³GLP-1(7-39); Gly⁸Arg³⁴Lys²³GLP-1(7-39);
Gly⁸Arg²⁶Lys²⁷-GLP-1(7-36); Gly⁸Arg³⁴Lys²⁷-GLP-1(7-36); Gly⁸Arg²⁶Lys²⁷GLP-1 (7-37); Gly⁸Arg³⁴Lys²⁷GLP-1(7-37); Gly⁸Arg²⁶Lys²⁷GLP-1(7-38); Gly⁸Arg³⁴Lys²⁷GLP-1(7-38); Gly⁸Arg²⁶Lys²⁷GLP-1(7-39); Gly⁸Arg³⁴Lys²⁷GLP-1 (7-39);
Gly⁸Arg^{26,34}Lys^{18,36}-GLP-1(7-36); Gly⁸Arg^{26,34}Lys¹⁸GLP-1(7-37); Gly⁸Arg2^{6,34}Lys^{18,37}GLP-1(7-37); Gly⁸Arg^{26,34}Lys^{18,38}GLP-1(7-38); Gly⁸Arg^{26,34}Ly^{18,39}GLP-1(7-39); Gly⁸Arg^{26,34}Lys^{23,34}Lys-GLP-1(7-36); Gly⁸Arg^{26,34}Lys²³GLP-1(7-37); Gly⁸Arg^{26,34}Lys^{23,37}GLP-1(7-37); Gly⁸Arg^{26,34}Ly^{23,38}GLP-1(7-38); Gly⁸Arg^{26,34}Lys^{23,39}GLP-1(7-39); Gly⁸Arg^{26,34}Lys^{27,36}-GLP-1(7-36); Gly⁸Arg^{26,34}Lys²⁷GLP-1(7-37); Gly⁸Arg^{26,34}Lys^{27,37}GLP-1(7-37); Gly⁸Arg^{26,34}Lys^{27,38}GLP-1(7-38); Gly⁸Arg^{26,34}Lys^{27,39}GLP-1 (7-39);
Val⁸GLP-1 (7-36); Val⁸GLP-1(7-37); Val⁸GLP-1(7-38); Val⁸GLP-1(7-39) Val⁸Arg²⁶Lys³⁶-GLP-1(7-36); Val⁸Arg³⁴Lys³⁶-GLP-1 (7-36); Val⁸Arg²⁶Lys³⁶-GLP-1(7-37); Val⁸Arg³⁴Lys³⁶-GLP-1(7-37); Val⁸Arg²⁶Lys³⁷-GLP-1 (7-37); Val⁸Arg³⁴Lys³⁷-GLP-1(7-37); Val⁸Arg²⁶Lys³⁹-G LP-1(7-39); Val⁸Arg³⁴Lys³⁹-GLP-1 (7-39); Val⁸Arg^{26,34}Lys^{36,39}-GLP-1 (7-39);
Val⁸Arg²⁶Lys¹⁸GLP-1(7-36); Val⁸Arg³⁴Lys¹⁸GLP-1(7-36); Val⁸Arg²⁶Lys¹⁸GLP-1 (7-37); Val⁸Arg³⁴Lys¹⁸GLP-1(7-37); Val⁸Ar²⁶Lys¹⁸GLP-1(7-38); Val⁸Arg³⁴Lys¹⁸GLP-1(7-38); Val⁸Arg²⁶Lys¹⁸GLP-1(7-39); Val⁸Arg³⁴Lys¹⁸GLP-1(7-39);
Val⁸Arg²⁶Lys²³-GLP-1(7-36); Val⁸Arg³⁴Lys²³-GLP-1(7-36); Val⁸Arg²⁶Lys²³GLP-1 (7-37); Val⁸Arg³⁴Lys²³GLP-1(7-37); Val⁸Arg²⁶Lys²³GLP-1(7-38); Val⁸Arg³⁴Lys²³GLP-1 (7-38); Val⁸Arg²⁶Lys²³GLP-1(7-39); Val⁸Arg³⁴Lys²³GLP-1(7-39);
Val⁸Arg²⁶Lys²⁷-GLP-1(7-36); Val⁸Arg³⁴Lys²⁷-GLP-1(7-36); Val⁸Arg²⁶Lys²⁷GLP-1 (7-37); Val⁸Arg³⁴Lys²⁷GLP-1(7-37); Val⁸Arg²⁶Lys²⁷GLP-1(7-38); Val⁸Arg³⁴Lys²⁷GLP-1 (7-38); Val⁸Arg²⁶Lys²⁷GLP-1(7-39); Val⁸Arg³⁴Lys²⁷GLP-1(7-39);
Val⁸Arg^{26,34}Lys^{18,36}-GLP-1(7-36); Val⁸Arg^{26,34}Lys¹⁸GLP-1(7-37); Val⁸Arg^{26,34}Lys^{18,37}GLP-1(7-37); Val⁸Arg^{26,34}Lys^{18,38}GLP-1(7-38); Val⁸Arg^{26,34}Lys^{18,39}GLP-1(7-39); Val⁸Arg^{26,34}Lys^{23,36}-GLP-1(7-36); Val⁸Arg^{26,34}Lys²³GLP-1(7-37); Val⁸Arg^{26,34}Lys^{23,37}GLP-1(7-37); Val⁸Arg^{26,34}Lys^{23,38}GLP-1 (7-38); Val⁸Arg^{26,34}Lys^{23,39}GLP-1(7-39); Val⁸Arg^{26,34}Lys^{27,36}-GLP-1(7-36); Val⁸Arg^{26,34}Lys²⁷GLP-1(7-37); Val⁸Arg^{26,34}Lys^{27,37}GLP-1(7-37); Val⁸Arg^{26,34}Lys^{27,28}GLP-1(7-38); Val⁸Arg^{26,34}Lys^{27,39}GLP-1 (7-39).

Other preferred embodiments will be described using the following abbreviations:
Glut = N^{ε}-(γ-L-glutamyl)
Aspa = N^{ε}-(β-L-asparagyl)
Glyc = N^{ε}-glycyl
GAB = N^{ε}-(α-(γ-aminobutanoyl)
ADod = N^{α}-dodecanoyl
ATet = N^{α}-tetradecanoyl
AHex = N^{α}-hexadecanoy)
AOct = N^{α}-octadecanoyl
ALit = N^{α}-lithocholyl
GDod = N^{γ}-dodecanoyl
GTet = N^{γ}-tetradecanoyl
GHex = N^{γ}-hexadecanoyl
GOct = N^{γ}-octadecanoyl
GLit = N^{γ}-lithocholyl

Other preferred derivatives of GLP-1 analogues of the present invention are:
Arg^{26,34}Lys³⁶-(Glut-ADod)-GLP-1(7-36); Arg^{26,34}Lys³⁶-(Glut-ADod)GLP-1(7-36)amide; Arg^{26,34}Lys³⁸-(Glut-ADod)-GLP-1(7-38); Arg^{26,34}Lys³⁹-(Glut-ADod)-GLP-1(7-39);
Gly⁸Arg^{26,34}Lys³⁶-(Glut-ADod)-GLP-1(7-36); Gly⁸Arg^{26,34}Lys³⁶-(Glut-ADod)-GLP-(7-36)amide; Gly⁸Arg^{26,34}Lys³⁸-(Glut-ADod)-GLP-1(7-38); Gly⁸Arg^{26,34}Lys³⁹-(Glut-ADod)-GLP-1(7-39);
Val⁸Arg^{26,34}Lys³⁶-(Glut-ADod)-GLP-1(7-36); Val⁸Arg^{26,34}Lys³⁶-(Glut-ADod)-GLP-1(7-36)amide; Val⁸Arg^{26,34}Lys³⁸-(Glut-ADod)-GLP-1(7-38); Val⁸Arg^{26,34}Lys³⁹-(Glut-ADod)-GLP-1(7-39);
Ser⁸Arg^{26,34}Lys³⁶-(Glut-ADod)-GLP-1(7-36); Ser⁸Arg^{26,34}Lys³⁶-(Glut-ADod)-GLP-1(7-36)amide; Ser⁸Arg^{26,34}Lys³⁸-(Glut-ADod)-GLP-1(7-38); Ser⁸Arg^{26,34}Lys³⁹-(Glut-ADod)-GLP-1(7-39);
Thr⁸Arg^{26,34}Lys³⁶-(Glut-ADod)-GLP-1(7-36); Thr⁸Arg^{26,34}Lys³⁶-(Glut-ADod)-GLP-1(7-36)amide; Thr⁸Arg^{26,34}Lys³⁸-(Glut-ADod)-GLP-1(7-38); Thr⁸Arg^{26,34}Lys³⁹-(Glut-ADod)-GLP-1(7-39);
Gly⁸Glu³⁵Arg^{26,34}Lys³⁶-(Glut-ADod)-GLP-1(7-36); Gly⁸Glu³⁵Arg^{26,34}Lys³⁶-(Glut-ADod)-GLP-1 (7-36)amide; Gly⁸Glu³⁶Arg^{26,34}Lys³⁷-(Glut-ADod)-GLP-1(7-37); Gly⁸Glu³⁷Arg^{26,34}Lys³⁸-(Glut-ADod)-GLP-1 (7-38); Gly⁸Glu³⁸Arg^{26,34}Lys³⁹-(Glut-ADod)-GLP-1(7-39); Gly⁸Glu³⁵Arg^{26,34}Lys³⁶-(Glut-ADod)-GLP-1 (7-36); Gly⁸Glu³⁵Arg^{26,34}Lys³⁶-(Glut-ADod)-GLP-1 (7-36)amide; Gly⁸Glu³⁶Arg^{26,34}Lys³⁷-(Glut-ADod)-GLP-1(7-37); Gly⁸Glu³⁷Arg^{26,34}Lys³⁸-(Glut-ADod)GLP-1 (7-38); Gly⁸Glu³⁸Arg^{26,34}Lys³⁹-(Glut-ADod)GLP-1(7-39);
Gly⁸Asp³⁵Arg^{26,34}Lys³⁶-(Glut-ADod)-GLP-1(7-36); Gly⁸Asp³⁵Arg^{26,34}Lys³⁶-(Glut-ADod)-GLP-1(7-36)amide; Gly⁸Asp³⁶Arg^{26,34}Lys³⁷-(Glut-ADod)-GLP-1 (7-37); Gly⁸Asp³⁷Arg^{26,34}Lys³⁸-(Glut-ADod)-GLP-1(7-38); Gly⁸Asp³⁸Arg^{26,34}Lys³⁹-(Glut-ADod)-GLP-1 (7-39); Gly⁸Asp³⁵Arg^{26,34} Lys³⁶-(Glut-ADod)-GLP-1 (7-36); Gly⁸Asp³⁵Arg^{26,34}Lys³⁶-(Glut-ADod)-GLP-1 (7-36)amide; Gly⁸Asp³⁶Arg^{26,34}Lys³⁷-(Glut-ADod)-GLP-1(7-37); Gly⁸Asp³⁷Arg^{26,34}Lys³⁸-(Glut-ADod)-GLP-1(7-38); Gly⁸Asp³⁸Arg^{26,34} Lys³⁹-(Glut-ADod)-GLP-1 (7-39);
Val⁸Glu³⁵Arg^{26,34}Lys³⁶-(Glut-ADod)-GLP-1(7-36); Val⁸Glu³⁵Arg^{26,34}Lys³⁶-(Glut-ADod)-GLP-1(7-36)amide; Val⁸Glu³⁶Arg^{26,34}Lys³⁷-(Glut-ADod)-GLP-1(7-37); Val⁸Glu³⁷Arg^{26,34} Lys³⁸ -(Glut-ADod)-GLP-1 (7-38); Val⁸Glu³⁸Arg^{26,34}Lys³⁹-(Glut-ADod)-GLP-1 (7-39); Val⁸Glu³⁵Arg^{26,34}Lys³⁶-(Glut-ADod)-GLP-1 (7-36); Val⁸Glu³⁵Arg^{26,34}Lys³⁶-(Glut-ADod)-GLP-1 (7-36)amide; Val⁸Glu³⁶Arg^{26,34}Lys³⁷-(Glut-ADod)-GLP-1(7-37); Val⁸Glu³⁷Arg^{26,34}Lys³⁸-(Glut-ADod)-GLP-1(7-38); Val⁸Glu³⁸Arg^{26,34}Lys³⁹-(Glut-ADod)-GLP-1 (7-39);
Val⁸Asp³⁵Arg^{26,34}Lys³⁶-(Glut-ADod)-GLP-1 (7-36); Val⁸ Asp³⁵Arg^{26,34}Lys³⁶-(Glut-ADod)-GLP-1(7-36)amide; Val⁸Asp³⁶Arg^{26,34}Lys³⁷-(Glut-ADod)-GLP-1(7-37); Val⁸Asp³⁷Arg^{26,34}Lys³⁸-(Glut-ADod)-GLP-1 (7-38); Val⁸Asp³⁸Arg^{26,34}Lys³⁹-(Glut-ADod)-GLP-1(7-39); Val⁸Asp³⁵Arg^{26,34}Lys³⁶-(Glut-ADod)-GLP-1 (7-36); Val⁸Asp³⁵Arg^{26,34}Lys³⁶-(Glut-ADod)-GLP-1 (7-36)amide; Val⁸Asp³⁶Arg^{26,34}Lys³⁷-(Glut-ADod)-GLP-1(7-37); Val⁸Asp³⁷Arg^{26,34}Lys³⁸-(Glut-ADod)-GLP-1(7-38); Val⁸Asp³⁸Arg^{26,34}Lys³⁹-(Glut-ADod)-GLP-1(7-39);
Ser⁸Glu³⁵Arg^{26,34} Lys³⁶-(Glut-ADod)-GLP-1(7-36); Ser⁸Glu³⁵ Arg^{26,34}Lys³⁶-(Glut-ADod)-GLP-1(7-36)amide; Ser⁸Glu³⁶Arg^{26,34}Lys³⁷-(Glut-ADod)-GLP-1(7-37); Ser⁸Glu³⁷Arg^{26,34}Lys³⁸-(Glut-ADod)-GLP-1 (7-38); Ser⁸Glu³⁸Arg^{26,34}Lys³⁹-(Glut-ADod)-GLP-1 (7-39); Ser⁸Glu³⁵ Arg^{26,34}Lys³⁶-(Glut-ADod)-GLP-1 (7-36); Ser⁸Glu³⁵Arg^{26,34}Lys³⁶-(Glut-ADod)-GLP-1 (7-36)amide; Ser⁸Glu³⁶Arg^{26,34}Lys³⁷-(Glut-ADod)-GLP-1(7-37); Ser⁸Glu³⁷Arg^{26,34}Lys³⁸-(Glut-ADod)-GLP-1(7-38); Ser⁸Glu³⁸Arg^{26,34}Lys³⁹-(Glut-ADod)GLP-1(7-39);
Ser⁸ Asp³⁵ Arg^{26,34}Lys³⁶-(Glut-ADod)-GLP-1(7-36); Ser⁸ Asp³⁵ Arg^{26,34}Lys³⁶ -(Glut-ADod)-GLP-1(7-36)amide; Ser⁸Asp³⁶Arg^{26,34}Lys³⁷-(Glut-ADod)-GLP-1(7-37); Ser⁸Asp³⁷ Arg^{26,34}Lys³⁸-(Glut-ADod)-GLP-1(7-38); Ser⁸Asp³⁸Arg^{26,34}Lys³⁹-(Glut-ADod)-GLP-1(7-39); Ser⁸Asp³⁵Arg^{26,34}Lys³⁶-(Glut-ADod)-GLP-1(7-36); Ser⁸Asp³⁵Arg^{26,34}Lys³⁶-(Glut-ADod)GLP-1(7-36)amide; Ser⁸Asp³⁶Arg^{26,34}Lys³⁷-(Glut-ADod)-GLP-1(7-37); Ser⁸Asp³⁷Arg^{26,34}Lys³⁸-(Glut-ADod)-GLP-1(7-38); Ser⁸Asp³⁸Arg^{26,34}Lys³⁹-(Glut-ADod)-GLP-1(7-39);
Thr⁸Glu³⁵Arg^{26,34}Lys³⁶-(Glut-ADod)-GLP-1(7-36); Thr⁸Glu³⁵ Arg^{26,34}Lys³⁶-(Glut-ADod)-GLP-1(7-36)amide; Thr⁸Glu³⁶Arg^{26,34}Lys³⁷-(Glut-ADod)-GLP-1(7-37); Thr⁸Glu³⁷Arg^{26,34}Lys³⁸-(Glut-ADod)-GLP-1(7-38); Thr⁸Glu³⁸Arg^{26,34}Lys³⁹-(Glut-ADod)GLP-1(7-39); Thr⁸Glu³⁵Arg^{26,34} Lys³⁶-(Glut-ADod)-GLP-1(7-36); Thr⁸Glu³⁵Arg^{26,34}Lys³⁶-(Glut-ADod)GLP-1 (7-36)amide; Thr⁸Glu³⁶Arg^{26,34}Lys³⁷-(Glut-ADod)-GLP-1(7-37); Thr⁸Glu³⁷Arg^{26,34}Lys³⁸-(Glut-ADod)-GLP-1(7-38); Thr⁸Glu³⁸Arg^{26,34}Lys³⁹-(Glut-ADod)-GLP-1(7-39);
Thr⁸Asp³⁵Arg^{26,34}Lys³⁶-(Glut-ADod)-GLP-1(7-36); Thr⁸Asp³⁵Arg^{26,34}Lys³⁶-(Glut-ADod)-GLP-1(7-36)amide; Thr⁸Asp³⁶Arg^{26,34}Lys³⁷-(Glut-ADod)-GLP-1(7-37); Thr⁸Asp³⁷Arg^{26,34}Lys³⁸-(Glut-ADod)-GLP-1(7-38); Thr⁸Asp³⁸Arg^{26,34}Lys³⁹-(Glut-ADod)-GLP-1(7-39); Thr⁸Asp³⁵Arg^{26,34}Lys³⁶-(Glut-ADod)GLP-1(7-36); Thr⁸Asp³⁵Arg^{26,34}Lys³⁶-(Glut-ADod)-GLP-1(7-36)amide; Thr⁸Asp³⁶Arg^{26,34}Lys³⁷-(Glut-ADod)-GLP-1(7-37); Thr⁸Asp³⁷Arg^{26,34}Lys³⁸-(Glut-ADod)-GLP-1 (7-38); Thr⁸Asp³⁸Arg^{26,34}Lys³⁹-(Glut-ADod)-GLP-1(7-39);
Arg^{26,34}Lys³⁶-(Glut-ATet)GLP-1(7-36); Arg^{26,34}Lys³⁶-(Glut-ATet)GLP-1(7-36)amide; Arg^{26,34}Lys³⁶-(Glut-ATet)-GLP-1(7-37); Arg^{26,34}Lys³⁸-(Glut-ATet)-GLP-1(7-38); Arg^{26,34}Lys³⁹-(Glut-AT et) GLP-1 (7-39);
Gly⁸Arg^{26,34}Lys³⁶-(Glut-ATet)-GLP-1(7-36); Gly⁸Arg^{26,34}Lys³⁶-(Glut-ATet)-GLP-1(7-36)amide; Gly⁸Arg^{26,34}Lys³⁶-(Glut-ATet)-GLP-1(7-37); Gly⁸Arg^{26,34}Lys³⁸-(Glut-ATet)-GLP-1(7-38); Gly⁸Arg^{26,34}Lys³⁹-(Glut-ATet)-GLP-1(7-39);
Val⁸Arg^{26,34}Lys³⁶-(Glut-ATet)-GLP-1(7-36); Val⁸Arg^{26,34}Lys³⁶-(Glut-ATet)-GLP-1(7-36)amide; Val⁸Arg^{26,34}Lys³⁸-(Glut-ATet)-GLP-1(7-38); Val⁸Arg^{26,34}Lys³⁹-(Glut-ATet)-GLP-1(7-39);
Ser⁸Arg^{26,34}Lys³⁶-(Glut-ATet)-GLP-1(7-36); Ser⁸Arg^{26,34}Lys³⁶-(Glut-ATet)-GLP-1(7-36)amide; Ser⁸Arg^{26,34}Lys³⁸-(Glut-ATet)GLP-1(7-38); Ser⁸Arg^{26,34}Lys³⁹-(Glut-ATet)-GLP-1(7-39);
Thr⁸Arg^{26,34}Lys³⁶-(Glut-ATet)-GLP-1(7-36); Thr⁸Arg^{26,34}Lys³⁶-(Glut-ATet)-GLP-1(7-36)amide; Thr⁸Arg^{26,34}Lys³⁸-(Glut-ATet)GLP-1(7-38); Thr⁸Arg^{26,34}Lys³⁹-(Glut-ATet)GLP-1(7-39);
Gly⁸Glu³⁵Arg^{26,34}Lys³⁶-(Glut-ATet)-GLP-1(7-36); Gly⁸Glu³⁵Arg^{26,34}Lys³⁶-(Glut-ATet)-GLP-1 (7-36)amide; Gly⁸Glu³⁶Arg^{26,34}Lys³⁷-(Glut-ATet)-GLP-1(7-37); Gly⁸Glu³⁷Arg^{26,34}Lys³⁸-(Glut-ATet)-GLP-1 (7-38); Gly⁸Glu³⁸Arg^{26,34}Lys³⁹-(Glut-ATet)-GLP-1(7-39); Gly⁸Glu³⁵Arg^{26,34}Lys³⁶-(Glut-ATet)-GLP-1 (7-36); Gly⁸Glu³⁵Arg^{26,34}Lys³⁶-(Glut-ATet)GLP-1(7-36)amide; Gly⁸Glu³⁶Arg^{26,34}Lys³⁷-(Glut-ATet)-GLP-1(7-37); Gly⁸Glu³⁷Arg^{26,34}Lys³⁸-(Glut-ATet)-GLP-1 (7-38); Gly⁸Glu³⁸Arg^{26,34}Lys³⁹-(Glut-ATet)-GLP-1(7-39);
Gly⁸Asp³⁵Arg^{26,34}Lys³⁶-(Glut-ATet)-GLP-1(7-36); Gly⁸Asp³⁵Arg^{26,34}Lys³⁶-(Glut-ATet)-GLP-1 (7-36)amide; Gly⁸Asp³⁶Arg^{26,34}Lys³⁷-(Glut-ATet)-GLP-1(7-37); Gly⁸Asp³⁷ Arg^{26,34}Lys³⁸ -(Glut-AT et)-GLP-1(7-38); Gly⁸Asp³⁸ Arg^{26,34}Lys³⁹-(Glut-ATet)-GLP-1(7-39); Gly⁸Asp³⁵Arg^{26,34} Lys³⁶ -(Glut-ATet)-GLP-1 (7-36); Gly⁸Asp³⁵Arg^{26,34}Lys³⁶-(Glut-ATet)-GLP-1(7-36)amide; Gly⁸Asp³⁶ Arg^{26,34}Lys³⁷-(Glut-ATet)-GLP-1(7-37); Gly Asp³⁷Arg^{26,34} Lys³⁸ -(Glut-ATet)-GLP-1(7-38); Gly⁸Asp³⁸Arg^{26,34}Lys³⁹-(Glut-ATet)-GLP-1(7-39);
Val⁸Glu³⁵Arg^{26,34}Lys³⁶-(Glut-ATet)GLP-1 (7-36); Val⁸Glu³⁵Arg^{26,34}Lys³⁶-(Glut-ATet)GLP-1 (7-36)amide; Val⁸Glu³⁶Arg^{26,34}Lys³⁷-(Glut-ATet)-GLP-1(7-37); Val⁸Glu³⁷Arg^{26,34} Lys³⁸ -(Glut-ATet)-GLP-1 (7-38); Val⁸Glu³⁸Arg^{26,34}Lys³⁹-(Glut-ATet)-GLP-1(7-39); Val⁸Glu³⁵Arg^{26,34}Lys³⁶-(Glut-ATet)-GLP-1 (7-36); Val⁸Glu³⁵Arg^{26,34}Lys³⁶-(Glut-ATet)-GLP-1(7-36)amide; Val⁸Glu³⁶Arg^{26,34}Lys³⁷-(Glut-ATet)-GLP-1(7-37); Val⁸Glu³⁷Arg^{26,34} Lys³⁸-(Glut-ATet)-GLP-1 (7-38); Val⁸Glu³⁸Arg^{26,34}Lys³⁹-(Glut-ATet)-GLP-1(7-39);
Val⁸Asp³⁵Arg^{26,34}Lys³⁶-(Glut-ATet)GLP-1(7-36); Val⁸Asp³⁵Arg^{26,34} Lys³⁶ -(Glut-ATet)-GLP-1 (7-36)amide; Val⁸Asp³⁶Arg^{26,34}Lys³⁷-(Glut-ATet)-GLP-1(7-37); Val⁸Asp³⁷Arg^{26,34}Lys³⁸-(Glut-ATet)-GLP-1(7-38); Val⁸Asp³⁸Arg^{26,34}Lys³⁹-(Glut-ATet)-GLP-1(7-39); Val⁸Asp³⁵Arg^{26,34}Lys³⁶-(Glut-ATet)-GLP-1 (7-36); Val⁸Asp³⁵Arg^{26,34} Lys³⁶-(Glut-ATet)-GLP-1(7-36)amide; Val⁸Asp³⁶Arg^{26,34}Lys³⁷-(Glut-ATet)-GLP-1(7-37); Val⁸Asp³⁷Arg^{26,34}Lys³⁸-(Glut-ATet)GLP-1 (7-38); Val⁸Asp³⁸Arg^{26,34}Lys³⁹-(Glut-ATet)-GLP-1(7-39);
Ser⁸Glu³⁵Arg^{26,34}Lys³⁶-(Glut-ATet)GLP-1(7-36); Ser⁸Glu³⁵Arg^{26,34}Lys³⁶-(Glut-ATet)GLP-1 (7-36)amide; Ser⁸Glu³⁶Arg^{26,34}Lys³⁷-(Glut-ATet)-GLP-1(7-37); Ser⁸Glu³⁷Arg^{26,34}Lys³⁸-(Glut-ATet)-GLP-1 (7-38); Ser⁸Glu³⁸Arg^{26,34}Lys³⁹-(Glut-ATet)-GLP-1(7-39); Ser⁸Glu³⁵Arg^{26,34}Lys³⁶-(Glut-ATet)-GLP-1 (7-36); Ser⁸Glu³⁵Arg^{26,34}Lys³⁶-(Glut-ATet)GLP-1(7-36)amide; Ser⁸Glu³⁶Arg^{26,34}Lys³⁷-(Glut-ATet)-GLP-1(7-37); Ser⁸Glu³⁷Arg^{26,34}Lys³⁸-(Glut-ATet)-GLP-1 (7-38); Ser⁸Glu³⁸Arg^{26,34}Lys³⁹-(Glut-ATet)-GLP-1(7-39);
Ser⁸Asp³⁵Arg^{26,34}Lys³⁶-(Glut-ATet)-GLP-1(7-36); Ser⁸Asp³⁵Arg^{26,34}Lys³⁶-(Glut-ATet)GLP-1 (7-36)amide; Ser⁸Asp³⁶Arg^{26,34}Lys³⁷-(Glut-ATet)-GLP-1(7-37); Ser⁸Asp³⁷Arg^{26,34}Lys³⁸-(Glut-ATet)-GLP-1 (7-38); Ser⁸Asp³⁸Arg^{26,34}Lys³⁹-(Glut-ATet)-GLP-1(7-39); Ser⁸Asp³⁵Arg^{26,34}Lys³⁶-(Glut-ATet)GLP-1 (7-36); Ser⁸Asp³⁵Arg^{26,34}Lys³⁶-(Glut-ATet)-GLP-1(7-36)amide; Ser⁸Asp³⁶Arg^{26,34}Lys³⁷-(Glut-ATet)-GLP-1(7-37); Ser⁸Asp³⁷Arg^{26,34}Lys³⁸-(Glut-ATet)-GLP-1 (7-38); Ser⁸Asp³⁸Arg^{26,34}Lys³⁹-(Glut-ATet)-GLP-1(7-39);
Thr⁸Glu³⁵Arg^{26,34}Lys³⁶-(Glut-ATet)GLP-1(7-36); Thr⁸Glu³⁵Arg^{26,34}Lys³⁶-(Glut-ATet)-GLP-1 (7-36)amide; Thr⁸Glu³⁶Arg^{26,34}Lys³⁷-(Glut-ATet)-GLP-1(7-37); Thr⁸Glu³⁷Arg^{26,34}Lys³⁸ -(Glut-ATet)-GLP-1 (7-38); Thr⁸Glu³⁸Arg^{26,34}Lys³⁹-(Glut-ATet)GLP-1(7-39); Thr⁸Glu³⁵Arg^{26,34}Lys³⁶-(Glut-ATet)-GLP-1 (7-36); Thr⁸Glu³⁵Arg^{26,34}Lys³⁶-(Glut-ATet)GLP-1(7-36)amide; Thr⁸Glu³⁶Arg^{26,34}Lys³⁷-(Glut-ATet)-GLP-1(7-37); Thr⁸Glu³⁷Arg^{26,34}Lys³⁸-(Glut-A Tet)-GLP-1 (7-38); Thr⁸Glu³⁸Arg²⁶³⁴Lys³⁹-(Glut-ATet)GLP-1(7-39);
Thr⁸Asp³⁵Arg^{26,34}Lys³⁶-(Glut-ATet)-GLP-1(7-36); Thr⁸Asp³⁵Arg^{26,34}Lys³⁶-(Glut-ATet)-GLP-1 (7-36)amide; Thr⁸Asp³⁶Arg^{26,34}Lys³⁷-(Glut-ATet)-GLP-1(7-37); Thr⁸Asp³⁷Arg^{26,34}Lys³⁸-(Glut-ATet)-GLP-1 (7-38); Thr⁸Asp³⁸Arg^{26,34}Lys³⁹-(Glut-ATet)-GLP-1(7-39); Thr⁸Asp³⁵Arg^{26,34}Lys³⁶-(Glut-ATet)-GLP-1(7-36); Thr⁸Asp³⁵Arg^{26,34}Lys³⁶-(Glut-ATet)-GLP-1(7-36)amide; Thr⁸Asp³⁶Arg^{26,34}Lys³⁷-(Glut-ATet)-GLP-1(7-37); Thr⁸Asp³⁷Arg^{26,34}Lys³⁸-(Glut-ATet)-GLP-1 (7-38); Thr⁸Asp³⁸Arg^{26,34}Lys³⁹-(Glut-ATet)-GLP-1(7-39);
Arg^{26,34}Lys³⁶-(Glut-AHex)-GLP-1(7-36); Arg^{26,34}Lys³⁶-(Glut-AHex)-GLP-1 (7-36)amide; Arg^{26,34}Lys³⁸-(Glut-AHex)-GLP-1(7-38); Arg^{26,34}Lys³⁹-(Glut-AHex)-GLP-1 (7-39);
Gly⁸Arg^{26,34}Lys³⁶-(Glut-AHex)-GLP-1(7-36); Gly⁸Arg^{26,34}Lys³⁶-(Glut-AHex)-GLP-1 (7-36)amide; Gly⁸Arg^{26,34}Lys³-(Glut-AHex)-GLP-1(7-38); Gly⁸Arg^{26,34}Lys³⁹-(Glut-AHex)-GLP-1(7-39);
Val⁸Arg^{26,34}Lys³⁶-(Glut-AHex)-GLP-1(7-36); Val⁸Arg^{26,34}Lys-16-(Glut-AHex)-GLP-1 (7-36)amide; Val⁸Arg^{26,34}Lys³⁸-(Glut-AHex)-GLP-1(7-38); Val⁸Arg^{26,34}Lys³⁹-(Glut-AHex)-GLP-1 (7-39);
Ser⁸Arg^{26,34} Lys³⁶-(Glut-AHex)-GLP-1(7-36); Ser⁸Arg^{26,34}Lys³⁶-(Glut-AHex)-GLP-1(7-36)amide; Ser⁸Arg^{26,34}Lys³⁸-(Glut-AHex)-GLP-1(7-38); Ser⁸Arg^{26,34} Lys³⁹ -(Glut-AHex)-GLP-1 (7-39);
Thr⁸Arg^{26,34}Lys³⁶ -(Glut-AHex)-GLP-1(7-36); Thr⁸Arg^{26,34}Lys³⁶-(Glut-AHex)-GLP-1 (7-36)amide; Thr⁸Arg^{26,34} Lys³⁸ -(Glut-AHex)-GLP-1(7-38); Thr⁸Arg^{26,34}Lys³⁹ -(Glut-AHex)-GLP-1 (7-39);
Gly⁸Glu³⁵Arg^{26,34}Lys³⁶-(Glut-AHex)-GLP-1(7-36); Gly⁸Glu³⁵Arg^{26,34}Lys³⁶-(Glut-AHex)-GLP-1 (7-36)amide; Gly⁸Glu³⁶Arg^{26,34}Lys³⁷-(Glut-AHex)-GLP-1(7-37); Gly⁸Glu³⁷ Arg^{26,34} Lys³⁸ -(Glut-AHex)-GLP-1 (7-38); Gly⁸Glu³⁸Arg^{26,34}Lys-(Glut-AHex)-GLP-1 (7-39); Gly⁸Glu³⁵Arg^{26,34}Lys³⁶ -(Glut-AHex)-GLP-1 (7-36); Gly⁸Glu³⁵Arg^{26,34}Lys³⁶-(Glut-AHex)-GLP-1 (7-36)amide; Gly⁸Glu³⁶Arg^{26,34}Lys³⁷-(Glut-AHex)-GLP-1(7-37); Gly⁸Glu³⁷Arg^{26,34}Lys³⁸-(Glut-AHex)-GLP-1(7-38); Gly⁸Glu³⁸Arg^{26,34}Lys³⁹-(Glut-AHex)-GLP-1 (7-39);
Gly⁸Asp³⁵Arg^{26,34}Lys³⁶-(Glut-AHex)-GLP-1(7-36); Gly⁸Asp³⁵ Arg^{26,34}Lys³⁶-(Glut-AHex)-GLP-1(7-36)amide; Gly⁸Asp³⁶Arg^{26,34}Lys³⁷-(Glut-AHex)-GLP-1(7-37); Gly⁸Asp³⁷Arg^{26,34}Lys³⁸-(Glut-AHex)-GLP-1 (7-38); Gly⁸Asp³⁸Arg^{26,34}Lys³⁹-(Glut-AHex)-GLP-1(7-39); Gly⁸Asp³⁵Arg^{26,34}Lys³⁶-(Glut-AHex)-GLP-1 (7-36); Gly⁸Asp³⁵Arg^{26,34}Lys³⁶-(Glut-AHex)-GLP-1 (7-36)amide; Gly⁸Asp³⁶Arg^{26,34}Lys³⁷-(Glut-AHex)-GLP-1(7-37); Gly⁸Asp³⁷Arg^{26,34}Lys³⁸-(Glut-AHex)-GLP-1(7-38); Gly⁸Asp³⁸Arg^{26,34}Lys³⁹-(Glut-AHex)-GLP-1 (7-39);
Val⁸Glu³⁵Arg^{26,34}Lys³⁶-(Glut-AHex)-GLP-1(7-36); Val⁸Glu³⁵Arg^{26,34}Lys³⁶-(Glut-AHex)-GLP-1 (7-36)amide; Val⁸Glu³⁶Arg^{26,34}Lys³⁷-(Glut-AHex)-GLP-1(7-37); Val⁸Glu³⁷Arg^{26,34}Lys³⁸-(Glut-AHex)-GLP-1 (7-38); Val⁸Glu³⁸Arg^{26,34}Lys³⁹-(Glut-AHex)-GLP-1(7-39); Val⁸Glu³⁵Arg^{26,34}Lys³⁶-(Glut-AHex)-GLP-1 (7-36); Val⁸Glu³⁵Arg^{26,34}Lys³⁶-(Glut-AHex)-GLP-1(7-36)amide; Val⁸Glu³⁶Arg^{26,34}Lys³⁷-(Glut-AHex)-GLP-1(7-37); Val⁸Glu³⁷Arg^{26,34} Lys³⁸-(Glut-AHex)-GLP-1(7-38); Val⁸Glu³⁸Arg^{26,34}Lys³⁹-(Glut-AHex)-GLP-1(7-39);
Val⁸Asp³⁵Arg^{26,34}Lys³⁶ -(Glut-AHex)-GLP-1(7-36); Val⁸ Asp³⁵Arg^{26,34}Lys³⁶-(Glut-AHex)-GLP-1 (7-36)amide; Val⁸Asp³⁶Arg^{26,34}Lys³⁷-(Glut-AHex)-GLP-1(7-37); Val⁸Asp³⁷Arg^{26,34}Lys³⁸-(Glut-AHex)-GLP-1 (7-38); Val⁸Asp³⁸Arg^{26,34}Lys³⁹-(Glut-AHex)-GLP-1(7-39); Val⁸Asp³⁵Arg^{26,34}Lys³⁶-(Glut-AHex)-GLP-1 (7-36); Val⁸Asp³⁵Arg^{26,34}Lys³⁶-(Glut-AHex)-GLP-1(7-36)amide; Val⁸Asp³⁶Arg^{26,34}Lys³⁷-(Glut-AHex)-GLP-1(7-37); Val⁸Asp³⁷Arg^{26,34}Lys³⁸-(Glut-AHex)-GLP-1 (7-38); Val⁸Asp³⁸Arg^{26,34}Lys³⁹-(Glut-AHex)-GLP-1(7-39);
Ser⁸Glu³⁵Arg^{26,34}Lys³⁶-(Glut-AHex)-GLP-1(7-36); Ser⁸Glu³⁵Arg^{26,34}Lys³⁶-(Glut-AHex)-GLP-1 (7-36)amide; Ser⁸Glu³⁶Arg^{26,34}Lys³⁷-(Glut-AHex)-GLP-1(7-37); Ser⁸Glu³⁷ Arg^{26,34}Lys³⁸ -(Glut-AHex)-GLP-1 (7-38); Ser⁸Glu³⁸Arg^{26,34} Lys³⁹-(Glut-AHex)-GLP-1(7-39); Ser⁸Glu³⁵ Arg^{26,34}Lys³⁶-(Glut-AHex)-GLP-1 (7-36); Ser⁸Glu³⁵Arg^{26,34}Lys³⁶-(Glut-AHex)-GLP-1(7-36)amide; Ser⁸Glu³⁶Arg^{26,34}Lys³⁷-(Glut-AHex)-GLP-1(7-37); Ser⁸Glu³⁷Arg^{26,34}Lys³⁸-(Glut-AHex)-GLP-1(7-38); Ser⁸Glu³⁸Arg^{26,34}Lys³⁹-(Glut-AHex)-GLP-1(7-39);
Ser⁸Asp³⁵Arg^{26,34}Lys³⁶-(Glut-AHex)-GLP-1(7-36); Ser⁸Asp³⁵Arg^{26,34}Lys³⁶-(Glut-AHex)-GLP-1 (7-36)amide; Ser⁸Asp³⁶Arg^{26,34}Lys³⁷-(Glut-AHex)-GLP-1(7-37); Ser⁸Asp³⁷Arg^{26,34}Lys³⁸-(Glut-AHex)-GLP-1 (7-38); Ser⁸Asp³⁸ Arg^{26,34} Lys³⁹-(Glut-AHex)-GLP-1(7-39); Ser⁸Asp³⁵ Arg^{26,34}Lys³⁶-(Glut-AHex)GLP-1(7-36); Ser⁸Asp³⁵Arg^{26,34}Lys³⁶-(Glut-AHex)-GLP-1(7-36)amide; Ser⁸Asp³⁶Arg^{26,34}Lys³⁷-(Glut-AHex)-GLP-1(7-37); Ser⁸ Asp³⁷ Arg^{26,34}Lys³⁸ -(Glut-AHex)-GLP-1 (7-38); Ser⁸Asp³⁸Arg^{26,34}Lys³⁹-(Glut-AHex)-GLP-1(7-39);
Thr⁸Glu³⁵Arg^{26,34}Lys³⁶-(Glut-AHex)-GLP-1(7-36); Thr⁸Glu³⁵ Arg^{26,34}Lys³⁶-(Glut-AHex)-GLP-1 (7-36)amide; Thr⁸Glu³⁶Arg^{26,34}Lys³⁷-(Glut-AHex)-GLP-1(7-37); Thr⁸Glu³⁷Arg^{26,34}Lys³⁸-(Glut-AHex)-GLP-1 (7-38); Thr⁸Glu³⁸Arg^{26,34}Lys³⁹-(Glut-AHex)-GLP-1(7-39); Thr⁸Glu³⁵Arg^{26,34}Lys³⁶-(Glut-AHex)-GLP-1(7-36); Thr⁸Glu³⁵Arg^{26,34}Lys³⁶-(Glut-AHex)-GLP-1 (7-36)amide; Thr⁸Glu³⁶Arg^{26,34}Lys³⁷-(Glut-AHex)-GLP-1(7-37); Thr⁸Glu³⁷Arg^{26,34}Lys³⁸-(Glut-AHex)-GLP-1 (7-38); Thr⁸Glu³⁸Arg^{26,34}Lys³⁹-(Glut-AHex)-GLP-1 (7-39);
Thr⁸Asp³⁵Arg^{26,34}Lys³⁶-(Glut-AHex)-GLP-1(7-36); Thr⁸Asp³⁵Arg^{26,34}Lys³⁶-(Glut-AHex)-GLP-1 (7-36)amide; Thr⁸Asp³⁶ Arg^{26,34}Lys³⁷-(Glut-AHex)-GLP-1(7-37); Thr⁸Asp³⁷Arg^{26,34}Lys³⁸-(Glut-AHex)-GLP-1 (7-38); Thr⁸Asp³⁸ Arg^{26,34} Lys³⁹-(Glut-AHex)-GLP-1(7-39); Thr⁸Asp³⁵Arg^{26,34}Lys³⁶-(Glut-AHex)-GLP-1 (7-36); Thr⁸Asp³⁵Arg^{26,34}Lys³⁶-(Glut-AHex)-GLP-1 (7-36)amide; Thr⁸Asp³⁶Arg^{26,34}Lys³⁷-(Glut-AHex)-GLP-1(7-37); Thr⁸Asp³⁷Arg^{26,34}Lys³⁸-(Glut-AHex)GLP-1 (7-38); Thr⁸Asp³⁸Arg^{26,34}Lys³⁹-(Glut-AHex)-GLP-1 (7-39);
Arg^{26,34}Lys³⁶-(Glut-AOcty)-GLP-1(7-36); Arg^{26,34}Lys³⁶-(Glut-AOct)-GLP-1(7-36)amid; Arg^{26,34}Lys³⁸-(Glut-AOct)-GLP-1(7-38); Arg³⁴Lys²⁶-(Glut-AOct)-GLP-1(7-39); Arg^{26,34} Lys³⁹ -(Glut-AOct)-GLP-1 (7-39);
Gly⁸Arg^{26,34}Lys³⁶-(Glut-AOct)GLP-1(7-36); Gly Arg^{26,34} Lys³⁶-(Glut-AOct)-GLP-1 (7-36)amide; Gly⁸Arg^{26,34}Lys³⁸-(Glut-AOct)-GLP-1(7-38); Gly⁸Arg^{26,34}Lys³⁹-(Glut-AOct)-GLP-1 (7-39);
Val⁸Arg^{26,34}Lys³⁶-(Glut-AOct)-GLP-1(7-36); Val⁸Arg^{26,34}Lys³⁶-(Glut-AOct)-GLP-1 (7-36)amide; Val⁸Arg^{26,34}Lys³⁸-(Glut-AOct)-GLP-1(7-38); Val⁸ Arg^{26,34} Lys³⁹-(Glut-AOct)-GLP-1 (7-39);
Ser⁸Arg^{26,34}Lys³⁶-(Glut-AOct)-GLP-1(7-36); Ser⁸Arg^{26,34}Lys³⁶-(Glut-AOct)-GLP-1 (7-36)amide; Ser⁸Arg^{26,34}Lys³⁸-(Glut-AOct)-GLP-1(7-38); Ser⁸Arg^{26,34}Lys³⁹-(Glut-AOct)-GLP-1 (7-39);
Thr⁸Arg^{26,34}Lys³⁶-(Glut-AOct)-GLP-1(7-36); Thr⁸Arg^{26,34}Lys³⁶-(Glut-AOct)-GLP-1 (7-36)amide; Thr⁸Arg^{26,34}Lys³⁸-(Glut-AOct)-GLP-1(7-38); Thr⁸Arg^{26,34} Lys³⁹-(Glut-AOct)-GLP-1 (7-39);
Gly⁸Glu³⁵Arg^{26,34}Lys³⁶-(Glut-AOct)-GLP-1(7-36); Gly⁸Glu³⁵Arg^{26,34}Lys³⁶-(Glut-AOct)-GLP-1(7-36)amide; Gly⁸Glu³⁶Arg^{26,34}Lys³⁷-(Glut-AOct)-GLP-1(7-37); Gly⁸Glu³⁷Arg^{26,34} Lys³⁸ -(Glut-AOct)-GLP-1 (7-38); Gly⁸Glu³⁸Arg^{26,34}Lys³⁹-(Glut-AOct)-GLP-1 (7-39); Gly⁸Glu³⁵Arg^{26,34}Lys³⁶-(Glut-AOct)-GLP-1 (7-36); Gly⁸Glu³⁵Arg^{26,34}Lys³⁶-(Glut-AOct)-GLP-1 (7-36)amide; Gly⁸Glu³⁶Arg^{26,34}Lys³⁷-(Glut-AOct)-GLP-1 (7-37); Gly⁸Glu³⁷Arg^{26,34}Lys³⁸-(Glut-AOct)-GLP-1 (7-38); Gly⁸Glu³⁸Arg^{26,34}Lys³⁹-(Glut-AOct)-GLP-1 (7-39);
Gly⁸Asp³⁵Arg^{26,34}Lys³⁶-(Glut-AOct)-GLP-1 (7-36); Gly⁸Asp³⁵Arg^{26,34}Lys³⁶-(Glut-AOct)-GLP-1 (7-36)amide; Gly⁸Asp³⁶Arg^{26,34}Lys³⁷-(Glut-AOct)-GLP-1(7-37); Gly⁸Asp³⁷Arg^{26,34}Lys³⁸-(Glut-AOct)-GLP-1 (7-38); Gly⁸Asp³⁸Arg^{26,34}Lys³⁹-(Glut-AOct)-GLP-1 (7-39); Gly⁸Asp³⁵Arg^{26,34}Lys³⁶-(Glut-AOct)-GLP-1 (7-36); Gly⁸Asp³⁵Arg^{26,34}Lys³⁶-(Glut-AOct)-GLP-1 (7-36)amide; Gly⁸Asp³⁶Arg^{26,34}Lys³⁷-(Glut-AOct)-GLP-1 (7-37); Gly⁸Asp³⁷Arg^{26,34}Lys³⁸-(Glut-AOct)-GLP-1 (7-38); Gly⁸Asp³⁸Arg^{26,34}Lys³⁹-(Glut-AOct)-GLP-1 (7-39);
Val⁸Glu³⁵Arg^{26,34}Lys³⁶-(Glut-AOct)-GLP-1 (7-36); Val⁸Glu³⁵Arg^{26,34}Lys³⁶-(Glut-AOct)-GLP-1 (7-36)amide; Val⁸Glu³⁶Arg^{26,34}Lys³⁷-(Glut-AOct)-GLP-1(7-37); Val⁸Glu³⁷Arg^{26,34}Lys³⁸-(Glut-AOct)-GLP-1 (7-38); Val⁸Glu³⁸Arg^{26,34}Lys³⁹-(Glut-AOct)-GLP-1 (7-39); Val⁸Glu³⁵Arg^{26,34}Lys³⁶-(Glut-AOct)-GLP-1 (7-36); Val⁸Glu³⁵Arg^{26,34}Lys³⁶-(Glut-AOct)-GLP-1 (7-36)amide; Val⁸Glu³⁶Arg^{26,34}Lys³⁷-(Glut-AOct)-GLP-1 (7-37); Val⁸Glu³⁷Arg^{26,34}Lys³⁸-(Glut-AOct)-GLP-1 (7-38); Val⁸Glu³⁸Arg^{26,34}Lys³⁹-(Glut-AOct)-GLP-1 (7-39);
Val⁸Asp³⁵Arg^{26,34}Lys³⁶-(Glut-AOct)-GLP-1 (7-36); Val⁸Asp³⁵Arg^{26,34}Lys³⁶-(Glut-AOct)-GLP-1(7-36)amide; Val⁸Asp³⁶Arg^{26,34}Lys³⁷-(Glut-AOct)-GLP-1(7-37); Val⁸Asp³⁷ Arg^{26,34}Lys³⁸-(Glut-AOct)-GLP-1 (7-38); Val⁸Asp³⁸Arg^{26,34}Lys³⁹-(Glut-AOct)-GLP-1 (7-39); Val⁸Asp³⁵Arg^{26,34}Lys³⁶-(Glut-AOct)-GLP-1 (7-36); Val⁸Asp³⁵Arg^{26,34}Lys³⁶-(Glut-AOct)-GLP-1 (7-36)amide; Val⁸Asp³⁶Arg^{26,34}Lys³⁷-(Glut-AOct)-GLP-1 (7-37); Val⁸Asp³⁷Arg^{26,34}Lys³⁸-(Glut-AOct)-GLP-1(7-38); Val⁸Asp³⁸Arg^{26,34}Lys³⁹-(Glut-AOct)-GLP-1 (7-39);
Ser⁸Glu³⁵Arg^{26,34}Lys³⁶-(Glut-AOct)-GLP-1 (7-36); Ser⁸Glu³⁵Arg^{26,34}Lys³⁶-(Glut-AOct)-GLP-1(7-36)amide; Ser⁸Glu³⁶Arg^{26,34}Lys³⁷-(Glut-AOct)-GLP-1(7-37); Ser⁸Glu³⁷Arg^{26,34}Lys³⁸-(Glut-AOct)-GLP-1 (7-38); Ser⁸Glu³⁸Arg^{26,34}Lys³⁹-(Glut-AOct)-GLP-1 (7-39); Ser⁸Glu³⁵Arg^{26,34}Lys³⁶-(Glut-AOct)-GLP-1 (7-36); Ser⁸Glu³⁵Arg^{26,34}Lys³⁶-(Glut-AOct)-GLP-1 (7-36)amide; Ser⁸Glu³⁶ Arg^{26,34}Lys³⁷-(Glut-AOct)-GLP-1 (7-37); Ser⁸Glu³⁷Arg^{26,34} Lys³⁸-(Glut-AOct)-GLP-1 (7-38); Ser⁸Glu³⁸Arg^{26,34}Lys³⁹-(Glut-AOct)-GLP-1(7-39);
Ser⁸ Asp³⁵ Arg^{26,34}Lys³⁶-(Glut-AOct)-GLP-1 (7-36); Ser-⁸Asp³⁵Arg^{26,34}Lys³⁶-(Glut-AOct)-GLP-1 (7-36)amide; Ser-⁸Asp³⁶Arg^{26,34}Lys³⁷-(Glut-AOct)-GLP-1(7-37); Ser⁸Asp³⁷Arg^{26,34}Lys³⁸-(Glut-AOct)-GLP-1 (7-38); Ser⁸Asp³⁸Arg^{26,34}Lys³⁹-(Glut-AOct)-GLP-1(7-39); Ser⁸Asp³⁵Arg^{26,34}Lys³⁶-(Glut-AOct)-GLP-1 (7-36); Ser⁸Asp³⁵Arg^{26,34}Lys³⁶-(Glut-AOct)-GLP-1 (7-36)amide; Ser⁸Asp³⁶Arg^{26,34}Lys³⁷-(Glut-AOct)-GLP-1 (7-37); Ser⁸Asp³⁷Arg^{26,34}Lys³⁸-(Glut-AOct)-GLP-1(7-38); Ser⁸Asp³⁸Arg^{26,34}Lys³⁹-(Glut-AOct)-GLP-1 (7-39);
Thr⁸Glu³⁵Arg^{26,34}Lys³⁶-(Glut-AOct)-GLP-1 (7-36); Thr⁸Glu³⁵Arg^{26,34}Lys³⁶-(Glut-AOct)-GLP-1(7-36)amide; Thr⁸Glu³⁶Arg^{26,34}Lys³⁷-(Glut-AOct)-GLP-1(7-37); Thr⁸Glu³⁷Arg^{26,34}Lys³⁸-(Glut-AOct)-GLP-1 (7-38); Thr⁸Glu³⁸Arg^{26,34}Lys³⁹-(Glut-AOct)-GLP-1 (7-39); Thr⁸Glu³⁵Arg^{26,34}Lys³⁶-(Glut-AOct)-GLP-1 (7-36); Thr⁸Glu³⁵Arg^{26,34}Lys³⁶-(Glut-AOct)-GLP-1 (7-36)amide; Thr⁸Glu³⁶Arg^{26,34}Lys³⁷-(Glut-AOct)-GLP-1 (7-37); Thr⁸Glu³⁷Arg^{26,34}Lys³⁸-(Glut-AOct)-GLP-1 (7-38); Thr⁸Glu³⁸Arg^{26,34} Lys³⁹-(Glut-AOct)-GLP-1 (7-39);
Thr⁸Asp³⁵Arg^{26,34}Lys³⁶-(Glut-AOct)-GLP-1 (7-36); Thr⁸Asp³⁵Arg^{26,34}Lys³⁶-(Glut-AOct)-GLP-1 (7-36)amide; Thr⁸Asp³⁶Arg^{26,34} Lys³⁷-(Glut-AOct)-GLP-1(7-37); Thr⁸Asp³⁷Arg^{26,34}Lys³⁸-(Glut-AOct)-GLP-1 (7-38); Thr⁸Asp³⁸Arg^{26,34}Lys³⁹-(Glut-AOct)-GLP-1 (7-39); Thr⁸Asp³⁵Arg^{26,34}Lys³⁶-(Glut-AOct)-GLP-1 (7-36); Thr⁸Asp³⁵Arg^{26,34}Lys³⁶-(Glut-AOct)-GLP-1 (7-36)amide; Thr⁸Asp³⁶Arg^{26,34}Lys³⁷-(Glut-AOct)-GLP-1 (7-37); Thr⁸Asp³⁷Arg^{26,34}Lys³⁸-(Glut-AOct)-GLP-1 (7-38); Thr⁸Asp³⁸Arg^{26,34}Lys³⁹-(Glut-AOct)-GLP-1 (7-39);
Arg^{26,34}Lys³⁶-(Glut-ALit)-GLP-1(7-36); Arg^{26,34}Lys³⁶-(Glut-ALit)-GLP-1(7-36)amide; Arg²⁶Lys³⁴-(Glut-ALit)-GLP-1 (7-38); Arg^{26,34}Lys³⁹-(Glut-ALit)-GLP-1(7-39);
Gly⁸Arg^{26,34}Lys³⁶-(Glut-ALit)-GLP-1 (7-36); Gly⁸Arg^{26,34}Lys³⁶-(Glut-ALit)-GLP-1(7-36)amide; Gly⁸Arg^{26,34}Lys³⁸-(Glut-ALit)-GLP-1(7-38); Gly⁸Arg^{26,34}Lys³⁹-(Glut-ALit)-GLP-1 (7-39);
Val⁸Arg^{26,34}Lys³⁶-(Glut-ALit)-GLP-1(7-36); Val⁸Arg^{26,34}Lys³⁶-(Glut-ALit)-GLP-1 (7-36)amide; Val⁸Arg^{26,34}Lys³⁸-(Glut-ALit)-GLP-1 (7-38); Val⁸Arg^{26,34}Lys³⁹-(Glut-ALit)-GLP-1 (7-39);
Ser⁸Arg^{26,34}Lys³⁶-(Glut-ALit)-GLP-1(7-36); Ser⁸Arg^{26,34}Lys³⁶-(Glut-ALit)-GLP-1 (7-36)amide; Ser⁸Arg^{26,34}Lys³⁸-(Glut-ALit)-GLP-1 (7-38); Ser⁸Arg^{26,34}Lys³⁹-(Glut-ALit)-GLP-1 (7-39);
Thr⁸Arg^{26,34}Lys³⁶-(Glut-ALit)-GLP-1(7-36); Thr⁸Arg^{26,34}Lys³⁶-(Glut-ALit)-GLP-1 (7-36)amide; Thr⁸Arg^{26,34}Lys³⁸-(Glut-ALit)-GLP-1(7-38); Thr⁸Arg^{26,34}Lys³⁹-(Glut-ALit)-GLP-1 (7-39);
Gly⁸Glu³⁵Arg^{26,34}Lys³⁶-(Glut-ALit)-GLP-1 (7-36); Gly⁸Glu³⁵Arg^{26,34}Lys³⁶-(Glut-ALit)-GLP-1 (7-36)amide; Gly⁸Glu³⁶Arg^{26,34}Lys³⁷-(Glut-ALit)-GLP-1 (7-37); Gly⁸Glu³⁷Arg^{26,34}Lys³⁸-(Glut-ALit)-GLP-1 (7-38); Gly⁸Glu³⁸Arg^{26,34}Lys³⁹-(Glut-ALit)-GLP-1(7-39); Gly⁸Glu³⁵Arg^{26,34}Lys³⁶-(Glut-ALit)-GLP-1 (7-36); Gly⁸Glu³⁵Arg^{26,34}Lys³⁶-(Glut-ALit)-GLP-1(7-36)amide; Gly⁸Glu³⁶Arg^{26,34}Lys³⁷-(Glut-ALit)-GLP-1(7-37); Gly⁸Glu³⁷Arg^{26,34}Lys³⁸-(Glut-ALit)-GLP-1 (7-38); Gly⁸Glu³⁸Arg^{26,34}Lys³⁹-(Glut-_{.} ALit)-GLP-1 (7-39);
Gly⁸Asp³⁵Arg^{26,34}Lys³⁶-(Glut-ALit)-GLP-1 (7-36); Gly⁸Asp³⁵Arg^{26,34}Lys³⁶-(Glut-ALit)-GLP-1 (7-36)amide; Gly⁸Asp³⁶Arg^{26,34}Lys³⁷-(Glut-ALit)-GLP-1(7-37); Gly⁸Asp³⁷Arg^{26,34}Lys³⁸-(Glut-ALit)-GLP-1 (7-38); Gly⁸Asp³⁸Arg^{26,34}Lys³⁹-(Glut-ALit)-GLP-1 (7-39); Gly⁸Asp³⁵Arg^{26,34} Lys³⁶-(Glut-ALit)-GLP-1 (7-36); Gly⁸Asp³⁵Arg^{26,34}Lys³⁶-(Glut-ALit)-GLP-1 (7-36)amide; Gly⁸Asp³⁶Arg^{26,34}Lys³⁷-(Glut-ALit)-GLP-1 (7-37); Gly⁸Asp³⁷Arg^{26,34}Lys³⁸-(Glut-ALit)-GLP-1 (7-38); Gly⁸Asp³⁸Arg^{26,34}Lys³⁹-(Glut=ALit)-GLP-1(7-39);
Val⁸Glu³⁵Arg^{26,34}Lys³⁶-(Glut-ALit)-GLP-1 (7-36); Val⁸Glu³⁵Arg^{26,34}Lys³⁶-(Glut-ALit)-GLP-1 (7-36)amide; Val⁸Glu³⁶Arg^{26,34}Lys³⁷-(Glut-ALit)-GLP-1(7-37); Val⁸Glu³⁷Arg^{26,34}Lys³⁸-(Glut-ALit)-GLP-1 (7-38); Val⁸ Glu³⁸Arg^{26,34}Lys³⁹-(Glut-ALit)-GLP-1(7-39); Val⁸Glu³⁵Arg^{26,34}Lys³⁶-(Glut-ALit)-GLP-1 (7-36); Val⁸Glu³⁵Arg^{26,34}Lys³⁶-(Glut-ALit)-GLP-1 (7-36)amide; Val⁸Glu³⁶Arg^{26,34}Lys³⁷-(Glut-ALit)-GLP-1 (7-37); Val⁸Glu³⁷Arg^{26,34}Lys³⁸-(Glut-ALit)-GLP-1(7-38); Val⁸Glu³⁸Arg^{26,34}Lys³⁹-(Glut-ALit)-GLP-1 (7-39);
Val⁸Asp³⁵Arg^{26,34}Lys³⁶-(Glut-ALit)-GLP-1 (7-36); Val⁸Asp³⁵Arg^{26,34}Lys³⁶-(Glut-ALit)-GLP-1 (7-36)amide; Val⁸Asp³⁶Arg^{26,34}Lys³⁷-(Glut-ALit)-GLP-1(7-37); Val⁸Asp³⁷Arg^{26,34}Lys³⁸-(Glut-ALit)-GLP-1 (7-38); Val⁸Asp³⁸Arg^{26,34}Lys³⁹-(Glut-ALit)-GLP-1 (7-39); Val⁸Asp³⁵Arg^{26,34}Lys³⁶-(Glut-ALit)-GLP-1 (7-36); Val⁸Asp³⁵Arg^{26,34}Lys³⁶-(Glut-ALit)-GLP-1 (7-36)amide; Val⁸Asp³⁶Arg^{26,34}Lys³⁷-(Glut-ALit)-GLP-1 (7-37); Val⁸Asp³⁷Arg^{26,34}Lys³⁸-(Glut-ALit)-GLP-1 (7-38); Val⁸Asp³⁸Arg^{26,34}Lys³⁹-(Glut-ALit)-GLP-1 (7-39);
Ser⁸Glu³⁵Arg^{26,34}Lys³⁶-(Glut-ALit)-GLP-1 (7-36); Ser⁸Glu³⁵Arg^{26,34}Lys³⁶-(Glut-ALit)-GLP-1 (7-36)amide; Ser⁸Glu³⁶Arg^{26,34}Lys³⁷-(Glut-ALit)-GLP-1 (7-37); Ser⁸Glu³⁷Arg^{26,34}Lys³⁸-(Glut-ALit)-GLP-1 (7-38); Ser⁸Glu³⁸Arg^{26,34}Lys³⁹-(Glut-ALit)-GLP-1 (7-39); Ser⁸Glu³⁵Arg^{26,34}Lys³⁶-(Glut-ALit)-GLP-1 (7-36); Ser⁸Glu³⁵Arg^{26,34}Lys³⁶-(Glut-ALit)-GLP-1 (7-36)amide; Ser⁸Glu³⁶Arg^{26,34}Lys³⁷-(Glut-ALit)-GLP-1 (7-37); Ser⁸Glu³⁷Arg^{26,34}Lys³⁸-(Glut-ALit)-GLP-1 (7-38); Ser⁸Glu³⁸Arg^{26,34}Lys³⁹-(Glut-ALit)-GLP-1(7-39);
Ser⁸Asp³⁵Arg^{26,34} Lys³⁶-(Glut-ALit)-GLP-1 (7-36); Ser⁸Asp³⁵Arg^{26,34}Lys³⁶-(Glut-ALit)-GLP-1 (7-36)amide; Ser⁸Asp³⁶Arg^{26,34}Lys³⁷-(Glut-ALit)-GLP-1 (7-37); Ser⁸Asp³⁷Arg^{26,34}Lys³⁸-(Glut-ALit)-GLP-1 (7-38); Ser⁸Asp³⁸Arg^{26,34}Lys³⁹-(Glut-ALit)-GLP-1 (7-39); Ser⁸Asp³⁵Arg^{26,34}Lys³⁶-(Glut-ALit)-GLP-1 (7-36); Ser⁸Asp³⁵Arg^{26,34}Lys³⁶-(Glut-ALit)-GLP-1 (7-36)amide; Ser⁸Asp³⁶ Arg^{26,34}Lys³⁷-(Glut-ALit)-GLP-1 (7-37); Ser⁸Asp³⁷Arg^{26,34}Lys³⁸-(Glut-ALit)-GLP-1 (7-38); Ser⁸Asp³⁸Arg^{26,34}Lys³⁹-(Glut-ALit)-GLP-1 (7-39);
Thr⁸Glu³⁵Arg^{26,34} Lys³⁶-(Glut-ALit)-GLP-1 (7-36); Thr⁸Glu³⁵ Arg^{26,34} Lys³⁶-(Glut-ALit)-GLP-1 (7-36)amide; Thr⁸Glu³⁶Arg^{26,34}Lys³⁷-(Glut-ALit)-GLP-1 (7-37); Thr⁸Glu³⁷Arg^{26,34}Lys³⁸-(Glut-ALit)-GLP-1 (7-38); Thr⁸Glu³⁸Arg^{26,34}Lys³⁹-(Glut-ALit)-G LP-1(7-39); Thr⁸Glu³⁵Arg^{26,34}Lys³⁶-(Glut-ALit)-GLP-1 (7-36); Thr⁸Glu³5 Arg^{26,34}Lys³⁶-(Glut-ALit)-GLP-1 (7-36)amide; Thr⁸Glu³⁶Arg^{26,34}Lys³⁷-(Glut-ALit)-GLP-1 (7-37); Thr⁸Glu³⁷Arg^{26,34}Lys³⁸-(Glut-ALit)-GLP-1(7-38); Thr⁸Glu38Arg^{26,34}Lys³⁹-(Glut-ALit)-GLP-1 (7-39);
Thr⁸Asp³⁵Arg^{26,34}Lys³⁶-(Glut-ALit)-GLP-1 (7-36); Thr⁸Asp³⁵Arg^{26,34}Lys³⁶-(Glut-ALit)-GLP-1(7-36)amide; Thr⁸Asp³⁶Arg^{26,34} Lys³⁷-(Glut-ALit)-GLP-1 (7-37); Thr-⁸Asp³⁷Arg^{26,34}Lys³⁸-(Glut-ALit)-GLP-1 (7-38); Thr⁸ Asp³⁸ Arg^{26,34}Lys³⁹-(Glut-ALit)-GLP-1 (7-39); Thr⁸Asp³⁵Arg^{26,34}Lys³⁶-(Glut-ALit)-GLP-1 (7-36); Thr⁸Asp³⁵Arg^{26,34}Lys³⁶-(Glut-ALit)-GLP-1 (7-36)amide; Thr⁸Asp³⁶Arg^{26,34}Lys³⁷-(Glut-ALit)-GLP-1 (7-37); Thr⁸Asp³⁷Arg^{26,34}Lys³⁸-(Glut-ALit)-GLP-1 (7-38); Thr⁸Asp³⁸Arg^{26,34}Lys³⁹-(Glut-ALit)-GLP-1 (7-39);
Arg^{26,34}Lys³⁶-(Aspa-ADod)-GLP-1 (7-36); Arg^{26,34}Lys³⁶-(Aspa-ADod)-GLP-1 (7-36)amide; ; Arg^{26,34}Lys³⁸-(Aspa-ADod)-GLP-1(7-38); Arg^{26,34}Lys³⁹-(Aspa-ADod)-GLP-1 (7-39);
Gly⁸Arg^{26,34}Lys³⁶-(Aspa-ADod)-GLP-1(7-36); Gly⁸Arg^{26,34}Lys³⁶-(Aspa-ADod)-GLP-1 (7-36)amide; Gly⁸Arg^{26,34} Lys³-(Aspa-ADod)-GLP-1(7-38); Gly⁸Arg^{26,34}Lys³⁹-(Aspa-ADod)-GLP-1 (7-39);
Val⁸Arg^{26,34}Lys³⁶-(Aspa-ADod)-GLP-1 (7-36); Val⁸Arg^{26,34}Lys³⁶-(Aspa-ADod)-GLP-1 (7-36)amide; Val⁸Arg^{26,34}Lys³⁸-(Aspa-ADod)-GLP-1 (7-38); Val⁸Arg^{26,34}Lys³⁹-(Aspa-ADod)-GLP-1 (7-39);
Ser⁸Arg^{26,34}Lys³⁶-(Aspa-ADod)-GLP-1 (7-36); Ser⁸Arg^{26,34}Lys³⁶-(Aspa-ADod)-GLP-1 (7-36)amide; Ser⁸ Arg^{26,34}Lys³⁸-(Aspa-ADod)-GLP-1 (7-38); Ser⁸Arg^{26,34}Lys³⁹-(Aspa-ADod)-GLP-1 (7-39);
Thr⁸ Arg^{26,34}Lys³⁶-(Aspa-ADod)-GLP-1 (7-36); Thr⁸Arg^{26,34}Lys³⁶-(Aspa-ADod)-GLP-1 (7-36)amide; Thr⁸Arg^{26,34}Lys³⁸-(Aspa-ADod)-GLP-1 (7-38); Thr⁸Arg^{26,34} Lys³⁹-(Aspa-ADod)-GLP-1 (7-39); Gly⁸Glu³⁵Arg^{26,34}Lys³⁶-(Aspa-ADod)-GLP-1 (7-36); Gly⁸Glu³⁵Arg^{26,34}Lys³⁶-(Aspa-ADod)-GLP-1 (7-36)amide; Gly⁸Glu³⁶ Arg^{26,34}Lys³⁷-(Aspa-ADod)-GLP-1 (7-37); Gly⁸Glu³⁷Arg^{26,34}Lys³⁸-(Aspa-ADod)-GLP-1 (7-38); Gly⁸Glu³⁸Arg^{26,34}Lys³⁹-(Aspa-ADod)-GLP-1 (7-39); Gly⁸Glu³⁵Arg^{26,34}Lys³⁶-(Aspa-ADod)-GLP-1(7-36); Gly⁸Glu³⁵Arg^{26,34}Lys³⁶-(Aspa-ADod)-GLP-1 (7-36)amide; Gly⁸Glu³⁶Arg^{26,34}Lys³⁷-(Aspa-ADod)-GLP-1 (7-37); Gly⁸Glu³⁷Arg^{26,34}Lys³⁸-(Aspa-ADod)-GLP-1 (7-38); Gly⁸Glu³⁸Arg^{26,34}Lys³⁹-(Aspa-ADod)-GLP-1 (7-39);
Gly⁸Asp-³⁵Arg^{26,34}Lys³⁶-(Aspa-ADod)-GLP-1 (7-36); Gly⁸Asp³⁵Arg^{26,34}Lys³⁶-(Aspa-ADod)-G LP-1 (7-36)amide; Gly⁸Asp³⁶Arg^{26,34}Lys³⁷-(Aspa-ADod)-GLP-1 (7-37); Gly⁸Asp³⁷Arg²⁶,³⁴Lys³⁸-(Aspa-ADod)-GLP-1 (7-38); Gly⁸Asp³⁸Arg^{26,34}Lys³⁹-(Aspa-ADod)-GLP-1 (7-39); Gly⁸Asp³⁵Arg^{26,34}Lys³⁶ - (Aspa-ADod)-GLP-1 (7-36); Gly⁸Asp³⁵Arg^{26,34}Lys³⁶-(Aspa-ADod)-GLP-1 (7-36)amide; Gly⁸Asp³⁶Arg^{26,34}Lys³⁷-(Aspa-ADod)-GLP-1 (7-37); Gly⁸Asp³⁷Arg^{26,34}Lys³⁸-(Aspa-ADod)-GLP-1 (7-38); Gly⁸Asp³⁸Arg^{26,34}Lys³⁹-(Aspa-ADod)-GLP-1 (7-39);
Val⁸Glu³⁵Arg^{26,34}Lys³⁶-(Aspa-ADod)-GLP-1 (7-36); Val⁸Glu³⁵Arg^{26,34}Lys³⁶-(Aspa-ADod)-GLP-1 (7-36)amide; Val⁸Glu³⁶Arg^{26,34}Lys³⁷-(Aspa-ADod)-GLP-1 (7-37); Val⁸Glu³⁷Arg^{26,34}Lys³⁸-(Aspa-ADod)-GLP-1(7-38); Val⁸Glu³⁸Arg^{26,34}Lys³⁹-(Aspa-ADod)-GLP-1 (7-39); Val⁸Glu³⁵Arg^{26,34}Lys³⁶-(Aspa-ADod)-GLP-1 (7-36); Val⁸Glu³⁵Arg^{26,34}Lys³⁶-(Aspa-ADod)-GLP-1 (7-36)amide; Val⁸Glu³⁶Arg^{26,34}Lys³⁷-(Aspa-ADod)-GLP-1(7-37); Val⁸Glu³⁷Arg^{26,38}Lys³⁸-(Aspa-ADod)-GLP-1 (7-38); Val⁸Glu³⁸Arg^{26,34}Lys³⁹-(Aspa-ADod)-GLP-1 (7-39);
Val⁸Asp³⁵Arg²⁶, ³⁴Lys³⁶-(Aspa-ADod)-GLP-1 (7-36); Val⁸Asp³⁵Arg^{26,34} Lys³⁶-(Aspa-ADod)-GLP-1 (7-36)amide; Val⁸Asp³⁶Arg^{26,34}Lys³⁷-(Aspa-ADod)-GLP-1 (7-37); Val⁸Asp³⁷Arg^{26,34}Lys³⁸-(Aspa-ADod)-GLP-1 (7-38); Val⁸Asp³⁸Arg^{26,34}Lys³⁹-(Aspa-ADod)-GLP-1 (7-39); Val⁸Asp-³⁵Arg^{26,34}Lys³⁶-(Aspa-ADod)-GLP-1 (7-36); Val⁸Asp³⁵Arg^{26,34}Lys³⁶-(Aspa-ADod)-GLP-1 (7-36)amide; Val⁸Asp³⁶Arg^{26,34}Lys³⁷-(Aspa-ADod)-GLP-1(7-37); Val⁸Asp³⁷Arg^{26,34}Lys³⁸-(Aspa-ADod)-GLP-1 (7-38); Val⁸Asp³⁸Arg^{26,38}Lys³⁹-(Aspa-ADod)-GLP-1 (7-39);
Ser⁸Glu³⁵Arg^{26,34}Lys³⁶-(Aspa-ADod)-GLP-1 (7-36); Ser⁸Glu³⁵Arg^{26,34}Lys³⁶-(Aspa-ADod)-GLP-1 (7-36)amide; Ser⁸Glu³⁶Arg^{26,34}Lys³⁷-(Aspa-ADod)-GLP-1 (7-37); Ser⁸Glu³⁷Arg^{26,34} Lys³⁸-(Aspa-ADod)-GLP-1(7-38); Ser⁸Glu³⁸Arg^{26,34}Lys³⁹-(Aspa-ADod)-GLP-1 (7-39); Ser⁸Glu³⁵Arg^{26,34}Lys³⁶-(Aspa-ADod)-GLP-1 (7-36); Ser⁸Glu³⁵Arg^{26,34}Lys³⁶-(Aspa-ADod)-GLP-1 (7-36)amide; Ser⁸Glu³⁶Arg^{26,34}Lys³⁷-(Aspa-ADod)-GLP-1 (7-37); Ser⁸Glu³⁷Arg^{26,34}Lys³⁸-(Aspa-ADod)-GLP-1 (7-38); Ser⁸Glu³⁸Arg^{26,34}Lys³⁹-(Aspa-ADod)-GLP-1 (7-39);
Ser⁸Asp³⁵Arg^{26,34}Lys³⁶-(Aspa-ADod)-GLP-1 (7-36); Ser⁸Asp³⁵Arg^{26,34} Lys³⁶-(Aspa-ADod)-GLP-1 (7-36)amide; Ser⁸Asp³⁶Arg^{26,34}Lys³⁷-(Aspa-ADod)-GLP-1(7-37); Ser⁸Asp³⁷Arg^{26,34}Lys³⁸-(Aspa-ADod)-GLP-1 (7-38); Ser⁸Asp³⁸Arg^{26,34}Lys³⁹-(Aspa-ADod)-GLP-1 (7-39); Ser⁸Asp³⁵Arg^{26,34} Lys³⁶-(Aspa-ADod)-GLP-1 (7-36); Ser⁸Asp³⁵Arg^{26,34}Lys³⁶-(Aspa-ADod)-GLP-1(7-36)amide; Ser⁸Asp³⁶Arg^{26,34} Lys³⁷-(Aspa-ADod)-GLP-1 (7-37); Ser⁸Asp³⁷Arg^{26,34}Lys³⁸-(Aspa-ADod)-GLP-1 (7-38); Ser⁸Asp³⁸Arg^{26,34}Lys³⁹-(Aspa-ADod)-GLP-1 (7-39);
Thr⁸Glu³⁵Arg^{26,34}Lys³⁶-(Aspa-ADod)-GLP-1 (7-36); Thr⁸Glu³⁵Arg^{26,34}Lys³⁶-(Aspa-ADod)-GLP-1 (7-36)amide; Thr⁸Glu³⁶Arg^{26,34}Lys³⁷-(Aspa-ADod)-GLP-1(7-37); Thr⁸Glu³⁷Arg^{26,34}Lys³⁸-(Aspa-ADod)-GLP-1 (7-38); Thr⁸Glu³⁸Arg^{26,34}Lys³⁹-(Aspa-ADod)-GLP-1(7-39); Thr⁸Glu³⁵Arg^{26,34}Lys³⁶-(Aspa-ADod)-GLP-1 (7-36); Thr⁸Glu³⁵Arg^{26,34}Lys³⁶-(Aspa-ADod)-GLP-1(7-36)amide; Thr⁸Glu³⁶Arg^{26,34}Lys³⁷-(Aspa-ADod)-GLP-1 (7-37); Thr⁸Glu³⁷Arg^{26,34}Lys³⁸-(Aspa-ADod)-GLP-1(7-38); Thr⁸Glu³⁸Arg^{26,34} Lys³⁹-(Aspa-ADod)-GLP-1 (7-39);
Thr⁸ Asp³⁵ Arg^{26,34} Lys³⁶ -(Aspa-ADod)-GLP-1 (7-36); Thr⁸Asp³⁵ Arg^{26,34} Lys³⁶-(Aspa-ADod)-GLP-1 (7-36)amide; Thr⁸Asp³⁶Arg^{26,34}Lys³⁷-(Aspa-ADod)-GLP-1(7-37); Thr⁸Asp³⁷Arg^{26,34}Lys³⁸-(Aspa-ADod)-GLP-1 (7-38); Thr⁸Asp³⁸Arg^{26,34}Lys³⁹-(Aspa-ADod)-GLP-1 (7-39); Thr⁸Asp³⁵Arg^{26,34}Lys³⁶-(Aspa-ADod)-GLP-1(7-36); The Asp³⁵Arg^{26,34}Lys³⁶-(Aspa-ADod)-GLP-1 (7-36)amide; Thr⁸ Asp³⁶Arg^{26,34}Lys³⁷-(Aspa-ADod)-GLP-1 (7-37); Thr⁸ Asp³⁷Arg^{26,34}Lys³⁸-(Aspa-ADod)-GLP-1 (7-38); Thr⁸Asp³⁸Arg^{26,34}Lys³⁹-(Aspa-ADod)-GLP-1 (7-39);
Arg^{26,34}Lys³⁶-(Aspa-ATet)-GLP-1 (7-36); Arg^{26,34}Lys³⁶-(Aspa-ATet)-GLP-1 (7-36)amide; Arg^{26,34}Lys³⁸-(Aspa-ATet)-GLP-1(7-38); Arg^{26,34}Lys³⁹-(Aspa-ATet)-GLP-1 (7-39);
Gly⁸Arg^{26,34}Lys³⁶-(Aspa-ATet)-GLP-1(7-36); Gly⁸Arg^{26,34}Lys³⁶-(Aspa-ATet)-GLP-1 (7-36)amide; Gly⁸Arg^{26,34}Lys³⁸-(Aspa-ATet)-GLP-1(7-38); Gly⁸Arg^{26,34}Lys³⁹-(Aspa-ATet)-GLP-1 (7-39);
Val⁸Arg^{26,34}Lys³⁶-(Aspa-A Tet)-GLP-1 (7-36); Val⁸Arg^{26,34}Lys³⁶-(Aspa-ATet)-GLP-1 (7-36)amide; Val⁸Arg^{26,34}Lys³⁸-(Aspa-ATet)-GLP-1 (7-38); Val⁸Arg^{26,34}Lys³⁹-(Aspa-ATet)-GLP-1 (7-39);
Ser⁸Arg^{26,34}Lys³⁶-(Aspa-ATet)-GLP-1(7-36); Ser⁸Arg^{26,34}Lys³⁶-(Aspa-ATet)-GLP-1 (7-36)amide; Ser⁸Arg^{26,34}Lys³⁸-(Aspa-ATet)-GLP-1 (7-38); Ser⁸Arg^{26,34}Lys³⁹-(Aspa-ATet)-GLP-1 (7-39);
Thr⁸Arg^{26,34}Lys³⁶-(Aspa-ATet)-GLP-1 (7-36); Thr⁸Arg^{26,34}Lys³⁶-(Aspa-ATet)-GLP-1 (7-36)amide; Thr⁸Arg^{26,34}Lys³⁸-(Aspa-ATet)-GLP-1 (7-38); Thr⁸Arg^{26,34} Lys³⁹-(Aspa-ATet)-GLP-1 (7-39);
Gly⁸Glu35 Arg^{26,34} Lys³⁶-(Aspa-ATet)-GLP-1 (7-36); Gly⁸Glu³⁵Arg^{26,34}Lys³⁶-(Aspa-ATet)-GLP-1(7-36)amide; Gly⁸Glu³⁶Arg^{26,34}Lys³⁷-(Aspa-ATet)-GLP-1 (7-37); Gly⁸Glu³⁷Arg^{26,34}Lys³⁸-(Aspa-ATet)-GLP-1(7-38); Gly⁸Glu³⁸Arg^{26,34}Lys³⁹-(Aspa-ATet)-GLP-1(7-39); Gly⁸Glu³⁵Arg^{26,34}Lys³⁶-(Aspa-ATet)-GLP-1(7-36); Gly⁸Glu³⁵Arg^{26,34}Lys³⁶-(Aspa-ATet)-GLP-1 (7-36)amide; Gly⁸Glu³⁶Arg^{26,34}Lys³⁷-(Aspa-ATet)-GLP-1(7-37); Gly⁸Glu³⁷Arg^{26,34}Lys³⁸-(Aspa-ATet)-GLP-1 (7-38); Gly⁸Glu³⁸Arg^{26,34}Lys³⁹-(Aspa-ATet)-GLP-1 (7-39);
Gly⁸Asp³⁵Arg^{26,34}Lys³⁶-(Aspa-ATet)-GLP-1(7-36); Gly Asp³5 Arg^{26,34}Lys³⁶-(Aspa-ATet)-GLP-1(7-36)amide; Gly⁸Asp³⁶Arg^{26,34}Lys³⁷-(Aspa-ATet)-GLP-1 (7-37); Gly⁸ Asp³⁷Arg^{26,34} Lys³⁸-(Aspa-ATet)-GLP-1 (7-38); Gly⁸Asp³⁸Arg^{26,34}Lys³⁹-(Aspa-ATet)-GLP-1(7-39); Gly⁸Asp³⁵Arg^{26,34}Lys³⁶-(Aspa-ATet)-GLP-1 (7-36); Gly⁸ Asp³⁵Arg^{26,34}Lys³⁶-(Aspa-ATet)-GLP-1 (7-36)amid; Gly⁸Asp³⁶Arg^{26,34}Lys³⁷(Aspa-ATet)-GLP-1(7-37); Gly⁸Asp³⁷Arg^{26,34}Lys³⁸-(Aspa-ATet)-GLP-1 (7-38); Gly⁸Asp³⁸Arg^{26,34}Lys³⁹-(Aspa-ATet)-GLP-1(7-39);
Val⁸Glu³⁵Arg^{26,34}Lys³⁶-(Aspa-ATet)-GLP-1(7-36); Val⁸Glu³⁵Arg^{26,34}Lys³⁶-(Aspa-ATet)-GLP-1 (7-36)amide; Val⁸Glu³⁶Arg^{26,34}Lys³⁷-(Aspa-ATet)GLP-1(7-37); Val⁸Glu³⁷Arg^{26,34}Lys³⁸-(Aspa-ATet)-GLP-1 (7-38); Val⁸Glu³⁸Arg^{26,34}Lys³⁹-(Aspa-ATet)GLP-1(7-39); Val⁸Glu³⁵Arg^{26,34}Lys³⁶-(Aspa-ATet)-GLP-1 (7-36); Val⁸Glu³⁵Arg^{26,34}Lys³⁶-(Aspa-ATet)-GLP-1 (7-36)amide; Val⁸Glu³⁶Arg^{26,34}Lys³⁷-(Aspa-ATet)-GLP-1(7-37); Val⁸Glu³⁷Arg^{26,34}Lys³⁸-(Aspa-ATet)-GLP-1(7-38); Val⁸Glu³⁸Arg^{26,34}Lys³⁹-(Aspa-ATet)-GLP-1(7-39);
Val⁸Asp³⁵Arg^{26,34} Lys³⁶-(Aspa-ATet)-GLP-1(7-36); Val⁸Asp³⁵Arg^{26,34}Lys³⁶-(Aspa-ATet)-GLP-1 (7-36)amide; Val⁸Asp³⁶Arg^{26,34}Lys³⁷-(Aspa-ATet)-GLP-1 (7-37); Val⁸Asp³⁷Arg^{26,34}Lys³⁸-(Aspa-ATet)-GLP-1 (7-38); Val⁸Asp³⁸Arg^{26,34}Lys³⁹-(Aspa-A Tet)-GLP-1 (7-39); Val⁸Asp³⁵Arg^{26,34}Lys³⁶-(Aspa-ATet)-GLP-1(7-36); Val⁸Asp³⁵Arg^{26,34}Lys³⁶-(Aspa-A Tet)-GLP-1 (7-36)amide; Val⁸Asp³⁶Arg^{26,34}Lys³⁷-(Aspa-ATet)-GLP-1(7-37); Val⁸Asp³⁷Arg^{26,34}Lys³⁸-(Aspa-ATet)-GLP-1 (7-38); Val⁸Asp³⁸Arg^{26,34}Lys³⁹-(Aspa-ATet)-GLP-1 (7-39);
Ser⁸Glu³⁵ Arg^{26,34} Lys³⁶-(Aspa-ATet)-GLP-1 (7-36); Ser⁸Glu³⁵Arg^{26,34}Lys³⁶-(Aspa-ATet)-GLP-1 (7-36)amide; Ser⁸Glu³⁶Arg^{26,34}Lys³⁷-(Aspa-ATet)-GLP-1 (7-37); Ser⁸Glu³⁷Arg^{26,34}Lys³⁸-(Aspa-ATet)-GLP-1 (7-38); Ser⁸Glu³⁸Arg^{26,34}Lys³⁹-(Aspa-ATet)-GLP-1(7-39); Ser⁸Glu³⁵Arg^{26,34}Lys³⁶-(Aspa-ATet)-GLP-1 (7-36); Ser⁸Glu³⁵Arg^{26,34}Lys³⁶-(Aspa-ATet)-GLP-1(7-36)amide; Ser⁸Glu³⁶Arg^{26,34}Lys³⁷-(Aspa-ATet)-GLP-1(7-37); Ser⁸Glu³⁷Arg^{26,34}Lys³⁸-(Aspa-ATet)-GLP.; (7-38); Ser⁸Glu³⁸Arg^{26,34}Lys³⁹-(Aspa-ATet)-GLP-1 (7-39);
Ser⁸Asp³⁵Arg^{26,34} Lys³⁶-(Aspa-ATet)-GLP-1 (7-36); Ser⁸Asp³⁵Arg^{26,34}Lys³⁶-(Aspa-A Tet)-GLP-1 (7-36)amide; Ser⁸Asp³⁶Arg^{26,34}Lys³⁷-(Aspa-ATet)-GLP-1 (7-37); Ser⁸Asp³⁷Arg^{26,34}Lys³⁸-(Aspa-ATet)-GLP-1 (7-38); Ser⁸Asp³⁸Arg^{26,34}Lys³⁹-(Aspa-ATet)-GLP-1 (7-39); Ser⁸Asp³⁵Arg^{26,34}Lys³⁶-(Aspa-ATet)-GLP-1 (7-36); Ser⁸Asp³⁵Arg^{26,34}Lys³⁶-(Aspa-ATet)-GLP-1(7-36)amide; Ser⁸Asp³⁶Arg^{26,34}Lys³⁷-(Aspa-ATet)-GLP-1 (7-37); Ser⁸ Asp³⁷Arg^{26,34}Lys³⁸-(Aspa-ATet)-GLP-1 (7-38); Ser-⁸Asp³⁸Arg^{26,34}Lys³⁹-(Aspa-ATet)-GLP-1 (7-39);
Thr⁸Glu³⁵Arg^{26,34}Lys³⁶-(Aspa-ATet)-GLP-1(7-36); Thr⁸Glu³⁵Arg^{26,34}Lys³⁶-(Aspa-ATet)-GLP-1 (7-36)amide; Thr⁸Glu³⁶Arg^{26,34}Lys³⁷-(Aspa-ATet)-GLP-1(7-37); Thr⁸Glu³⁷Arg^{26,34}Lys³⁸-(Aspa-A Tet)-GLP-1 (7-38); Thr⁸Glu³⁸Arg^{26,34}Lys³⁹-(Aspa-ATet)-GLP-1(7-39); Thr⁸Glu³⁵Arg^{26,34}Lys³⁶-(Aspa-ATet)-GLP-1 (7-36); Thr⁸Glu³⁵Arg^{26,34}Lys³⁶-(Aspa-ATet)-GLP-1(7-36)amide; Thr⁸Glu³⁶Arg^{26,34}Lys³⁷-(Aspa-ATet)-GLP-1 (7-37); Thr⁸Glu³⁷Arg^{26,34}Lys³⁸-(Aspa-A Tet)-GLP-1 (7-38); Thr⁸Glu³⁸Arg^{26,34}Lys³⁹-(Aspa-ATet)-GLP-1 (7-39);
Thr⁸Asp³⁵Arg^{26,34}Lys³⁶-(Aspa-ATet)-GLP-1 (7-36); Thr⁸Asp³⁵Arg^{26,34}Lys³⁶-(Aspa-A Tet)-GLP-1 (7-36)amide; Thr⁸Asp³⁶Arg^{26,34}Lys³⁷-(Aspa-ATet)-GLP-1 (7-37); Thr⁸Asp³⁷Arg^{26,34}Lys³⁸-(Aspa-ATet)-GLP-1 (7-38); Thr⁸Asp³⁸Arg^{26,34}Lys³⁹-(Aspa-ATet)-GLP-1 (7-39); Thr⁸Asp³⁵Arg^{26,34}Lys³⁶-(Aspa-ATet)-GLP-1 (7-36); Thr⁸Asp³⁵Arg^{26,34}Lys³⁶-(Aspa-ATet)-GLP-1 (7-36)amide; Thr⁸Asp³⁶Arg^{26,34}Lys³⁷-(Aspa-ATet)-GLP-1 (7-37); Thr⁸Asp³⁷Arg^{26,34} Lys³⁸-(Aspa-ATet)-GLP-1 (7-38); Thr⁸Asp³⁸Arg^{26,34}Lys³⁹-(Aspa-ATet)-GLP-1 (7-39);
Arg^{26,34}Lys³⁶-(Aspa-AHex)-GLP-1 (7-36); Arg^{26,34}Lys³⁶-(Aspa-AHex)-GLP-1 (7-36)amide; Arg^{26,34} Lys³⁸-(Aspa-AHex)-GLP-1 (7-38); Arg^{26,34}Lys³⁹-(Aspa-AHex)-GLP-1 (7-39);
Gly⁸Arg^{26,34}Lys³⁶-(Aspa-AHex)-GLP-1 (7-36); Gly⁸Arg^{26,34}Lys³⁶-(Aspa-AHex)-GLP-1 (7-36)amide; Gly⁸Arg^{26,34}Lys³⁸-(Aspa-AHex)-GLP-1(7-38); Gly⁸Arg^{26,34} Lys³⁹-(Aspa-AHex)-GLP-1(7-39);
Val⁸Arg^{26,34}Lys³⁶-(Aspa-AHex)-GLP-1(7-36); Val⁸Arg^{26,34}Lys³⁶-(Aspa-AHex)-GLP-1(7-36)amide; Val⁸Arg^{26,34}Lys³⁸-(Aspa-AHex)-GLP-1(7-38); Val⁸Arg^{26,34}Lys³⁹-(Aspa-AHex)-GLP-1 (7-39);
Ser⁸Arg^{26,34}Lys³⁶-(Aspa-AHex)-GLP-1(7-36); Ser⁸Arg^{26,34}Lys³⁶-(Aspa-AHex)-GLP-1 (7-36)amide; Ser⁸Arg²⁶Lys³⁸-(Aspa-AHex)-GLP-1(7-38); Ser⁸Arg^{26,34}Lys³⁹-(Aspa-AHex)-GLP-1 (7-39);
Thr⁸Arg^{26,34}Lys³⁶-(Aspa-AHex)-GLP-1(7-36); Thr⁸Arg^{26,34}Lys³⁶-(Aspa-AHex)-GLP-1 (7-36)amide; Thr⁸Arg^{26,34}Lys³⁸-(Aspa-AHex)-GLP-1(7-38); Thr⁸Arg^{26,34}Lys³⁹-(Aspa-AHex)-GLP-1 (7-39);
Gly⁸Glu³⁵Arg^{26,34}Lys³⁶-(Aspa-AHex)-GLP-1(7-36); Gly⁸Glu³⁵Arg^{26,34}Lys³⁶-(Aspa-AHex)-GLP-1 (7-36)amide; Gly⁸Glu³⁶Arg^{26,34}Lys³⁷-(Aspa-AHex)-GLP-1 (7-37); Gly⁸Glu³⁷Arg^{26,34}Lys³⁸-(Aspa-AHex)-GLP-1 (7-38); Gly⁸Glu³⁸Arg^{26,34}Lys³⁹-(Aspa-AHex)-GLP-1(7-39); Gly⁸Glu³⁵Arg^{26,34}Lys³⁶-(Aspa-AHex)-GLP-1 (7-36); Gly⁸Glu³⁵Arg^{26,34}Lys³⁶-(Aspa-AHex)-GLP-1(7-36)amide; Gly⁸Glu³⁶Arg^{26,34} Lys³⁷-(Aspa-AHex)-GLP-1 (7-37); Gly⁸Glu³⁷Arg^{26,34} Lys³⁸-(Aspa-AHex)-GLP-1 (7-38); Gly⁸Glu³⁸Arg^{26,34}Lys³⁹-(Aspa-AHex)-GLP-1 (7-39);
Gly⁸Asp³⁵Arg^{26,34}Lys³⁶-(Aspa-AHex)-GLP-1 (7-36); Gly⁸Asp³⁵Arg^{26,34}Lys³⁶-(Aspa-AHex)-GLP-1 (7-36)amide; Gly⁸Asp³⁶Arg^{26,34} Lys³⁷-(Aspa-AHex)-GLP-1 (7-37); Gly⁸Asp³⁷Arg^{26,34}Lys³⁸-(Aspa-AHex)-GLP-1 (7-38); Gly⁸Asp³⁸Arg^{26,34}Lys³⁹-(Aspa-AHex)-GLP-1(7-39); Gly⁸Asp³⁵Arg^{26,34}Lys³⁶-(Aspa-AHex)-GLP-1(7-36); Gly⁸Asp³⁵Arg^{26,34}Lys³⁶-(Aspa-AHex)-GLP-1 (7-36)amide; Gly⁸Asp³⁶Arg^{26,34}Lys³⁷-(Aspa-AHex)-GLP-1 (7-37); Gly⁸Asp³⁷Arg^{26,34}Lys³⁸-(Aspa-AHex)-GLP-1 (7-38); Gly⁸Asp³⁸Arg^{26,34}Lys³⁹-(Aspa-AHex)-GLP-1 (7-39);
Val⁸Glu³⁵Arg^{26,34}Lys³⁶-(Aspa-AHex)-GLP-1(7-36); Val⁸Glu³⁵Arg^{26,34}Lys³⁶-(Aspa-AHex)-GLP-1 (7-36)amide; Val⁸Glu³⁶Arg^{26,34}Lys³⁷-(Aspa-AHex)-GLP-1 (7-37); Val⁸Glu³⁷Arg^{26,34}Lys³⁸-(Aspa-AHex)-GLP-1 (7-38); Val⁸Glu³⁸Arg^{26,34}Lys³⁹-(Aspa-AHex)-GLP-1(7-39); Val⁸Glu³⁵Arg^{26,34}Lys³⁶-(Aspa-AHex)-GLP-1(7-36); Val⁸Glu³⁵Arg^{26,34}Lys³⁶-(Aspa-AHex)-GLP-1(7-36)amide; Val⁸ Glu³⁶Arg^{26,34}Lys³⁷-(Aspa-AHex)-GLP-1 (7-37); Val⁸Glu³⁷Arg^{26,34}Lys³⁸-(Aspa-AHex)-GLP-1 (7-38); Val⁸Glu³⁸Arg^{26,34}Lys³⁹-(Aspa-AHex)-GLP-1 (7-39);
Val⁸Asp³⁵Arg^{26,34}Lys³⁶-(Aspa-AHex)-GLP-1(7-36); Val⁸Asp³⁵Arg^{26,34} Lys³⁶-(Aspa-AHex)-GLP-1 (7-36)amide; Val⁸Asp³⁶Arg^{26,34}Lys³⁷-(Aspa-AHex)-GLP-1 (7-37); Val⁸ Asp³⁷Arg^{26,34}Lys³⁸-(Aspa-AHex)-GLP-1 (7-38); Val⁸Asp³⁸Arg^{26,34}Lys³⁹-(Aspa-AHex)-GLP-1(7-39); Val⁸Asp³⁵Arg^{26,34}Lys³⁶-(Aspa-AHex)-GLP-1 (7-36); Val⁸ Asp³⁵Arg^{26,34}Lys³⁶-(Aspa-AHex)-GLP-1(7-36)amide; Val⁸Asp³⁶Arg^{26,34} Lys³⁷-(Aspa-AHex)-GLP-1 (7-37); Val⁸ Asp³⁷ Arg^{26,34} Lys³⁸-(Aspa-AHex)-GLP-1 (7-38); Val⁸Asp³⁸Arg^{26,34}Lys³⁹-(Aspa-AHex)-GLP-1 (7-39);
Ser⁸Glu³⁵Arg^{26,34} Lys³⁶-(Aspa-AHex)-GLP-1(7-36); Ser⁸Glu³⁵ Arg^{26,34}Lys³⁶-(Aspa-AHex)-GLP-1 (7-36)amide; Ser⁸Glu³⁶Arg^{26,34}Lys³⁷-(Aspa-AHex)-GLP-1(7-37); Ser⁸Glu³⁷Arg^{26,34}Lys³⁸-(Aspa-AHex)-GLP-1 (7-38); Ser⁸Glu³⁸Arg^{26,34} Lys³⁹-(Aspa-AHex)-GLP-1(7-39); Ser⁸Glu³⁵Arg^{26,34}Lys³⁶-(Aspa-AHex)-GLP-1(7-36); Ser⁸Glu³⁵Arg^{26,34} Lys³⁶-(Aspa-AHex)-GLP-1 (7-36)amide; Ser⁸Glu³⁶ Arg^{26,34} Lys³⁷-(Aspa-AHex)-GLP-1(7-37); Ser⁸Glu³⁷Arg^{26,34}Lys³⁸-(Aspa-AHex)-GLP-1 (7-38); Ser⁸Glu³⁸Arg^{26,34}Lys³⁹-(Aspa-AHex)-GLP-1 (7-39);
Ser⁸Asp³⁵Arg^{26,34}Lys³⁶-(Aspa-AHex)-GLP-1 (7-36); Ser⁸Asp³⁵Arg^{26,34}Lys³⁶-(Aspa-AHex)-GLP-1 (7-36)amide; Ser⁸Asp³⁶Arg^{26,34}Lys³⁷-(Aspa-AHex)-GLP-1(7-37); Ser⁸ Asp³⁷Arg^{26,34}Lys³⁸-(Aspa-AHex)-GLP-1 (7-38); Ser⁸Asp³⁸Arg^{26,34}Lys³⁹-(Aspa-AHex)-GLP-1 (7-39); Ser⁸Asp³⁵Arg^{26,3}4Lys³⁶-(Aspa-AHex)-GLP-1(7-36); Ser⁸Asp³⁵Arg^{26,34}Lys³⁶-(Aspa-AHex)-GLP-1 (7-36)amide; Ser⁸Asp³⁶Arg^{26,34}Lys³⁷-(Aspa-AHex)-GLP-1 (7-37); Ser⁸Asp³⁷Arg^{26,34}Lys³⁸-(Aspa-AHex)-GLP-1 (7-38); Ser⁸Asp³⁸Arg^{26,34}Lys³⁹-(Aspa-AHex)-GLP-1 (7-39);
Thr⁸Glu³⁵Arg^{26,34}Lys³⁶-(Aspa-AHex)-GLP-1(7-36); Thr⁸Glu³⁵Arg^{26,34}Lys³⁶-(Aspa-AHex)-GLP-1 (7-36)amide; Thr⁸Glu³⁶Arg^{26,34}Lys³⁷-(Aspa-AHex)-GLP-1(7-37); Thr⁸Glu³⁷Arg^{26,34}Lys³⁸-(Aspa-AHex)-GLP-1 (7-38); Thr⁸Glu³⁸Arg^{26,34}Lys³⁹-(Aspa-AHex)-GLP-1(7-39); Thr⁸Glu³⁵Arg^{26,34}Lys³⁶-(Aspa-AHex)-GLP-1 (7-36); Thr⁸Glu³⁵Arg^{26,34}Lys³⁶-(Aspa-AHex)-GLP-1 (7-36)amide; Thr⁸Glu³⁶Arg^{26,34}Lys³⁷-(Aspa-AHex)-GLP-1 (7-37); Thr⁸Glu³⁷Arg^{26,34}Lys³⁸-(Aspa-AHex)-GLP-1 (7-38); Thr⁸Glu³⁸Arg^{26,34}Lys³⁹-(Aspa-AHex)-GLP-1 (7-39);
Thr⁸ Asp³⁵Arg^{26,34}Lys³⁶-(Aspa-AHex)-GLP-1(7-36); Thr⁸Asp³⁵Arg^{26,34}Lys³⁶-(Aspa-AHex)-GLP-1(7-36)amide; Thr⁸Asp³⁶Arg^{26,34}Lys³⁷-(Aspa-AHex)-GLP-1(7-37); Thr⁸Asp³⁷Arg^{26,34}Lys³⁸-(Aspa-AHex)-GLP-1 (7-38); Thr⁸Asp³⁸Arg^{26,34}Lys³⁹-(Aspa-AHex)-GLP-1(7-39); Thr⁸Asp³⁵Arg^{26,34}Lys³⁶-(Aspa-AHex)-GLP-1 (7-36); Thr⁸Asp³⁵Arg^{26,34} Lys³⁶-(Aspa-AHex)-GLP-1 (7-36)amide; Thr⁸Asp³⁶Arg^{26,34} Lys³⁷ -(Aspa-AHex)-GLP-1 (7-37); Thr⁸Asp³⁷Arg^{26,34}Lys³⁸-(Aspa-AHex)-GLP-1 (7-38); Thr⁸Asp³⁸Arg^{26,34}Lys³⁹-(Aspa-AHex)-GLP-1(7-39);
Arg^{26,34}Lys³⁶-(Aspa-AOct)-GLP-1 (7-36); Arg^{26,34}Lys³⁶-(Aspa-AOct)-GLP-1(7-36)amide; Arg^{26,34}Lys³⁸-(Aspa-AOct)-GLP-1(7-38); Arg^{26,34}Lys³⁹-(Aspa-AOct)-GLP-1(7-39);
Gly⁸Arg^{26,34}Lys³⁶-(Aspa-AOct)-GLP-1(7-36); Gly⁸Arg^{26,34}Lys³⁶-(Aspa-AOct)-GLP-1(7-36)amide; Gly⁸Arg^{26,34} Lys³⁸-(Aspa-AOct)-GLP-1 (7-38); Gly⁸Arg^{26,34}Lys³⁹-(Aspa-AOct)-GLP-1 (7-39);
Val⁸Arg^{26,34}Lys³⁶-(Aspa-AOct)-GLP-1 (7-36); Val⁸Arg^{26,34}Lys³⁶-(Aspa-AOct)-GLP-1 (7-36)amide; Val⁸Arg^{26,34}Lys³⁸-(Aspa-AOct)-GLP-1 (7-38); Val⁸Arg^{26,34}Lys³⁹-(Aspa-AOct)-GLP-1 (7-39);
Ser⁸Arg^{26,34}Lys³⁶-(Aspa-AOct)-GLP-1(7-36); Ser⁸Arg^{26,34}Lys³⁶-(Aspa-AOct)-GLP-1 (7-36)amide; Ser⁸Arg^{26,34}Lys-³⁸-(Aspa-AOct)-GLP-1(7-38); Ser⁸Arg^{26,34}Lys³⁹-(Aspa-AOct)-GLP-1 (7-39);
Thr⁸Arg^{26,34}Lys³⁶-(Aspa-AOct)-GLP-1 (7-36); Thr⁸Arg^{26,34}Lys³⁶-(Aspa-AOct)-GLP-1(7-36)amide; Thr⁸Arg^{26,34}Lys³⁸-(Aspa-AOct)-GLP-1 (7-38); Thr⁸Arg^{26,34}Lys³⁹-(Aspa-AOct)-GLP-1(7-39);
Gly⁸Glu³⁵Arg^{26,34}Lys³⁶-(Aspa-AOct)-GLP-1(7-36); Gly⁸Glu³⁵Arg^{26,34}Lys³⁶-(Aspa-AOct)-GLP-1 (7-36)amide; Gly⁸Glu³⁶Arg^{26,34}Lys³⁷-(Aspa-AOct)-GLP-1 (7-37); Gly⁸Glu³⁷Arg^{26,34} Lys³⁸-(Aspa-AOct)-GLP-1 (7-38); Gly⁸Glu³⁸Arg^{26,34}Lys³⁹-(Aspa-AOct)-GLP-1(7-39); Gly⁸Glu³⁵Arg^{26,34}Lys³⁶-(Aspa-AOct)-GLP-1 (7-36); Gly⁸Glu³⁵Arg^{26,34}Lys³⁶-(Aspa-AOct)-GLP-1 (7-36)amide; Gly⁸Glu³⁶Arg^{26,34}Lys³⁷-(Aspa-AOct)-GLP-1(7-37); Gly⁸Glu³⁷Arg^{26,34}Lys³⁸-(Aspa-AOct)-GLP-1(7-38); Gly⁸Glu³⁸Arg^{26,34}Lys³⁹-(Aspa-AOct)-GLP-1 (7-39);
Gly⁸Asp³⁵Arg^{26,34}Lys³⁶-(Aspa-AOct)-GLP-1(7-36); Gly⁸Asp³⁵Arg^{26,34}Lys³⁶-(Aspa-AOct)-GLP-1(7-36)amide; Gly⁸Asp³⁶Arg^{26,34}Lys³⁷-(Aspa-AOct)-GLP-1 (7-37); Gly⁸Asp³⁷Arg^{26,34}Lys³⁸-(Aspa-AOct)-GLP-1 (7-38); Gly⁸Asp³⁸Arg^{26,34}Lys³⁹-(Aspa-AOct)-GLP-1(7-39); Gly⁸Asp³⁵Arg^{26,34} Lys³⁶-(Aspa-AOct)-GLP-1 (7-36); Gly⁸Asp³⁵Arg^{26,34}Lys³⁶-(Aspa-AOct)-GLP-1 (7-36)amide; Gly⁸Asp³⁶Arg^{26,34}Lys³⁷-(Aspa-AOct)-GLP-1(7-37); Gly⁸ Asp³⁷Arg^{26,34}Lys³⁸-(Aspa-AOct)-GLP-1 (7-38); Gly⁸Asp³⁸Arg^{26,34}Lys³⁹-(Aspa-AOct)-GLP-1(7-39);
Val⁸Glu³⁵Arg^{26,34}Lys³⁶-(Aspa-AOct)-GLP-1 (7-36); Val⁸Glu³⁵Arg^{26,34}Lys³⁶-(Aspa-AOct)-GLP-1 (7-36)amide; Val⁸Glu³⁶Arg^{26,34} Lys³⁷-(Aspa-AOct)-GLP-1 (7-37); Val⁸Glu³⁷Arg^{26,34}Lys³⁸-(Aspa-AOct)-GLP-1(7-38); Val⁸Glu³⁸Arg^{26,34}Lys³⁹-(Aspa-AOCt)-GLP-1(7-39); Val⁸Glu³⁵Arg^{26,34}Lys³⁶-(As pa-AOct)-GLP-1 (7-36); Val⁸Glu³⁵Arg^{26,34}Lys³⁶-(Aspa-AOct)-GLP-1 (7-36)amide; Val⁸ Glu³⁶Arg^{26,34}Lys³⁷-(Aspa-AOct)-GLP-1(7-37); Val⁸Glu³⁷Arg^{26,34}Lys³⁸-(Aspa-AOct)-GLP-1 (7-38); Val⁸ Glu³⁸Arg^{26,34}Lys³⁹-(Aspa-AOct)-GLP-1 (7-39);
Val⁸Asp³⁵Arg^{26,34} Lys³⁶-(Aspa-AOct)-GLP-1 (7-36); Val⁸Asp³⁵Arg^{26,34}Lys³⁶-(Aspa-AOct)-GLP-1 (7-36)amide; Val⁸Asp³⁶Arg^{26,34}Lys³⁷-(Aspa-AOct)-GLP-1 (7-37); Val⁸ Asp³⁷Arg^{26,34}Lys³⁸-(Aspa-AOct)-GLP-1 (7-38); Val⁸Asp³⁸Arg^{26,34}Lys³⁹-(Aspa-AOct)-GLP-1(7-39); Val⁸Asp³⁵Arg^{26,34}Lys³⁶-(Aspa-AOct)-GLP-1 (7-36); Val⁸Asp³⁵Arg^{26,34}Lys³⁶-(Aspa-AOct)-GLP-1 (7-36)amide; Val⁸Asp³⁶Arg^{26,34}Lys³⁷-(Aspa-AOct)-GLP-1(7-37); Val⁸Asp³⁷Arg^{26,34} Lys³⁸-(Aspa-AOct)-GLP-1 (7-38); Val⁸Asp³⁸ Arg^{26,34} Lys³⁹-(Aspa-AOct)-GLP-1 (7-39);
Ser⁸Glu³⁵Arg^{26,34} Lys³⁶-(Aspa-AOct)-GLP-1 (7-36); Ser⁸Glu³⁵ Arg^{26,34}Lys³⁶-(Aspa-AOct)-GLP-1 (7-36)amide; Ser⁸Glu³⁶Arg^{26,34} Lys³⁷-(Aspa-AOct)-GLP-1 (7-37); Ser⁸Glu³⁷Arg^{26,34}Lys³⁸-(Aspa-AOct)-GLP-1(7-38); Ser⁸Glu³⁸ Arg^{26,34}Lys³⁹-(Aspa-AOct)-GLP-1(7-39); Ser⁸Glu³⁵Arg^{26,34}Lys³⁶-(Aspa-AOct)-GLP-1 (7-36); Ser⁸Glu³⁵Arg^{26,34}Lys³⁶-(Aspa-AOct)-GLP-1(7-36)amide; Ser⁸Glu³⁶Arg^{26,34}Lys³⁷-(Aspa-AOct)-GLP-1(7-37); Ser⁸Glu³⁷Arg^{26,34}Lys³⁸-(Aspa-AOct)-GLP-1 (7-38); Ser⁸Glu³⁸ Arg^{26,34}Lys³⁹-(Aspa-AOct)-GLP-1 (7-39);
Ser⁸Asp³⁵Arg^{26,34}Lys³⁶-(Aspa-AOct)-GLP-1 (7-36); Ser⁸ Asp³⁵Arg^{26,34} Lys³⁶-(Aspa-AOct)-GLP-1 (7-36)amide; Ser⁸Asp³⁶Arg^{26,34}Lys³⁷-(Aspa-AOct)-GLP-1 (7-37); Ser⁸Asp³⁷Arg^{26,34}Lys³⁸-(Aspa-AOct)-GLP-1(7-38); Ser⁸Asp³⁸Arg^{26,34}Lys³⁹-(Aspa-AOct)-GLP-1(7-39); Ser⁸Asp³⁵Arg^{26,34}Lys³⁶-(Aspa-AOct)-GLP-1 (7-36); Ser⁸ Asp³⁵ Arg^{26,34}Lys³⁶-(Aspa-AOct)-GLP-1 (7-36)amide; Ser⁸ Asp³⁶Arg^{26,34}Lys³⁷-(Aspa-AOct)-GLP-1 (7-37); Ser⁸ Asp³⁷Arg^{26,34}Lys³⁸-(Aspa-AOct)-GLP-1 (7-38); Ser⁸Asp³⁸Arg^{26,34}Lys³⁹-(Aspa-AOct)-GLP-1(7-39);
Thr⁸Glu³⁵Arg^{26,34} Lys³⁶-(Aspa-AOct)-GLP-1(7-36); Thr⁸Glu³⁵Arg^{26,34}Lys³⁶-(Aspa-AOct)-GLP-1 (7-36)amide; Thr⁸Glu³⁶Arg^{26,34} Lys³⁷-(As pa-AOct)-G LP- (7-37); Thr⁸Glu³⁷Arg^{26,34} Lys³⁸-(Aspa-AOct)-GLP-1(7-38); Thr⁸Glu³⁸Arg^{26,34}Lys³⁹-(Aspa-AOct)-GLP-1(7-39); Thr⁸Glu³⁵Arg^{26,34}Lys³⁶-(Aspa-AOct)-GLP-1 (7-36); Thr⁸Glu³⁵Arg^{26,34}Lys³⁶-(Aspa-AOct)-GLP-1(7-36)amide; Thr⁸Glu³⁶Arg^{26,34}Lys³⁷-(Aspa-AOct)-GLP-1(7-37); Thr⁸Glu³⁷Arg^{26,34}Lys³⁸-(Aspa-AOct)-GLP-1 (7-38); Thr⁸Glu³⁸Arg^{26,34}Lys³⁹-(Aspa-AOct)-GLP-1 (7-39);
Thr⁸Asp³⁵Arg^{26,34}Lys³⁶-(Aspa-AOct)-GLP-1(7-36); Thr⁸ Asp³⁵Arg^{26,34}Lys³⁶-(Aspa-AOct)-GLP-1 (7-36)amide; Thr⁸Asp³⁶Arg^{26,34} Lys³⁷-(As pa-AOct)-G LP-1 (7-37); Thr⁸Asp³⁷Arg^{26,34} Lys³⁸-(Aspa-AOct)-GLP-1(7-38); Thr⁸Asp³⁸Arg^{26,34}Lys³⁹-(Aspa-AOct)-GLP-1(7-39); Thr⁸Asp³⁵Arg^{26,34} Lys³⁶-(Aspa-AOct)-GLP-1 (7-36); Thr⁸Asp³⁵Arg^{26,34}Lys³⁶-(Aspa-AOct)-GLP-1 (7-36)amide; Thr⁸ Asp³⁶Arg^{26,34}Lys³⁷-(Aspa-AOct)-GLP-1(7-37); Thr⁸Asp³⁷Arg^{26,34}Lys³⁸-(Aspa-AOct)-GLP-1(7-38); Thr⁸Asp³⁸Arg^{26,34}Lys³⁹-(Aspa-AOct)-GLP-1 (7-39);
Arg^{26,34}Lys³⁶-(Aspa-ALit)-GLP-1(7-36); Arg^{26,34}Lys-16-(Aspa-ALit)-GLP-1 (7-36)amide; Arg^{26,34} Lys³⁸-(Aspa-ALit)-GLP-1(7-38); Arg^{26,34}Lys³⁹-(Aspa-ALit)-GLP-1 (7-39);
Gly⁸Arg^{26,34}Lys³-(Aspa-ALit)-GLP-1(7-36); Gly⁸Arg^{26,34}Lys³⁶-(Aspa-ALit)-GLP-1(7-36)amide; Gly⁸Arg^{26,34}Lys³⁸-(Aspa-ALit)-GLP-1(7-38); Gly⁸Arg^{26,34}Lys³⁹-(Aspa-ALit)-GLP-1 (7-39);
Val⁸Arg^{26,34}Lys³⁶-(Aspa-ALit)-GLP-1(7-36); Val⁸ Arg^{26,34} Lys³⁶-(Aspa-ALit)-GLP-1 (7-36)amide; Val⁸Arg^{26,34}Lys³⁸-(Aspa-ALit)-GLP-1(7-38); Val⁸Arg^{26,34}Lys³⁹-(Aspa-ALit)-GLP-1 (7-39);
Ser⁸Arg^{26,34}Lys³⁶-(Aspa-ALit)-GLP-1(7-36); Ser⁸Arg^{26,34}Lys³⁶-(Aspa-ALit)-GLP-1 (7-36)amide; Ser⁸Arg^{26,34}Lys³⁸-(Aspa-ALit)-GLP-1(7-38); Ser⁸Arg^{26,34}Lys³⁹-(Aspa-ALit)-GLP-1 (7-39);
Thr⁸Arg^{26,34}Lys³⁶-(Aspa-ALit)-GLP-1(7-36); Thr⁸Arg^{26,34} Lys³⁶-(Aspa-ALit)-GLP-1 (7-36)amide; Thr⁸Arg^{26,34}Lys³⁸-(Aspa-ALit)-GLP-1(7-38); Thr⁸ Arg^{26,34} Lys³⁹-(Aspa-ALit)-GLP-1 (7-39);
Gly⁸Glu³⁵Arg^{26,34}Lys³⁶-(Aspa-ALit)-GLP-1 (7-36); Gly⁸Glu³⁵Arg^{26,34} Lys³⁶-(Aspa-ALit)-GLP-1 (7-36)amide; Gly⁸Glu³⁶Arg^{26,34}Lys³⁷-(Aspa-ALit)-GLP-1(7-37); Gly⁸Glu³⁷Arg^{26,34} Lys³⁸-(Aspa-ALit)-GLP-1 (7-38); Gly⁸Glu³⁸ Arg^{26,34}Lys³⁹-(Aspa-ALit)-GLP-1 (7-39); Gly⁸Glu³⁵Arg^{26,34} Lys³⁶-(Aspa-ALit)-GLP-1 (7-36); Gly⁸Glu³⁵Arg^{26,34}Lys³⁶-(Aspa-ALit)-GLP-1(7-36)amide; Gly⁸Glu³⁶Arg^{26,34}Lys³⁷-(Aspa-ALit)-GLP-1(7-37); Gly⁸Glu³⁷Arg^{26,34} Lys³⁸-(Aspa-ALit)-GLP-1 (7-38); Gly⁸Glu³⁸Arg^{26,34}Lys³⁹-(Aspa-ALit)-GLP-1 (7-39);
Gly⁸ Asp³⁵Arg^{26,34} Lys³⁶-(Aspa-ALit)-GLP-1 (7-36); Gly⁸ Asp³⁵Arg^{26,34}Lys³⁶-(Aspa-ALit)-GLP-1 (7-36)amide; Gly⁸Asp³⁶Arg^{26,34}Lys³⁷-(Aspa-ALit)-GLP-1(7-37); Gly⁸Asp³⁷Arg^{26,34}Lys³⁸-(Aspa-ALit)-GLP-1 (7-38); Gly⁸Asp³⁸Arg^{26,34}Lys³⁹-(Aspa-ALit)-GLP-1(7-39); Gly⁸Asp³⁵Arg^{26,34}Lys³⁶-(Aspa-ALit)-GLP-1 (7-36); Gly⁸Asp³⁵Arg^{26,34}Lys³⁶-(Aspa-ALit)-GLP-1 (7-36)amide; Gly⁸Asp³⁶Arg^{26,34}Lys³⁷-(Aspa-ALit)-GLP-1 (7-37); Gly⁸Asp³⁷Arg^{26,34}Lys³⁸-(Aspa-ALit)-GLP-1 (7-38); Gly⁸Asp³⁸Arg^{26,34}Lys³⁹-(Aspa-ALit)-GLP-1 (7-39);
Val⁸Glu³⁵ Arg^{26,34}Lys³⁶-(Aspa-ALit)-GLP-1 (7-36); Val⁸Glu³⁵ Arg^{26,34} Lys³⁶-(Aspa-ALit)-GLP-1 (7-36)amide; Val⁸Glu³⁶Arg^{26,34} Lys³⁷-(Aspa-ALit)-GLP-1(7-37); Val⁸Glu³⁷Arg^{26,34}Lys³⁸-(Aspa-ALit)-GLP-1 (7-38); Val⁸Glu³⁸Arg^{26,34}Lys³⁹ -(Aspa-ALit)-GLP-1 (7-39); Val⁸Glu³⁵Arg^{26,34}Lys³⁶ -(Aspa-ALit)-GLP-1 (7-36); Val⁸Glu³⁵Arg^{26,34}Lys³⁶-(Aspa-ALit)-GLP-1(7-36)amide; Val⁸Glu³⁶Arg^{26,34}Lys³⁷-(Aspa-ALit)-GLP-1 (7-37); Val⁸Glu³⁷Arg^{26,34}Lys³⁸-(Aspa-ALit)-GLP-1 (7-38); Val⁸Glu³⁸Arg^{26,34}Lys³⁹-(Aspa-ALit)-GLP-1 (7-39);
Val⁸Asp³⁵Arg^{26,34}Lys³⁶-(Aspa-ALit)-GLP-1 (7-36); Val⁸Asp³⁵Arg^{26,34}Lys³⁶-(Aspa-ALit)-GLP-1 (7-36)amide; Val⁸Asp³⁶Arg^{26,34}Lys³⁷-(Aspa-ALit)-GLP-1(7-37); Val⁸Asp³⁷Arg^{26,34}Lys³⁸-(Aspa-ALit)-GLP-1 (7-38); Val⁸Asp³⁸Arg^{26,34}Lys³⁹-(Aspa-ALit)-GLP-1(7-39); Val⁸Asp³⁵Arg^{26,34}Lys³⁶-(Aspa-ALit)-GLP-1 (7-36); Val⁸Asp³⁵Arg^{26,34}Lys³⁶-(Aspa-ALit)-GLP-1(7-36)amide; Val⁸Asp³⁶Arg^{26,34}Lys³⁷-(Aspa-ALit)-GLP-1 (7-37); Val⁸Asp³⁷Arg^{26,34}Lys³⁸-(Aspa-ALit)-GLP-1 (7-38); Val⁸Asp³⁸Arg^{26,34}Lys³⁹-(Aspa-ALit)-GLP-1 (7-39);
Ser⁸Glu³⁵Arg^{26,34}Lys³⁶-(Aspa-ALit)GLP-1 (7-36); Ser⁸Glu³⁵Arg^{26,34}Lys³⁶-(Aspa-ALit)-GLP-1 (7-36)amide; Ser⁸Glu³⁶Arg^{26,34}Lys³⁷-(Aspa-ALit)-GLP-1(7-37); Ser⁸Glu³⁷Arg^{26,34}Lys³⁸-(Aspa-ALit)-GLP-1 (7-38); Ser⁸Glu³⁸Arg^{26,34}Lys³⁹-(Aspa-ALit)-GLP-1(7-39); Ser⁸Glu³⁵Arg^{26,34}Lys³⁶-(Aspa-ALit)-GLP-1 (7-36); Ser⁸Glu³⁵Arg^{26,34}Lys³⁶-(Aspa-ALit)-GLP-1 (7-36)amide; Ser⁸Glu³⁶Arg^{26,34}Lys³⁷-(Aspa-ALit)-GLP-1 (7-37); Ser⁸Glu³⁷Arg^{26,34}Lys³⁸-(Aspa-ALit)-GLP-1 (7-38); Ser⁸Glu³⁸Arg^{26,34}Lys³⁹-(Aspa-ALit)-GLP-1 (7-39);
Ser⁸Asp³⁵Arg^{26,34}Lys³⁶-(Aspa-ALit)-GLP-1 (7-36); Ser⁸Asp³⁵Arg^{26,34}Lys³⁶-(Aspa-ALit)-GLP-1 (7-36)amide; Ser⁸Asp³⁶Arg^{26,34}Lys³⁷-(Aspa-ALit)-GLP-1(7-37); Ser⁸Asp³⁷Arg^{26,34}Lys³⁸-(Aspa-ALit)-GLP-1 (7-38); Ser⁸Asp³⁸Arg^{26,34}Lys³⁹-(Aspa-ALit)-GLP-1(7-39); Ser⁸Asp³⁵Arg^{26,34}Lys³⁶-(Aspa-ALit)-GLP-1 (7-36); Ser⁸Asp³⁵-Arg^{26,34}Lys³⁶-(Aspa-ALit)-GLP-1 (7-36)amide; Ser⁸Asp³⁶Arg^{26,34}Lys³⁷-(Aspa-ALit)-GLP-1 (7-37); Ser⁸Asp³⁷Arg^{26,34}Lys³⁸-(Aspa-ALit)-GLP-1 (7-38); Ser⁸Asp³⁸Arg^{26,34}Lys³⁹-(Aspa-ALit)-GLP-1 (7-39);
Thr⁸Glu³⁵Arg^{26,34}Lys³⁶-(Aspa-ALit)-GLP-1(7-36); Thr⁸Glu³⁵Arg^{26,34}Lys³⁶-(Aspa-ALit)-GLP-1(7-36)amide; Thr⁸Glu³⁶Arg^{26,34}Lys³⁷-(Aspa-ALit)-GLP-1(7-37); Thr⁸Glu³⁷Arg^{26,34}Lys³⁸-(Aspa-ALit)-GLP-1 (7-38); Thr⁸Glu³⁸Arg^{26,34}Lys³⁹-(Aspa-ALit)-GLP-1 (7-39); Thr⁸Glu³⁵Arg^{26,34}Lys³⁶-(Aspa-ALit)-GLP-1 (7-36); Thr⁸Glu³⁵Arg^{26,34}Lys³⁶-(Aspa-ALit)-GLP-1 (7-36)amide; Thr⁸Glu³⁶Arg^{26,34}Lys³⁷-(Aspa-ALit)-GLP-1 (7-37); Thr⁸Glu³⁷Arg^{26,34}Lys³⁸-(Aspa-ALit)-GLP-1 (7-38); Thr⁸Glu³⁸Arg^{26,34}Lys³⁹-(Aspa-ALit)-GLP-1 (7-39);
Thr⁸Asp³⁵Arg^{26,34}Lys³⁶-(Aspa-ALit)-GLP-1 (7-36); Thr⁸Asp³⁵Arg^{26,34}Lys³⁶-(Aspa-ALit)-GLP-1 (7-36)amide; Thr⁸Asp³⁶Arg^{26,34}Lys³⁷-(Aspa-ALit)-GLP-1 (7-37); Thr⁸Asp³⁷Arg^{26,34}Lys³⁸-(Aspa-ALit)-GLP-1 (7-38); Thr⁸Asp³⁸Arg^{26,34}Lys³⁹-(Aspa-ALit)-GLP-1 (7-39); Thr⁸Asp³⁵Arg^{26,34}Lys³⁶-(Aspa-ALit)-GLP-1 (7-36); Thr⁸Asp³⁵Arg^{26,34}Lys³⁶-(Aspa-ALit)-GLP-1 (7-36)amide; Thr⁸Asp³⁶Arg^{26,34}Lys³⁷-(Aspa-ALit)-GLP-1 (7-37); Thr⁸Asp³⁷Arg^{26,34}Lys³⁸-(Aspa-ALit)-GLP-1 (7-38); Thr⁸Asp³⁸Arg^{26,34}Lys³⁹-(Aspa-ALit)-GLP-1 (7-39);

### Pharmaceutical compositions

The present invention also relates to pharmaceutical compositions comprising a derivative of a GLP-1 analog of the present invention and a pharmaceutically acceptable vehicle or carrier.

Preferably, the pharmaceutical compositions comprise an isotonic agent, a preservative and a buffer. Examples of isotonic agents are sodium chloride, mannitol and glycerol. Examples of preservatives are phenol, m-cresol, methyl p-hydroxybenzoate and benzyl alcohol. Suitable buffers include sodium acetate and sodium phosphate.

The pharmaceutical compositions preferably further comprise a surfactant in order to improve the solubility and/or the stability of the GLP-1 derivative.

The pharmaceutical compositions preferably also comprise zinc.

The pharmaceutial compositions preferably further comprise another antidiabetic agent. The term "antidiabetic agent" includes compounds for the treatment and/or prophylaxis of insulin resistance and diseases wherein insulin resistance is the pathophysiological mechanism.

In one embodiment of this invention, the antidiabetic agent is an insulin, more preferably human insulin.

In another embodiment the antidiabetic agent is a hypoglycaemic agent, preferably an oral hypoglycaemic agent. Oral hypoglycaemic agents are preferably selected from the group consisting of sulfonylureas, biguanides, thiazolidinediones, glucosidase inhibitors, glucagon antagonists, GLP-1 agonists, potasium channel openers, insulin sensitizers, hepatic enzyme inhibitors, glucose uptake modulators, compounds modifying the lipid metabolism, compounds lowering food intake, and agents acting on the ATP-dependent potassium channel of the β-cells. Preferred sulfonylureas are tolbutamide, glibenclamide, glipizide and gliclazide. A preferred biguanide is metformin. Preferred thiazolidinediones are troglitazone and ciglitazone. A preferred glucosidase inhibitors is acarbose. Preferred agents acting on the ATP-dependent potassium channel of the ß-cells are: glibenclamide, glipizide, gliclazide, and repaglinide.

The pharmaceutical compositions of the present invention may be administered parenterally to patients in need of such a treatment. Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of the GLP-1 derivative in the form of a nasal or pulmonal spray. As a still further option, the GLP-1 derivatives of the invention can also be administered transdermally, *e.g*. from a patch, optionally a iontophoretic patch, or transmucosally, *e.g*. bucally.

The pharmaceutical compositions of the present invention may be prepared by conventional techniques, *e.g*. as described in Remington's Pharmaceutical Sciences, 1985 or in Remington: The Science and Practice of Pharmacy, 19th edition, 1995.

For example, injectable compositions of the GLP-1 derivative of the invention can be prepared using the conventional techniques of the pharmaceutical industry which involves dissolving and mixing the ingredients as appropriate to give the desired end product.

A composition for nasal administration of certain peptides may, for example, be prepared as described in European Patent No. 272097 (to Novo Nordisk A/S) or in WO 93/18785.

In a preferred embodiment of the present invention, the GLP-1 derivative is provided in the form of a composition suitable for administration by injection. Such a composition can either be an injectable solution ready for use or it can be an amount of a solid composition, e.g*.* a lyophilised product, which has to be dissolved in a solvent before it can be injected. The injectable solution preferably contains not less than about 2 mg/ml, preferably not less than about 5 mg/ml, more preferred not less than about 10 mg/ml of the GLP-1 derivative and, preferably, not more than about 100 mg/ml of the GLP-1 derivative.

### Uses

The present invention also relates to the use of a GLP-1 derivative of the invention for the preparation of a medicament which has a protracted profile of action relative to GLP-1 (7-37).

The present invention relates also to the use of a GLP-1 derivative of the invention for the preparation of a medicament with protracted effect for the treatment of non-insulin dependent diabetes mellitus.

The present invention also relates to the use of a GLP-1 derivative of the invention for the preparation of a medicament with protracted effect for the treatment of insulin dependent diabetes mellitus.

The present invention also relates to the use of a GLP-1 derivative of the invention for the preparation of a medicament with protracted effect for the treatment of obesity.

In a further preferred embodiment, the present invention relates to a method of treating insulin dependent or non-insulin dependent diabetes mellitus in a patient in need of such a treatment, comprising administering to the patient a therapeutically effective amount of a derivative of GLP-1 analog of the present invention together with a pharmaceutically acceptable carrier.

### Methods of Production

The parent peptide can be produced by a method which comprises culturing a host cell containing a DNA sequence encoding the polypeptide and capable of expressing the polypeptide in a suitable nutrient medium under conditions permitting the expression of the peptide, after which the resulting peptide is recovered from the culture.

The medium used to culture the cells may be any conventional medium suitable for growing the host cells, such as minimal or complex media containing appropriate supplements. Suitable media are available from commercial suppliers or may be prepared of published recipes (*e.g*. in catalogues of the American Type Culture Collection). The peptide produced by the cells may then be recovered from the culture medium by conventional procedures including separating the host cells from the medium by centrifugation or filtration, precipitating the proteinaceous components of the supernatant or filtrate by means of a salt, *e.g*. ammonium sulphate, purification by a variety of chromatographic procedures, *e.g*. ion exchange chromatography, gel filtration chromatography, affinity chromatography, or the like, dependent on the type of peptide in question.

The DNA sequence encoding the parent peptide may suitably be of genomic or cDNA origin, for instance obtained by preparing a genomic or cDNA library and screening for DNA sequences coding for all or part of the peptide by hybridisation using synthetic oligonucleotide probes in accordance with standard techniques (see, for example, Sambrook, J, Fritsch, EF and Maniatis, T, Molecular Cloning: A Laboratory Manual, Cold Spring Harbor Laboratory Press, New York, 1989). The DNA sequence encoding the peptide may also be prepared synthetically by established standard methods, *e.g*. the phosphoamidite method described by Beaucage and Caruthers, Tetrahedron Letters 22 (1981), 1859 - 1869, or the method described by Matthes et al., EMBO Journal 3 (1984), 801 - 805. The DNA sequence may also be prepared by polymerase chain reaction using specific primers, for instance as described in US 4,683,202 or Saiki et al., Science 239 (1988), 487 - 491.

The DNA sequence may be inserted into any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, *e.g*. a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the peptide is operably linked to additional segments required for transcription of the DNA, such as a promoter. The promoter may be any DNA sequence which shows transcriptional activity in the host cell of choice and may be derived from genes encoding proteins either homologous or heterologous to the host cell. Examples of suitable promoters for directing the transcription of the DNA encoding the peptide of the invention in a variety of host cells are well known in the art, cf. for instance Sambrook *et al*., *supra.*

The DNA sequence encoding the peptide may also, if necessary, be operably connected to a suitable terminator, polyadenylation signals, transcriptional enhancer sequences, and translational enhancer sequences. The recombinant vector of the invention may further comprise a DNA sequence enabling the vector to replicate in the host cell in question.

The vector may also comprise a selectable marker, *e.g*. a gene the product of which complements a defect in the host cell or one which confers resistance to a drug, *e.g*. ampicillin, kanamycin, tetracyclin, chloramphenicol, neomycin, hygromycin or methotrexate.

To direct a parent peptide of the present invention into the secretory pathway of the host cells, a secretory signal sequence (also known as a leader sequence, prepro sequence or pre sequence) may be provided in the recombinant vector. The secretory signal sequence is joined to the DNA sequence encoding the peptide in the correct reading frame. Secretory signal sequences are commonly positioned 5' to the DNA sequence encoding the peptide. The secretory signal sequence may be that normally associated with the peptide or may be from a gene encoding another secreted protein.

The procedures used to ligate the DNA sequences coding for the present peptide, the promoter and optionally the terminator and/or secretory signal sequence, respectively, and to insert them into suitable vectors containing the information necessary for replication, are well known to persons skilled in the art (cf., for instance, Sambrook *et a*/*., supra).*

The host cell into which the DNA sequence or the recombinant vector is introduced may be any cell which is capable of producing the present peptide and includes bacteria, yeast, fungi and higher eukaryotic cells. Examples of suitable host cells well known and used in the art are, without limitation, *E. coli, Saccharomyces cerevisiae,* or mammalian BHK or CHO cell lines.

The GLP-1 derivatives of this invention can be used in the treatment of various diseases. The particular GLP-1 derivative to be used and the optimal dose level for any patient will depend on the disease to be treated and on a variety of factors including the efficacy of the specific peptide derivative employed, the age, body weight, physical activity, and diet of the patient, on a possible combination with other drugs, and on the severity of the case. It is recommended that the dosage of the GLP-1 derivative of this invention be determined for each individual patient by those skilled in the art.

In particular, it is envisaged that the GLP-1 derivative will be useful for the preparation of a medicament with a protracted profile of action for the treatment of non-insulin dependent diabetes mellitus and/or for the treatment of obesity.

The present invention is further illustrated by the following examples which, however, are not to be construed as limiting the scope of protection. The features disclosed in the foregoing description and in the following examples may, both separately and in any combination thereof, be material for realising the invention in diverse forms thereof.

### EXAMPLES

The following acronyms for commercially available chemicals are used:

| | |
|---|---|
| DMF : | N,N-Dimethylformamide. |
| DCC : | N,N-Dicyclohexylcarbodiimide |
| NMP : | N-Methyl-2-pyrrolidone. |
| EDPA : | N-Ethyl-N,N-diisopropylamine. |
| EGTA : | Ethylene glycol-bis(β-aminoethyl ether)-N,N,N',N'-tetraacetic acid. |
| GTP | Guanosine 5'-triphosphate. |
| TFA : | Trifluoroacetic acid. |
| THF : | Tetrahydrofuran |
| H-Glu(OH)-OBu^{t}: | L-Glutamic acid α-tert-butyl ester |
| Cap-ONSu: | Octanoic acid 2,5-dioxopyrrolidin-1-yl ester |
| Lau-ONSu: | Dodecanoic acid 2,5-dioxopyrrolidin-1-yl ester |
| Myr-ONSu: | Tetradecanoic acid 2,5-dioxopyrrolidin-1-yl ester. |
| Pal-ONSu: | Hexadecanoic acid 2,5-dioxopyrrolidin-1-yl ester. |
| Ste-ONSu | Octadecanoic acid 2,5-dioxopyrrolidin-1-yl ester. |
| Cac-ONSu: | Decanoic acid 2,5-dioxopyrrolidin-1-yl ester. |

### Abbreviations:

PDMS: Plasma Desorption Mass Spectrometry
MALDI-MS: Matrix Assisted Laser Desorption/Ionisation Mass Spectrometry
HPLC: High Performance Liquid Chromatography
amu: atomic mass units
Lit-Glu(ONSu)-OBu^{t}: N^{α}-Lithochoyl-L-glutamic acid α-t-butyl ester γ-2,5-dioxopyrrolidin-1-yl ester
Cap-Glu(ONSu)-OBu^{t}: N^{α}-Octanoyl-L-glutamic acid α-t-butyl ester γ-2,5-dioxopyrrolidin-1-yl ester
Cac-Glu(ONSu)-OBu^{t}: N^{α}-Decanoyl-L-glutamic acid α-t-butyl ester γ-2,5-dioxopyrrolidin-1-yl ester
Lau-Glu(ONSu)-OBu^{t}: N^{α}-Dodecanoyl-L-glutamic acid α-t-butyl ester γ-2,5-dioxopyrrolidin-1-yl ester
Myr-Glu(ONSu)-OBu^{t}: Nα-Tetradecanoyl-L-glutamic acid α-t-butyl ester γ-2,5-dioxopyrrolidin-1-yl ester
Pal-Glu(ONSu)-OBu^{t} : N^{α}-Hexadecanoyl-(L)-glutamic acid α-t-butyl-γ-2,5-dioxopyrrolidin-1-yl diester.
Ste-Glu(ONSu)-OBu^{t} : N^{α}-Octadecanoyl-(L)-glutamic acid α-t-butyl-γ-2,5-dioxopyrrolidin-1-yl diester
Lau-β-Ala-ONSu: N^{β}-Dodecanoyl-β-alanine 2,5-dioxopyrrolidin-1-yl ester
Pal-o-Ala-ONSu: Nβ-Hexadecanoyl-β-alanine 2,5-dioxopyrrolidin-1-yl ester
Lau-GABA-ONSu: N^{γ}-Dodecanoyl-y-aminobutyric acid 2,5-dioxopyrrolidin-1-yl ester
Myr-GABA-ONSu: N^{γ}-Tetradecanoyl-y-aminobutyric acid 2,5-dioxopyrrolidin-1-yl ester
Pal-GABA-ONSu: N^{γ}-Hexadecanoyl-γ-aminobutyric acid 2,5-dioxopyrrolidin-1-yl ester
Ste-GABA-ONSu: N^{γ}-Octadecanoyl-γ-aminobutyric acid 2,5-dioxopyrrolidin-1-yl ester
Pal-Isonip-ONSu: N-Hexadecanoyl-piperidine-4-carboxylic acid 2,5-dioxopyrrolidin-1-yl ester
Pal-Glu(OBu')-ONSu: N^{α}-Hexadecanoyl-L-glutamic acid α-2,5-dioxopyrrolidin-1-yl ester γ-t-butyl ester
HOOC-(CH₂)₆-COONSu: ω-Carboxyheptanoic acid 2,5-dioxopyrrolidin-1-yl ester.
HOOC-(CH₂)₁₀-COONSu: ω-Carboxyundecanoic acid 2,5-dioxopyrrolidin-1-yl ester.
HOOC-(CH₂)₁₂-COONSu: ω-Carboxytridecanoic acid 2,5-dioxopyrrolidin-1-yl ester.
HOOC-(CH₂)₁₄-COONSu: ω-Carboxypentadecanoic acid 2,5-dioxopyrrolidin-1-yl ester.
HOOC-(CH₂)₁₆-COONSu: ω-Carboxyheptadecanoic acid 2,5-dioxopyrrolidin-1-yl ester.
HOOC-(CH₂)₁₈-COONSu: ω-Carboxynonadecanoic acid 2,5-dioxopyrrolidin-1-yl ester.

### Analytical

### Plasma Desorption Mass Spectrometry

### Sample preparation:

The sample is dissolved in 0.1 % TFA/EtOH (1:1) at a concentration of 1 µg/ul. The sample solution (5-10 µl) is placed on a nitrocellulose target (Bio-ion AB, Uppsala, Sweden) and allowed to adsorb to the target surface for 2 minutes. The target is subsequently rinsed with 2x25 µl 0.1 % TFA and spin-dried. Finally, the nitrocellulose target is placed in a target carrousel and introduced into the mass spectrometer.

### MS analysis:

PDMS analysis was carried out using a Bio-ion 20 time-of flight instrument (Bio-ion Nordic AB, Uppsala, Sweden). An acceleration voltage of 15 kV was applied and molecular ions formed by bombardment of the nitrocellulose surface with 252-Cf fission fragments were accelerated towards a stop detector. The resulting time-of-flight spectrum was calibrated into a true mass spectrum using the H⁺ and NO⁺ ions at m/z 1 and 30, respectively. Mass spectra were generally accumulated for 1.0x10⁶ fission events corresponding to 15-20 minutes. Resulting assigned masses all correspond to isotopically averaged molecular masses. The accuracy of mass assignment is generally better than 0.1 %.

### MALDI-MS

MALDI-TOF MS analysis was carried out using a Voyager RP instrument (PerSeptive Biosystems Inc., Framingham, MA) equipped with delayed extraction and operated in linear mode. Alpha-cyano-4-hydroxy-cinnamic acid was used as matrix, and mass assignments were based on external calibration.

### Example 1

### Synthesis of Arg^{26,34}, Lys³⁶ (N^{ε}-(γ-glutamyl(NCL-hexadecanoyl))) GLP-1 (7-36)-OH.

To a mixture of Arg^{26,34}, Lys³⁶ GLP-1 (7-36)-OH (12.2 mg, 3.67 µmol), EDPA (13.3 mg, 103 µmol), NMP (1.71 ml) and water (855 µl) was added a solution of Pal-Glu(ONSu)-OBu^{t} (5.94 mg, 11 µmol), prepared as described in PCT application no. PCT/DK97/00340, in NMP (148 µl). The reaction mixture was gently shaken for 5 min. at room temperature, and then allowed to stand for an additional 90 min. at room temperature. The reaction was quenched by the addition of a solution of glycine (6 mg, 81 µmol) in water (0.6 ml). A 0.5 % aqueous solution of ammonium-acetate (38 ml) was added, and the resulting mixture eluted onto a Varian 5g C8 Mega Bond Elut^{®} , the immobilised compound washed with 5% aqueous acetonitril (20 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65^{o}C and the acetonitril gradient was 0-100% in 60 minutes. The title compound (3.1 mg, 23 %) was isolated, and the product was analysed by PDMS. The m/z value for the protonated molecular ion was found to be 3695 +-3. The resulting molecular weight is thus 3694 +- 3 amu (theoretical value 3694 amu).

### Example 2

### Synthesis of Arg^{26,34}Lys36 (N^{ε}-(γ-glutamyl(N^{α}-octadecanoyl))) GLP-1 (7-36)-OH.

To a mixture of Arg^{26,34},Lys³⁶ GLP-1 (7-36)-OH (12.2 mg, 3.7 µmol), EDPA (13.3 mg, 103 µmol), NMP (1.71 ml) and water (855 µl) was added a solution of Ste-Glu(ONSu)-OBu^{t} (6.25 mg, 11 µmol), prepared as described in PCT application no. PCT/DK97/00340, in NMP (1 ml). The reaction mixture was gently shaken for 5 min. at room temperature, and then allowed to stand for an additional 90 min. at room temperature. The reaction was quenched by the addition of a solution of glycine (6 mg, 81 µmol) in water (0.6 ml). A 0.5 % aqueous solution of ammonium acetate (54 ml) was added, and the resulting mixture eluted onto a Varian 5g C8 Mega Bond Elut^{®} , the immobilised compound washed with 5% aqueous acetonitril (20 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (3.7 mg, 27 %) was isolated, and the product was analysed by PDMS. The m/z value for the protonated molecular ion was found to be 3723 +-3. The resulting molecular weight is thus 3722 +- 3 amu (theoretical value 3722 amu).

### Example 3

### Synthesis of lithocholic acid 2,5-dioxopyrrolidin-1-yi ester.

To a solution of lithocholic acid (5.44 g, 14.3 mmol) in a mixture of anhydrous THF (120 ml) and anhydrous acetonitril (30 ml) was added N-hydroxysuccinimide (1.78 g, 15 mmol). The mixture was cooled to 10°C, a solution of DCC (3.44 g, 16.7 mmol) in anhydrous THF (30 ml) was added drop wise, and the resulting reaction mixture stirred for 16 h at room temperature. The reaction mixture was filtered and partitioned between dichloromethane (450 ml) and 10% aqueous Na₂CO₃ (150 ml). The phases were separated, and the organic phase washed with 10% aqueous Na₂CO₃ (150 ml), water (2x150 ml), and dried (MgSO₄). The solvent was concentrated *in vacuo.* The residue was crystallised from a mixture of dichloromethane (30 ml) and n-heptane (30 ml). The precipitate was dried in a vacuum drying oven for 36 h to give the title compound (3.46 g, 51 %).

### Example 4

### Synthesis of Lit-Glu(ONSu)-OBu^{t}.

A suspension of H-Glu(OH)-OBu^{t} (1.28 g, 6.33 mmol), DMF (88 ml) and EDPA (0.82 g, 6.33 mmol) and lithocholic acid 2,5-dioxopyrrolidin-1-yl ester, prepared as described in example 3, was stirred for 16 h at room temperature. The reaction mixture was concentrated *in vacuo* and the residue dissolved in ethyl acetate (40 ml). The resulting solution was washed with 5% aqueous citric acid (2x25 ml), brine (10 ml), and filtered). The solvent was concentrated *in vacuo* and the residue dissolved in DMF (12 ml). The resulting solution was added drop wise to a 10% aqueous solution of citric acid whereby the product precipitates. The precipitate was collected and washed with iced water, and dried *in vacuo.* The crude product was recrystallised from a mixture of n-heptane (40 ml) and 2-propanol (17 ml). The precipitate was dried in a vacuum drying oven for 4 h to give the free acid intermediate. To a solution of the free acid intermediate in DMF (18 ml) was added hydroxysuccinimide (0.45 g, 3.91 mmol), followed by a solution of DCC (0.73 g, 3.56 mmol) in dichloromethane (18 ml). The resulting mixture was stirred at ambient temperature for 18 h, and then filtered. The filtrate was concentrated *in vacuo* to a solid, and the residue was dissolved in dichloromethane (25 ml), and the filtration repeated, the solvent removed *in vacuo* to give a foam. The residue was dissolved in refluxing n-heptane (35 ml), and the product crystallised b addition of 2-propanol. The precipitate was collected, washed with cold n-heptane, dried at 35°C *in vacuo* to give the title compound (1.34 g, 57%).

### Example 5

### Synthesis of Arg³⁴,Lys²⁶ (N^{ε}-(γ-glutamyl(N^{α}-lithochoyl))) GLP-1 (7-37)-OH.

To a mixture of Arg³⁴,Lys²⁶ GLP-1 (7-37)-OH (41.1 mg, 12.2 µmol), EDPA (44 mg, 340 µmol), NMP (5.76 ml) and water (2.88 ml) was added a solution of Lit-Glu(ONSu)-OBu^{t} (24 mg, 37 µmol), prepared as described in example 4, in NMP (600 µl). The reaction mixture was gently shaken for 5 min. at room temperature, and then allowed to stand for an additional 75 min. at room temperature. The reaction was quenched by the addition of a solution of glycine (20 mg, 268 µmol) in water (2 ml). A 0.5 % aqueous solution of ammonium acetate (128 ml) was added, and the resulting mixture divided into two equal portions, and each portion eluted onto a Varian 5g C8 Mega Bond Elut^{®} , the immobilised compound washed with 5% aqueous acetonitril (2x25 ml), and finally liberated from the cartridge by elution with TFA (2x25 ml). The combined eluates were concentrated *in vacuo,* and the residue purified by column chromato-graphy using a cyanopropyl column (Zorbax 300SB-CN) and a standard acetonitril/TFA system. The column was heated to 65°C and the acetonitril gradient was 0-100% in 60 minutes. The title compound (5 mg, 11 %) was isolated, and the product was analysed by PDMS. The m/z value for the protonated molecular ion was found to be 3872 +- 3. The resulting molecular weight is thus 3871 +- 3 amu (theoretical value 3871 amu).

### Example 6

### Synthesis of Arg²⁶,Lys³⁴ (N^{ε}-(γ-glutamyl(N^{α}-hexadecanoyl))) GLP-1 (7-37)-OH

To a mixture of Arg²⁶,Lys³⁴ GLP-1 (7-37)-OH (18 mg, 5.3 µmol), EDPA (19.3 mg, 149 µmol), NMP (2.52 ml) and water (1.26 ml) was added a solution of Pal-Glu(ONSu)-OBu^{t} (8.6 mg, 16 µmol) in NMP (215 µl). The reaction mixture was gently shaken for 5 min. at room temperature, and then allowed to stand for an additional 90 min. at room temperature. The reaction was quenched by the addition of a solution of glycine (8.8 mg, 117 µmol) in water (0.88 ml). A 0.5 % aqueous solution of ammonium acetate (50 ml) was added, and the resulting mixture eluted onto a Varian 5g C8 Mega Bond Elut^{®} , the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (6 mg, 30 %) was isolated, and the product was analysed by PDMS. The m/z value for the protonated molecular ion was found to be 3752 +- 3. The resulting molecular weight is thus 3751 +- 3 amu (theoretical value 3751 amu).

### Example 7

### Synthesis of Gly⁸,Arg^{26,34},Lys³⁸ (N^{ε}-(γ-glutamyl(N^{α}-hexadecanoyl))) GLP-1 (7-38)-OH.

To a mixture of Gly⁸,Arg^{26,34},Lys³⁸ GLP-1 (7-38)-OH (11.8 mg, 3.4 µmol), EDPA (12.1 mg, 94 µmol), NMP (1.65 ml) and water (0.83 ml) was added a solution of Pal-Glu(ONSu)-OBu^{t} (5.4 mg, 10 µmol) in NMP (135 µl). The reaction mixture was gently shaken for 5 min. at room temperature, and then allowed to stand for an additional 75 min. at room temperature. The reaction was quenched by the addition of a solution of glycine (5.5 mg, 73.7 µmol) in water (553 µl). A 0.5 % aqueous solution of ammonium acetate (36 ml) was added, and the resulting mixture eluted onto a Varian 5g C8 Mega Bond Elut^{®} , the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (5 mg, 38 %) was isolated, and the product was analysed by PDMS. The m/z value for the protonated molecular ion was found to be 3895 +- 3. The resulting molecular weight is thus 3894 +- 3 amu (theoretical value 3894 amu).

### Example 8

### Synthesis of Gly⁸,Glu³⁷,Arg^{26,34},Lys³⁸ (Nε-(-γ-glutamyl(N^{α}-hexadecanoyl))) GLP-1 (7-38)-OH.

To a mixture of Gly⁸,Glu³⁷,Arg^{26,34},Lys³⁸ GLP-1 (7-38)-OH (9 mg, 2.48 µmol), EDPA (9 mg, 69.4 µmol), NMP (1.25 ml) and water (0.63 ml) was added a solution of Pal-Glu(ONSu)-OBu^{t} (4 mg, 7.4 µmol) in NMP (100 µl). The reaction mixture was gently shaken for 5 min. at room temperature, and then allowed to stand for an additional 105 min. at room temperature. The reaction was quenched by the addition of a solution of glycine (4.1 mg, 54.6 µmol) in water (410 µl). A 0.5 % aqueous solution of ammonium acetate (27 ml) was added, and the resulting mixture eluted onto a Varian 5g C8 Mega Bond Elut^{®} , the immobilised compound washed with 5% aqueous acetonitril (15 ml), and finally liberated from the cartridge by elution with TFA (15 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (2.9 mg, 29 %) was isolated, and the product was analysed by PDMS. The m/z value for the protonated molecular ion was found to be 3967 +- 3. The resulting molecular weight is thus 3966 +- 3 amu (theoretical value 3967 amu).

### Example 9

### Synthesis of Gly⁸,Glu³⁷,Arg^{26,34},Lys³⁸ (N^{ε}-(γ-glutamyl(N^{α}-octadecanoyl))) GLP-1 (7-38)-OH.

To a mixture of Gly⁸,Glu³⁷,Arg^{26,34},Lys³⁸ GLP-1 (7-38)-OH (9 mg, 2.5 µmol), EDPA (9 mg, 69.4 µmol), NMP (1.25 ml) and water (0.63 ml) was added a solution of Ste-Glu(ONSu)-OBu^{t} (4.2 mg, 7.4 µmol in NMP (105 µl). The reaction mixture was gently shaken for 5 min. at room temperature, and then allowed to stand for an additional 105 min. at room temperature. The reaction was quenched by the addition of a solution of glycine (4.1 mg, 54.6 µmol) in water (409 µl). A 0.5 % aqueous solution of ammonium acetate (27 ml) was added, and the resulting mixture eluted onto a Varian 5g C8 Mega Bond Elut^{®} , the immobilised compound washed with 5% aqueous acetonitril (15 ml), and finally liberated from the cartridge by elution with TFA (15 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (3.2 mg, 32 %) was isolated, and the product was analysed by PDMS. The m/z value for the protonated molecular ion was found to be 3995 +- 3. The resulting molecular weight is thus 3994 +- 3 amu (theoretical value 3995 amu).

### Example 10

### Synthesis of Cap-Glu(ONSu)-OBu^{t}.

To a solution of octanoic acid (5 g, 34.7 mmol) and N-hydroxysuccinimide (4 g, 34.7 mmol) in anhydrous acetonitril (10 ml) was added a solution of DCC (7.15 g, 34.7 mmol) in anhydrous dichloromethane (15 ml), and the resulting reaction mixture stirred for 16 h at room temperature. The precipitated solid was filtered off and recrystallised from a mixture of n-heptane (40 ml) and 2-propanol (2 ml). The precipitate was dried in a vacuum drying oven for 16 h to give the intermediate Cap-ONSu. A suspension of the crude ester intermediate (3.9 g, 16.2 mmol), (L)-H-Glu(OH)-OBu^{t} (3.28 g, 16.2 mmol), DMF (268 ml) and EDPA (2.1 g, 16.2 mmol) was stirred for 64 h at room temperature. The reaction mixture was concentrated *in vacuo* and the residue dissolved in ethyl acetate (50 ml). The resulting solution was washed with 5% aqueous citric acid (2x25 ml). The solvent was concentrated in *vacuo* and the residue dissolved in DMF (36 ml). The resulting solution was added drop wise to a 10% aqueous solution of citric acid (357 ml) and extracted with ethyl acetate (200 ml), and dried (MgSO₄). The solvent was concentrated *in vacuo* to give the crude glutamic acid intermediate. To a mixture of the crude glutamic acid intermediate, N-hydroxysuccinimide (1.85 g, 16.1 mmol) and DMF (25 ml) was added a solution of DCC (3.32 g, 16.1 mmol) in dichloromethane (15 ml). The resulting mixture was stirred at ambient temperature for 20 h. The reaction mixture was filtered and the solvent concentrated *in vacuo.* The residue was purified on a silica gel column (40- 63µ), eluted with a mixture of dichloromethane and acetonitril (1:1) to give the title compound (0.63 g, 6% over all).

### Example 11

### Synthesis of Glu³⁷,Arg^{26,34},Lys³⁸ (N^{ε}-(γ-glutamyl(N^{α}-hexadecanoyl))) GLP-1 (7-38)-OH.

To a mixture of Glu³⁷,Arg^{26,34},Lys³⁸ GLP-1 (7-38)-OH (17.6 mg, 4.9 µmol), EDPA (17.6 mg, 136 µmol), NMP (1.23 ml) and water (2.46 ml) was added a solution of Pal-Glu(ONSu)OBu^{t} (7.9 mg, 14.6 µmol) in NMP (197 µl). The reaction mixture was gently shaken for 5 min. at room temperature, and then allowed to stand for an additional 2 h at room temperature. The reaction was quenched by the addition of a solution of glycine (8 mg, 107 µmol) in water (804 µl). A 0.5 % aqueous solution of ammonium acetate (49 ml) was added, and the resulting mixture eluted onto a Varian 5g C8 Mega Bond Elut^{®} , the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (5.1 mg, 26 %) was isolated, and the product was analysed by PDMS. The m/z value for the protonated molecular ion was found to be 3981 +- 3. The resulting molecular weight is thus 3980 +- 3 amu (theoretical value 3981 amu).

### Example 12

### Synthesis of Arg³⁴,Lys²⁶ (N^{ε}-(γ-glutamyl(N^{α}-octadecanoyl))) GLP-1 (7-37)-OH.

To a mixture of Arg³⁴ GLP-1 (7-37)-OH (41.1 mg, 12.2 µmol), EDPA (44 mg, 341 µmol), NMP (5.76 ml) and water (2.88 ml) was added a solution of Ste-Glu(ONSu)-OBu^{t} (20.7 mg, 36.5 µmol in NMP (517 µl). The reaction mixture was gently shaken for 5 min. at room temperature, and then allowed to stand for an additional 2 h at room temperature. The reaction was quenched by the addition of a solution of glycine (20.1 mg, 268 µmol) in water (2.01 ml). A 0.5 % aqueous solution of ammonium-acetate (120 ml) was added, and the resulting mixture eluted onto a Varian 5g C8 Mega Bond Elut^{®} , the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (15.4 mg, 34 %) was isolated, and the product was analysed by PDMS. The m/z value for the protonated molecular ion was found to be 3781 +-3. The resulting molecular weight is thus 3780 +- 3 amu (theoretical value 3779 amu).

### Example 13

### Synthesis of Arg³⁴,Lys²⁶(N^{ε}-decanoyl) GLP-1 (7-37)

To a mixture of Arg³⁴-GLP-1 (7-37)-OH (20 mg, 5.9 µmol), EDPA (21.4 mg, 165 µmol), NMP (2.8 ml) and water (1.4 ml) was added a solution of Cac-ONSu (4.8 mg, 17.7 µmol) in NMP (119 µl). The reaction mixture was gently shaken for 5 min., and then allowed to stand for an additional 2h at room temperature. The reaction was quenched by the addition of a solution of glycine (9.8 mg, 130 µmol) in water (98 µl). The resulting mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (7.4 mg, 35%) was isolated, and the product was analysed by PDMS. The m/z value for the protonated molecular ion was found to be 3539.6 ± 3. The resulting molecular weight is thus 3538.6 ± 3 amu (theoretical value 3538 amu).

### Example 14

### Synthesis of Arg³⁴,Lys²⁶ (N^{ε}-(hexadecanoyl)) GLP-1 (7-37)-OH.

To a mixture of Arg³⁴ GLP-1 (7-37)-OH (41.1 mg, 12.2 µmol), EDPA (44 mg, 340 µmol), NMP (2.88 ml) and water (2.88 ml) was added a solution of Pal-ONSu (12.9 mg, 36.5 µmol) in NMP (3.3 ml). The reaction mixture was gently shaken for 5 min. at room temperature, and then allowed to stand for an additional 110 min. at room temperature. The reaction was quenched by the addition of a solution of glycine (20.1 mg, 268 µmol) in water (201 µl). The solvent was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (15 mg, 34 %) was isolated, and the product was analysed by PDMS.

### Example 15

### Synthesis of Arg^{26,34},Lys²⁷ (N^{ε}-(γ-glutamyl(N^{α}-hexadecanoyl))) GLP-1 (7-37)-OH

To a mixture of Arg^{26,34}, Lys²⁷ GLP-1 (7-37)-OH (11.6 mg, 3.4 µmol), EDPA (12.3 mg, 94.9 µmol), NMP (1.6 ml) and water (0.8 ml) was added a solution of Pal-Glu(ONSu)-OBu^{t} (5.5 mg, 10.2 µmol) in NMP (137 µl). The reaction mixture was gently shaken for 5 min. at room temperature, and then allowed to stand for an additional 90 min. at room temperature. The reaction was quenched by the addition of a solution of glycine (5.6 mg, 74.6 µmol) in water (560 µl). A 0.5 % aqueous solution of ammonium acetate (34 ml) was added, and the resulting mixture eluted onto a Varian 5g C8 Mega Bond Elut^{®} , the immobilised compound washed with 5% aqueous acetonitril (15 ml), and finally liberated from the cartridge by elution with TFA (25 ml). The solvent was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (2.1 mg, 16 %) was isolated, and the product was analysed by PDMS.

### Example 16

### Synthesis of Arg^{26,34},Lys²³ (N^{ε}-(-γ-glutamyl(N^{α}-hexadecanoyl))) GLP-1 (7-37)-OH.

To a mixture of Arg^{26,34}, Lys²³ GLP-1 (7-37)-OH (11.6 mg, 3.4 µmol), EDPA (12.3 mg, 94.9 µmol), NMP (1.6 ml) and water (0.8 ml) was added a solution of Pal-Glu(ONSu)-OBu^{t} (5.5 mg, 10.2 µmol) in NMP (137 µl). The reaction mixture was gently shaken for 5 min. at room temperature, and then allowed to stand for an additional 90 min. at room temperature. The reaction was quenched by the addition of a solution of glycine (5.6 mg, 74.6 µmol) in water (560 µl). A 0.5 % aqueous solution of ammonium acetate (34 ml) was added, and the resulting mixture eluted onto a Varian 5g C8 Mega Bond Elut^{®} , the immobilised compound washed with 5% aqueous acetonitril (15 ml), and finally liberated from the cartridge by elution with TFA (25 ml). The solvent was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (3.1 mg, 24 %) was isolated, and the product was analysed by PDMS.

### Example 17

### Synthesis of Arg^{26,34},Lys¹⁸ (N^{ε}-(γ-glutamyl(N^{α}-hexadecanoyl))) GLP-1 (7-37)-OH

To a mixture of Arg^{26,34}, Lys¹⁸ GLP-1 (7-37)-OH (11.7 mg, 3.4 µmol), EDPA (12.2 mg, 94.6 µmol), NMP (1.6 ml) and water (0.8 ml) was added a solution of Pal-Glu(ONSu)-OBu^{t} (5.5 mg, 10.2 µmol) in NMP (137 µl). The reaction mixture was gently shaken for 5 min. at room temperature, and then allowed to stand for an additional 90 min. at room temperature. The reaction was quenched by the addition of a solution of glycine (5.6 mg, 74.6 µmol) in water (560 µl). A 0.5 % aqueous solution of ammonium acetate (34 ml) was added, and the resulting mixture eluted onto a Varian 5g C8 Mega Bond Elut^{®} , the immobilised compound washed with 5% aqueous acetonitril (25 ml), and finally liberated from the cartridge by elution with TFA (25 ml). The solvent was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (1.9 mg, 15 %) was isolated, and the product was analysed by PDMS.

### Example 18

### Synthesis of Arg³⁴,Lys²⁶ (N^{ε}-(octanoyl)) GLP-1 (7-37)-OH.

To a mixture of Arg³⁴ GLP-1 (7-37)-OH (41.1 mg, 12.2 µmol), EDPA (44 mg, 341 µmol), NMP (5.76 ml) and water (2.88 ml) was added a solution of Cap-ONSu (8.8 mg, 36.5 µmol, prepared as described in example 10, in NMP (106 µl). The reaction mixture was gently shaken for 5 min. at room temperature, and then allowed to stand for an additional 115 min. at room temperature. The reaction was quenched by the addition of a solution of glycine (20 mg, 268 µmol) in water (200 µl). The solvent was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (18.8 mg, 44 %) was isolated, and the product was analysed by PDMS.

### Example 19

### Synthesis of Arg³⁴,Lys²⁶ (N^{ε}-(dodecanoyl)) GLP-1 (7-37)-OH.

To a mixture of Arg³⁴ GLP-1 (7-37)-OH (41.1 mg, 12.2 µmol), EDPA (44 mg, 341 µmol), NMP (5.76 ml) and water (2.88 ml) was added a solution of Lau-ONSu (8.8 mg, 36.5 µmol, prepared in a similar manner as described for Cap-ONSu in example 10), in NMP (271 µl). The reaction mixture was gently shaken for 5 min. at room temperature, and then allowed to stand for an additional 100 min. at room temperature. The reaction was quenched by the addition of a solution of glycine (20.1 mg, 268 µmol) in water (200 µl). The solvent was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (18 mg, 42 %) was isolated, and the product was analysed by PDMS.

### Example 20

### Synthesis of Pal-GABA-ONSu.

A mixture of Pal-ONSu (3 g, 8.48 mmol), γ-aminobutyric acid (0.87 g, 8.48 mmol) in DMF (200 ml) was stirred at room temperature for 60 h. The reaction mixture was filtered and the filtrate was added drop wise to 10% aqueous citric acid (500 ml). The precipitated N-acylated intermediate was collected and dried *in vacuo.* To a suspension of the dried intermediate in DMF (35 ml) was added a solution of DCC (1.45 g, 7.0 mmol) in dichloromethane (20 ml). The resulting mixture was stirred at room temperature for 20 h, and then filtered. The solvent was removed *in vacuo* to give a solid residue. The residue was recrystallised from a mixture of n-heptane (50 ml) and 2-propanol (2.5 ml) to give the title compound (2.5 g, 75 %).

### Example 21

### Synthesis of Arg³⁴,Lys²⁶ (N^{ε}-(γ-aminobutyroyl(N^{γ}-hexadecanoyl))) GLP-1 (7-37)-OH.

To a mixture of Arg³⁴, Lys²⁶ GLP-1 (7-37)-OH (41.1 mg, 12.2 µmol), EDPA (44 mg, 341 µmol), NMP (5.76 ml) and water (2.88 ml) was added a solution of Pal-GABA-ONSu (16 mg, 36.5 µmol, prepared as described in example 20) in NMP (400 µl). The reaction mixture was gently shaken for 5 min. at room temperature, and then allowed to stand for an additional 100 min. at room temperature. The reaction was quenched by the addition of a solution of glycine (20 mg, 268 µmol) in water (200 µl). The solvent was concentrated *in vacuo*, and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (15.8 mg, 35 %) was isolated, and the product was analysed by PDMS.

### Example 22

### Synthesis of N^{α}-hexadecanoyl-D-glutamic acid α-t-butyl ester-y-2,5-dioxopyrrolidin-1-yl ester.

A mixture of Pal-ONSu (6.64 g, 18.8 mmol), D-glutamic acid α-tert-butyl ester (4.5 g, 18.8 mmol) and EDPA (4.85 g, 37.5 mmol) in DMF (538 ml) was stirred at room temperature for 60 h. The solvent was removed and the residue dissolved in ethyl acetate (175 ml). The resulting solution was extracted with 10% aqueous citric acid (2x125 ml), and the organic phase concentrated *in vacuo.* The residue was dissolved in DMF (60 ml), and the resulting mixture slowly added to 10% aqueous citric acid (500 ml). The precipitated compound was collected and dried *in vacuo,* to give the crude N-acylated glutamic acid intermediate. The crude intermediate was dissolved in DMF (35 ml), and a solution of DCC (3.5 g, 17 mmol) in dichloromethane (70 ml) was added. The resulting mixture was stirred at room temperature for 20 h, and then filtered. The filtrate was concentrated *in vacuo,* and the solid residue recrystallised from a mixture of n-heptane (75 ml) and 2-propanol (5 ml), to give the title compound (5.2 g, 50 %)

### Example 23

### Synthesis of Arg³⁴,Lys²⁶ (N^{ε}-(γ-D-glutamyl(N^{α}-hexadecanoyl))) GLP-1 (7-37)-OH.

To a mixture of Arg³⁴, Lys²⁶ GLP-1 (7-37)-OH (41.1 mg, 12.2 µmol), EDPA (44 mg, 341 µmol), NMP (5.76 ml) and water (2.88 ml) was added a solution of N^{α}-hexadecanoyl-D-glutamic acid α-t-butyl ester-γ-2,5-dioxopyrrolidin-1-yl ester (19.7 mg, 36.5 µmol) in NMP (491 µl). The reaction mixture was gently shaken for 5 min. at room temperature, and then allowed to stand for an additional 95 min. at room temperature. The reaction was quenched by the addition of a solution of glycine (20 mg, 268 µmol) in water (2 ml). A 0.5 % aqueous solution of ammonium acetate (120 ml) was added, and the resulting mixture divided into to equal portions, and each portion eluted onto a Varian 5g C8 Mega Bond Elut^{®} , the immobilised compound washed with 5% aqueous acetonitril (25 ml), and finally liberated from the cartridge by elution with TFA (25 ml). The combined eluates were concentrated in *vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (10.5 mg, 23 %) was isolated, and the product was analysed by PDMS.

### Example 24

### Synthesis of Lys³⁴ (N^{ε}-(γ-glutamyl(N^{α}-tetradecanoyl))) GLP-1 (7-37).

To a mixture of GLP-1 (7-37)-OH (33.6 mg, 8.9 µmol), EDPA (32.4 mg, 250 µmol), NMP (2.1 ml) and water (2.1 ml) was added a solution of Myr-Glu(ONSu)-OBu^{t} (9.1 mg, 17.9 µmol), prepared as described in PCT application no. PCT/DK97/00340, in NMP (228 µl). The reaction mixture was gently shaken for 5 min., and then allowed to stand for an additional 80 min. at room temperature. The reaction was quenched by the addition of a solution of glycine (14.8 mg, 197 µmol) in water (1.47 ml). A 0.5% aqueous solution of ammonium acetate (100 ml) was added, and the resulting mixture divided into two equal portions, and each portion eluted onto a Varian 5g C8 Mega Bond Elut^{®}, the immobilised compound washed with 5% aqueous acetonitril (2x25 ml), and finally liberated from the cartridge by elution with TFA (2x25 ml). The combined eluates were concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (0.19 mg, 0.6%) was isolated, and the product was analysed by PDMS. The m/z value for the protonated molecular ion was found to be 3693 ± 3. The resulting molecular weight is thus 3692 ± 3 amu (theoretical value 3695 amu).

### Example 25

### Synthesis of Arg^{26,34},Lys⁸(N^{ε}-(γ-glutamyl(N^{α}-hexadecanoyl))) GLP-1 (7-37).

To a mixture of Arg^{26,34}, Lys⁸-GLP-1 (7-37)-OH (10.3 mg, 3 µmol), EDPA (10.8 mg, 83 µmol), NMP (1.44 ml) and water (0.72 ml) was added a solution of Pal-Glu(ONSu)-OBu^{t} (4.8 mg, 8.9 µmol), prepared as described in PCT application no. PCT/DK97/00340, in NMP (120 µl). The reaction mixture was gently shaken for 5 min., and then allowed to stand for an additional 70 min. at room temperature. The reaction was quenched by the addition of a solution of glycine (4.9 mg, 65.3 µmol) in water (490 µl). A 0.5% aqueous solution of ammonium acetate (30 ml) was added, and the resulting mixture eluted onto a Varian 5g C8 Mega Bond Elut', the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (3.2 mg, 28%) was isolated, and the product was analysed by PDMS. The m/z value for the protonated molecular ion was found to be 3836 ± 3. The resulting molecular weight is thus 3835 ± 3 AMU (theoretical value 3836 AMU).

### Example 26

### Synthesis of Lau-Glu(ONSu)-OBu^{t}.

To a solution of H-Glu-OBu^{t} (3 g, 15 mmol) in DMF (344 ml) was added EDPA (2.58 ml, 15 mmol) and a solution of Lau-ONSu (4.5 g, 15 mmol), prepared in a similar manner as described for Cap-ONSu in example 10, in DMF (74 ml). The resulting mixture was stirred at ambient temperature for 18 h, and the solvent removed *in vacuo.* The oily residue was partitioned between ethyl acetate (150 ml) and 5% aqueous citric acid (250 ml). The organic phase was concentrated *in vacuo.* The residue was dissolved in DMF (40 ml) and the solution added drop by drop to a 10% aqueous citric acid solution (350 ml). The precipitated product was collected, washed with water and dried *in vacuo* for 18 h to give the intermediate free acid. To solution of the free acid intermediate in DMF (25 ml) was added N-hydroxysuccinimide (1.7 g, 14.8 mmol) and a solution of N-(3-dimethylaminopropyl)-N'-ethylcarbodiimide (2.58 g, 13.5 mmol) in dichloromethane (52 ml). The resulting mixture was stirred at room temperature for 18 h, and the solvents removed *in vacuo.* The oily residue was partitioned between dichloromethane (80 ml) and water (80 ml). The organic phase was washed with 5% aqueous citric acid, dried (MgSO₄), and concentrated *in vacuo* to a solid. The solid residue was crystallised from a mixture of n-heptane (77 ml) and 2-propanol (50 ml), and finally recrystallised from n-heptane (76 ml) to give the title compound (2.96 g, 46%).

### Example 27

### Synthesis of Arg³⁴,Lys²⁶(N^{ε}-(γ-glutamyl(N^{α}-glutamyl(N^{α}-dodecanoyl))) GLP-1 (7-37).

To a mixture of Arg³⁴-GLP-1 (7-37)-OH (20.6 mg, 6.1 µmol), EDPA (22 mg, 171 µmol), NMP (2.88 ml) and water (1.44 ml) was added a solution Lau-Glu(ONSu)-OBu^{t} (10.2 mg, 21.2 µmol), prepared as described in example 26, in NMP (255 µl). The reaction mixture was gently shaken for 5 min., and then allowed to stand for an additional 75 min. at room temperature. The reaction was quenched by the addition of a solution of glycine (10 mg, 134 µmol) in water (100 µl). A 0.5% aqueous solution of ammonium acetate (61 ml) was added, and the resulting mixture eluted onto a Varian 5g C8 Mega Bond Elut^{®}, the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (8.2 mg, 36%) was isolated, and the product was analysed by PDMS. The m/z value for the protonated molecular ion was found to be 3693 ± 3. The resulting molecular weight is 3692 ± 3 AMU (theoretical value 3693 AMU).

### Example 28

### Synthesis of Lau-β-Ala-ONSu.

To a solution of Lau-ONSu (4.25 g, 14.3 mmol), prepared in a similar manner to in DMF (400 ml) was added EDPA (1.84 g, 14.3 mmol) and β-alanine (1.27 g, 14.3 mmol). The resulting mixture was stirred at ambient temperature for 18 h. Water (250 ml) and DMF (50 ml) were added and the solution stirred for 1 h at room temperature. The solvents were removed *in vacuo* to give a solid. The solid residue was dissolved in DMF (50 ml) and the solution added drop by drop to a 5% aqueous solution of citric acid (200 ml). The precipitate collected, washed with water (50 ml) and dried *in vacuo* to give the title compound (3.6 g, 93%).

### Example 29

### Synthesis of Pal-β-Ala-ONSu.

To a solution of Pal-ONSu (4.25 g, 14.3 mmol) in DMF (400 ml) was added EDPA (1.84 g, 14.3 mmol) and β-alanine (1.27 g, 14.3 mmol). The resulting mixture was stirred at ambient temperature for 18 h. Water (250 ml) and DMF (50 ml) were added and the solution stirred for 1 h at room temperature. The solvents were removed *in vacuo* to give a solid. The solid residue was dissolved in DMF (50 ml) and the solution added drop by drop to a 5% aqueous solution of citric acid (200 ml). The precipitate collected, washed with water (50 ml) and dried *in vacuo* to give the title compound (3.6 g, 93%).

### Example 30

### Synthesis of Myr-GABA-ONSu.

To a solution of Myr-ONSu (4 g, 12.3 mmol) in DMF (350 ml) was added EDPA (1.58 g, 12.3 mmol) and γ-aminobutyric acid (1.26 g, 12.3 mmol). The resulting mixture was stirred at ambient temperature for 18 h. Water (50 ml) was added and the solution stirred for 1 h at room temperature. The solvents were removed *in vacuo* to give a solid. The solid residue was dissolved in DMF (75 ml) and the solution added drop by drop to a 5% aqueous solution of citric acid (250 ml). The precipitate collected, washed with water (100 ml) and dried *in vacuo* to give the free acid intermediate (3.65 g, 95%). To a solution of the free acid intermediate (3 g, 9.6 mmol), N-hydroxysuccinimide (1.65 g, 14.4 mmol) and N-(3-dimethylaminopropyl)-N'-ethylcarbodiimide hydrochloride (3.67 g, 19.1 mmol) in DMF (330 ml) was stirred for 18 h at room temperature, and the solvent removed *in vacuo* to give a solid. The solid residue was dissolved in dichloromethane (100 ml) and washed with brine (100 ml). The organic phase was dried (MgSO₄) and concentrated *in vacuo* to give a solid. The solid residue was recrystallised from n-heptane (75 ml) to give the title compound (2.8 g, 71 %).

### Example 31

### Synthesis of Pal-β-Ala-ONSu.

To a solution of Pal-ONSu (0.9 g, 2.8 mmol) in DMF (100 ml) were added N-hydroxysuccinimide (0.35 g, 3 mmol) and N-(3-dimethylaminopropyl)-N'-ethylcarbodiimide (0.79 g, 4.1 mmol). The resulting mixture was stirred at ambient temperature for 40h, and the solvent removed *in vacuo.* The solid residue was partitioned between water (50 ml) and dichloromethane (50 ml). The organic phase was separated, dried (MgSO₄) and the solvent removed *in vacuo* to give the title compound (1.1 g, 94%).

### Example 32

### Synthesis of Arg³⁴,Lys²⁶(N^{ε}-(N^{ε}-alanyl(N^{α}-hexadecanoyl))) GLP-1 (7-37).

To a mixture of Arg³⁴-GLP-1 (7-37)-OH (19.2 mg, 5.7 µmol), EDPA (20.5 mg, 159 µmol), NMP (2.7 ml) and water (1.35 ml) was added a solution Pal-[β-Ala-ONSu (7.2 mg, 17 µmol), prepared as described in example 31, in NMP (181 µl). The reaction mixture was gently shaken for 5 min., and then allowed to stand for an additional 90 min. at room temperature. The reaction was quenched by the addition of a solution of glycine (9.3 mg, 125 µmol) in water (93 µl). The reaction mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (11.6 mg, 55%) was isolated, and the product was analysed by PDMS. The m/z value for the protonated molecular ion was found to be 3694 ± 3. The resulting molecular weight is thus 3693 ± 3 AMU (theoretical value 3693 AMU).

### Example 33

### Synthesis of Pal-Glu(OBu^{t})-ONSu.

To a solution of H-Glu(OH)-OBu^{t} (2.7 g, 11.3 mmol) and Pal-ONSu (3.98 g, 11.3 mmol) in DMF (300 ml) was added EDPA (3.2 g, 24.8 mmol). The resulting mixture was stirred at ambient temperature for 18h, and the solvent concentrated *in vacuo* to give an oil. The oily residue was dissolved in DMF (60 ml) and the solution added drop by drop to a 10% aqueous solution of citric acid (300 ml) whereby a precipitate was formed. The precipitate was collected, washed with cold water (25 ml), and dried *in vacuo* to give free acid intermediate (4.44 g, 89%). The free acid intermediate (4 g, 9.1 mmol) was dissolved in DMF (50 ml) and N-hydroxysuccinimide (1.15 g, 10 mmol) and N-(3-dimethylaminopropyl)-N'-ethylcarbodiimide hydrochloride (2.6 g, 13.6 mmol) were added. The resulting mixture was stirred at room temperature for 60h, the solvent concentrated *in vacuo* to give the crude title compound (8.2 g)

### Example 34

### Synthesis of Arg³⁴,Lys²⁶(N^{ε}-(α-glutamyl(N^{α}-hexadecanoyl))) GLP-1 (7-37).

To a mixture of Arg³⁴-GLP-1 (7-37)-OH (25.6 mg, 7.6 µmol), EDPA (27.4 mg, 212 µmol), NMP (3.5 ml) and water (1.75 ml) was added a solution of Pal-Glu(OBu^{t})-ONSu (12.2 mg, 22.7 µmol), prepared as described in example 33, in NMP (305 µl). The reaction mixture was gently shaken for 5 min., and then allowed to stand for an additional 100 min. at room temperature. The reaction was quenched by the addition of a solution of glycine (12.5 mg, 168 µmol) in water (125 µl). A 0.5% aqueous solution of ammonium acetate (72.5 ml) was added, and the resulting mixture eluted onto a Varian 5g C8 Mega Bond Elut^{®}, the immobilised compound washed with 5% aqueous acetonitril (25 ml), and finally liberated from the cartridge by elution with TFA (30 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (6.1 mg, 22%) was isolated, and the product was analysed by PDMS. The m/z value for the protonated molecular ion was found to be 3751 ± 3. The resulting molecular weight is thus 3750 ± 3 AMU (theoretical value 3751 AMU).

### Example 35

### Synthesis of Ste-GABA-ONSu.

To a solution of Ste-ONSu (3 g, 7.9 mmol) in DMF (270 ml) was added EDPA (1 g, 7.9 mmol) and a solution of γ-aminobutyric acid (0.81 g, 7.9 mmol) in water (40 ml). The resulting suspension was stirred at ambient temperature for 18 h, and then concentrated in *vacuo* to a final volume of 50 ml. The resulting suspension was added to a 5% aqueous solution of citric acid (500 ml) whereby a precipitate is formed. The precipitate was collected and washed with water (50 ml), and dried *in vacuo* for 4h to give the free acid intermediate (2.8 g, 97%). To a mixture of the free acid intermediate (2.6 g, 7 mmol), N-hydroxysuccinimide (1.21 g, 10.5 mmol) and N-(3-dimethylaminopropyl)-N'-ethylcarbodiimide hydrochloride (2.69 g, 14 mmol) in NMP (300 ml) was stirred for 70 h, and the solvent removed *in vacuo* to give a solid. The solid residue was dissolved in dichloromethane (100 ml) and washed with brine (2x100 ml). The organic phase was dried (MgSO₄) and concentrated *in vacuo* to give a solid. The solid residue was recrystallised from n-heptane (75 ml) to give the title compound (2.2 g, 67%).

### Example 36

### Synthesis of Pal-isonip-ONSu.

To a suspension of 1-hexadecanoylbenzotriazole (3 g, 8.4 mmol), prepared as described in the literature (Kreutzberger; van der Goot, Arch.Pharm., 307, 1974), in DMF (350 ml) were added EDPA (1.08 g, 8.4 mmol) and a solution of piperidine-4-carboxylic acid in water ( 20 ml). The resulting suspension was stirred at room temperature for 12d, and then concentrated *in vacuo* to an oil. The oily residue was added drop by drop to a 5% aqueous solution of citric acid (300 ml) whereby a precipitate was formed. The precipitate was collected and washed with water (50 ml), dried *in vacuo* for 2 h to give the free acid intermediate (3 g, 97%). To a solution of the free acid intermediate (2.8 g, 7.6 mmol), N-hydroxysuccinimide (1.31 g, 11.4 mmol) in DMF (250 ml) was added N-(3-dimethylaminopropyl)-N'-ethylcarbodiimide hydrochloride (2.92 g, 15.2 mmol). The resulting mixture was stirred at ambient temperature for 18h, and the solvent removed *in vacuo to* give an oil. The oily residue was dissolved in dichloromethane (100 ml), washed with brine (50 ml), dried (MgSO₄) and concentrated *in vacuo* to give the crude title compound (4.1 g, quant.).

### Example 37

### Synthesis of Arg³⁴,Lys²⁶(N^{ε}-(piperidinyl-4-carbonyl(N-hexadecanoyl))) GLP-1 (7-37).

To a mixture of Arg³⁴-GLP-1 (7-37)-OH (25 mg, 7.4 µmol), EDPA (26.7 mg, 207 µmol), NMP (3.5 ml) and water (1.75 ml) was added a solution Pal-Isonip-ONSu (13.7 mg, 30 µmol), prepared as described in example 36 in NMP (343 µl). The reaction mixture was gently shaken for 5 min., and then allowed to stand for an additional 90 min. at room temperature. The reaction was quenched by the addition of a solution of glycine (12.2 mg, 163 µmol) in water (122 µl). The reaction mixture was purified by column chromatography using a cyanopropyl column (Zorbax 300SB-CN) and a standard acetonitril/TFA system. The column was heated to 65°C and the acetonitril gradient was 0-100% in 60 minutes. The title compound (12 mg, 44%) was isolated, and the product was analysed by PDMS. The m/z value for the protonated molecular ion was found to be 3734 ± 3. The resulting molecular weight is thus 3733 ± 3 AMU (theoretical value 3733 AMU).

### Example 38

### Synthesis of Arg³⁴,Lys²⁶(N^{ε}-(γ-glutamyl(N^{α}-decanoyl))) GLP-1 (7-37)

To a mixture of Arg³⁴-GLP-1 (7-37)-OH (25 mg, 7.4 µmol), EDPA (26.7 mg, 207 µmol), NMP (3.5 ml) and water (1.75 ml) was added a solution of Cac-Giu(ONSu)-OBu' (10 mg, 22.1 µmol) in NMP (252 µl). The reaction mixture was gently shaken for 5 min., and then allowed to stand for an additional 140 min. at room temperature. The reaction was quenched by the addition of a solution of glycine (12.2 mg, 162 µmol) in water (122 µl). A 0.5% aqueous solution of ammonium acetate (73 ml) was added, and the resulting mixture eluted onto a Varian 5g C8 Mega Bond Elut^{®}, the immobilised compound washed with 5% aqueous acetonitril (25 ml), and finally liberated from the cartridge by elution with TFA (25 ml). The eluate was concentrated *in vacuo,* and the residue purified by column chromatography using a cyanopropyl column (Zorbax 300SB-CN) and a standard acetonitril/TFA system. The column was heated to 65°C and the acetonitril gradient was 0-100% in 60 minutes. The title compound (12.2 mg, 45%) was isolated, and the product was analysed by PDMS. The m/z value for the protonated molecular ion was found to be 3669.7 ± 3. The resulting molecular weight is thus 3668.7 ± 3 amu (theoretical value 3667 amu).

### BIOLOGICAL FINDINGS

### Protraction of GLP-1 derivatives after s.c. administration

The protraction of a number GLP-1 derivatives of the invention was determined by monitoring the concentration thereof in plasma after sc administration to healthy pigs, using the method described below. For comparison also the concentration in plasma of GLP-1(7-37) after sc. administration was followed. The protraction of other GLP-1 derivatives of the invention can be determined in the same way.

Pigs (50% Duroc, 25% Yorkshire, 25% Danish Landrace, app 40 kg) were fasted from the beginning of the experiment. To each pig 0.5 nmol of test compound per kg body weight was administered in a 50 µM isotonic solution (5 mM phosphate, pH 7.4, 0.02% Tween^{®}-20 (Merck), 45 mg/ml mannitol (pyrogen free, Novo Nordisk). Blood samples were drawn from a catheter in vena jugularis at the hours indicated in Table 1. 5 ml of the blood samples were poured into chilled glasses containing 175 µl of the following solution: 0.18 M EDTA, 1500 KIE/ml aprotinin (Novo Nordisk) and 3% bacitracin (Sigma), pH 7.4. Within 30 min, the samples were centrifuged for 10 min at 5-6000*g. Temperature was kept at 4°C. The supernatant was pipetted into different glasses and kept at minus 20°C until use.

The plasma concentrations of the peptides were determined by RIA using a monoclonal antibody specific for the N-terminal region of GLP-1 (7-37). The cross reactivities were less than 1% with GLP-1(1-37) and GLP-1(8-36)amide and < 0.1% with GLP-1(9-37), GLP-1(10-36)amide and GLP-1(11-36)amide. The entire procedure was carried out at 4°C. The assay was carried out as follows: 100 µl plasma was mixed with 271 µl 96% ethanol, mixed using a vortex mixer and centrifuged at 2600*g for 30 min. The supernatant was decanted into Minisorp tubes and evaporated completely (Savant Speedvac AS290). The evaporation residue was reconstituted in the assay buffer consisting of 80 mM NaH₂PO₄/Na₂HPO₄, 0.1 % HSA (Orpha 20/21, Behring), 10 mM EDTA, 0.6 mM thiomersal (Sigma), pH 7.5. Samples were reconstituted in volumes suitable for their expected concentrations, and were allowed to reconstitute for 30 min. To 300 µl sample, 100 µl antibody solution in dilution buffer containing 40 mM NaH₂PO₄/Na₂HPO₄, 0.1 % HSA, 0.6 mM thiomersal, pH 7.5, was added. A non-specific sample was prepared by mixing 300 µl buffer with 100 µl dilution buffer. Individual standards were prepared from freeze dried stocks, dissolved in 300 µl assay buffer. All samples were pre-incubated in Minisorp tubes with antibody as described above for 72 h. 200 µl tracer in dilution buffer containing 6-7000 CPM was added, samples were mixed and incubated for 48 h. 1.5 ml of a suspension of 200 ml per litre of heparin-stabilised bovine plasma and 18 g per litre of activated carbon (Merck) in 40 mM NaH₂PO₄/Na₂HPO₄, 0.6 mM thiomersal, pH 7.5, was added to each tube. Before use, the suspension was mixed and allowed to stand for 2 h at 4°C. All samples were incubated for 1 h at 4°C and then centrifuged at 3400*g for 25 min. Immediately after the centrifugation, the supernatant was decanted and counted in a γ-counter. The concentration in the samples was calculated from individual standard curves.

The findings show that the GLP-1 derivatives of the invention have a protracted profile of action relative to GLP-1 (7-37) and are much more persistent in plasma than GLP-1(7-37). The time at which the peak concentration in plasma is achieved varies within wide limits, depending on the particular GLP-1 derivative selected.

### Stimulation of cAMP formation in a cell line expressing the cloned human GLP-1 receptor

In order to demonstrate efficacy of the GLP-1 derivatives, their ability to stimulate formation of cAMP in a cell line expressing the cloned human GLP-1 receptor was tested. An EC₅₀ was calculated from the dose-response curve.

Baby hamster kidney (BHK) cells expressing the human pancreatic GLP-1 receptor were used (Knudsen and Pridal, 1996, Eur. J. Pharm. 318, 429-435). Plasma membranes were prepared (Adelhorst et al, 1994, J. Biol. Chem. 269, 6275) by homogenisation in buffer (10 mmol/I Tris-HCl and 30 mmol/I NaCl pH 7.4, containing, in addition, 1 mmol/I dithiothreitol, 5 mg/l leupeptin (Sigma, St. Louis, MO, USA), 5 mg/l pepstatin (Sigma, St. Louis, MO, USA), 100 mg/l bacitracin (Sigma, St. Louis, MO, USA), and 16 mg/l aprotinin (Novo Nordisk A/S, Bagsvaerd, Denmark)). The homogenate was centrifuged on top of a layer of 41 w/v% sucrose. The white band between the two layers was diluted in buffer and centrifuged. Plasma membranes were stored at -80°C until used.

The assay was carried out in 96-well microtiter plates in a total volume of 140 µl. The buffer used was 50 mmol/I Tris-HCl, pH 7.4 with the addition of 1 mmol/I EGTA, 1.5 mmol/l MgSO₄, 1.7 mmol/I ATP, 20 mM GTP, 2 mmol/l 3-isobutyl-1-methylxanthine, 0.01 % Tween-20 and 0.1 % human serum albumin (Reinst, Behringwerke AG, Marburg, Germany). Compounds to be tested for agonist activity were dissolved and diluted in buffer, added to the membrane preparation and the mixture was incubated for 2 h at 37°C. The reaction was stopped by the addition of 25 µl of 0.05 mol/l HCl. Samples were diluted 10 fold before analysis for cAMP by a scintillation proximity assay (RPA 538, Amersham, UK).

## Claims

1. A derivative of a GLP-1 analog, which derivative has a protracted profile of action relative to GLP-1 (7-37), which GLP-1 analog is of formula I: 
 7   8    9  10  11  12  13  14  15  16  17
His-Xaa-Xaa-Gly-Xaa-Phe-Thr-Xaa-Asp-Xaa-Xaa-

18  19  20  21  22  23  24  25  26  27  28
Xaa-Xaa-Xaa-Xaa-Xaa-Xaa-Xaa-Xaa-Xaa-Xaa-Phe-

29  30  31  32  33  34  35  36  37  38
Ile-Xaa-Xaa-Xaa-Xaa-Xaa-Xaa-Xaa-Xaa-Xaa

39  40  41  42  43  44  45
Xaa-Xaa-Xaa-Xaa-Xaa-Xaa-Xaa
 wherein
Xaa at position 8 is Ala, Gly, Ser, Thr, Leu, Ile, Val, Glu or Asp,
Xaa at position 9 is Glu or Asp,
Xaa at position 11 is Thr, Ala, Gly, Ser, Leu, Ile, Val, Glu or Asp,
Xaa at position 14 is Ser, Ala, Gly, Thr, Leu, Ile, Val, Glu or Asp,
Xaa at position 16 is Val, Ala, Gly, Ser, Thr, Leu, Ile, Tyr, Glu or Asp,
Xaa at position 17 is Ser, Ala, Gly, Thr, Leu, Ile, Val, Glu or Asp,
Xaa at position 18 is Ser, Ala, Gly, Thr, Leu, Ile, Val, Glu or Asp,
Xaa at position 19 is Tyr, Phe, Trp, Glu or Asp,
Xaa at position 20 is Leu, Ala, Gly, Ser, Thr, Leu, Ile, Val, Glu or Asp,
Xaa at position 21 is Glu or Asp,
Xaa at position 22 is Gly, Ala, Ser, Thr, Leu, Ile, Val, Glu or Asp,
Xaa at position 23 is Gln, Asn, Arg, Glu or Asp,
Xaa at position 24 is Ala, Gly, Ser, Thr, Leu, Ile, Val, Arg, Glu or Asp,
Xaa at position 25 is Ala, Gly, Ser, Thr, Leu, Ile, Val, Glu or Asp,
Xaa at position 26 is Arg, Gln, Glu, Asp, or His,
Xaa at position 27 is Glu or Asp,
Xaa at position 30 is Ala, Gly, Ser, Thr, Leu, Ile, Val, Glu or Asp,
Xaa at position 31 is Trp, Phe, Tyr, Glu or Asp,
Xaa at position 32 is Leu, Gly, Ala, Ser, Thr, Ile, Val, Glu or Asp,
Xaa at position 33 is Val, Gly, Ala, Ser, Thr, Leu, Ile, Glu or Asp,
Xaa at position 34 is Arg, Glu, Asp, or His,
Xaa at position 35 is Gly, Ala, Ser, Thr, Leu, Ile, Val, Glu or Asp,
Xaa at position 36 is Arg, Lys, Glu, Asp, or His,
Xaa at position 37 is Gly, Ala, Ser, Thr, Leu, Ile, Val, Glu, Asp, or Lys, or is deleted,
Xaa at position 38 is Arg, Lys, Glu, Asp, or His, or is deleted,
Xaa at position 39 is Arg, Lys, Glu, Asp, or His, or is deleted,
Xaa at position 40 is Asp, Glu, or Lys, or is deleted,
Xaa at position 41 is Phe, Trp, Tyr, Glu, Asp, or Lys, or is deleted,
Xaa at position 42 is Pro, Lys, Glu, or Asp, or is deleted,
Xaa at position 43 is Glu, Asp, or Lys, or is deleted,
Xaa at position 44 is Glu, Asp, or Lys, or is deleted, and
Xaa at position 45 is Val, Glu, Asp, or Lys, or is deleted, or
(a) a C-1-6-ester thereof, (b) amide, C-1-6-alkylamide, or C-1-6-dialkylamide thereof and/or (c) a pharmaceutically acceptable salt thereof,
provided that
A. when the amino acid at position 37, 38, 39, 40, 41, 42, 43 or 44 is deleted, then each amino acid downstream of the amino acid is also deleted,
B. the derivative of the GLP-1 analog contains only one Lys at the carboxy terminus, and Glu or Asp is adjacent to Lys,
C. the ε-amino group of Lys is substituted with a lipophilic substituent via a spacer,
D. the total number of different amino acids between the derivative of the GLP-1 analog and the corresponding native form of GLP-1 does not exceed six,
E. the derivative of GLP-1 analog of formula I is not selected from:
Glu^{22,23,30}Arg^{26,34}Lys³⁸(N^{ε}-(γ-glutamyl(N^{α}-tetradecanoyl)))-GLP-1(7-38)OH,
Glu^{23,26}Arg³⁴Lys³⁸(N^{ε}-(γ-glutamyl(N^{α}-tetradecanoyl)))-GLP-1(7-38)-OH,
Arg^{26,36}Lys³⁸(N^{ε}-(γ-glutamyl(N^{α}-tetradecanoyl)))-GLP-1 (7-38)-OH,
Arg^{26,34}Lys³⁸(N^{ε}-(γ-glutamyl(N^{α}-hexadecanoyl)))-GLP-1 (7-38)-OH,
Arg^{26,34},Lys³⁸(N^{ε}-(γ-glutamyl(N^{α}-octadecanoyl)))-GLP-1 (7-38)-OH.

2. The derivative of GLP-1 analog of claim 1, wherein the amino acids at positions 37-45 are absent.

3. The derivative of GLP-1 analog of claim 1, wherein the amino acids at positions 38-45 are absent.

4. The derivative of GLP-1 analog of claim 1, wherein the amino acids at positions 39-45 are absent.

5. The derivative of GLP-1 analog of any of claims 1-4, wherein Xaa at position 8 is Ala, Gly, Ser, Thr, or Val.

6. The derivative of GLP-1 analog of any of claims 1-5, wherein Xaa at position 9 is Glu.

7. The derivative of GLP-1 analog of any of claims 1-6, wherein Xaa at position 11 is Thr.

8. The derivative of GLP-1 analog of any of claims 1-7, wherein Xaa at position 14 is Ser.

9. The derivative of GLP-1 analog of any of claims 1-8, wherein Xaa at position 16 is Val.

10. The derivative of GLP-1 analog of any of claims 1-9, wherein Xaa at position 17 is Ser.

11. The derivative of GLP-1 analog of any of claims 1-10, wherein Xaa at position 18 is Ser, Glu, or Asp.

12. The derivative of GLP-1 analog of any of claims 1-11, wherein Xaa at position 19 is Tyr, Glu, or Asp.

13. The derivative of GLP-1 analog of any of claims 1-12, wherein Xaa at position 20 is Leu, Glu, or Asp.

14. The derivative of GLP-1 analog of any of claims 1-13, wherein Xaa at position 21 is Glu, or Asp.

15. The derivative of GLP-1 analog of any of claims 1-14, wherein Xaa at position 22 is Gly, Glu ir Asp.

16. The derivative of GLP-1 analog of any of claims 1-15, wherein Xaa at position 23 is Gln, Glu, or Asp.

17. The derivative of GLP-1 analog of any of claims 1-16, wherein Xaa at position 24 is Ala, Glu or Asp.

18. The derivative of GLP-1 analog of any of claims 1-17, wherein Xaa at position 25 is Ala, Glu or Asp.

19. The derivative of GLP-1 analog of any of claims 1-18, wherein Xaa at position 26 is Glu, Asp, or Arg.

20. The derivative of GLP-1 analog any of claims 1-19, wherein Xaa at position 27 is Glu or Asp.

21. The derivative of GLP-1 analog of any of claims 1-20, wherein Xaa at position 30 is Ala, Glu or Asp.

22. The derivative of GLP-1 analog of any of claims 1-21, wherein Xaa at position 31 is Trp, Glu or Asp.

23. The derivative of GLP-1 analog of any of claims 1-22, wherein Xaa at position 32 is Leu, Glu or Asp.

24. The derivative of GLP-1 analog of any of claims 1-23, wherein Xaa at position 33 is Val, Glu or Asp.

25. The derivative of GLP-1 analog of any of claims 1-24, wherein Xaa at position 34 is Arg, Glu, or Asp.

26. The derivative of GLP-1 analog of any of claims 1-25, wherein Xaa at position 35 is Gly, Glu or Asp.

27. The derivative of GLP-1 analog of any of claims 1-26, wherein Xaa at position 36 is Arg, Lys, Glu, or Asp.

28. The derivative of GLP-1 analog of any of claims 1-27, wherein Xaa at position 37 is Gly, Glu, Asp, or Lys.

29. The derivative of GLP-1 analog of any of claims 1-31, wherein Xaa at position 38 is Arg or Lys.

30. The derivative of GLP-1 analog of any one of the preceding claims, wherein the lipophilic substituent comprises from 4 to 40 carbon atoms, more preferred from 8 to 25 carbon atoms.

31. The derivative of GLP-1 analog of any one of the preceding claims, wherein the spacer is Glu or Asp, the carboxyl group of which forms an amide bond with an amino group of the amino acid residue, and the amino group of which forms an amide bond with a carboxyl group of the lipophilic substituent.

32. The derivative of GLP-1 analog of any one of the preceding claims, wherein the spacer is N-epsilon-(gamma-L-glutamyl) or N-epsilon-(beta-L-asparagyl)).

33. The derivative of GLP-1 analog of claim 34, wherein the ε-amino group of Lys forms an amide bond with a carboxylic group of the amino acid residue spacer, and an amino group of the amino acid residue spacer forms an amide bond with a carboxyl group of the lipophilic substituent.

34. The derivative of GLP-1 analog of any one of the preceding claims, wherein the lipophilic substituent comprises a partially or completely hydrogenated cyclopentanophenathrene skeleton.

35. The derivative of GLP-1 analog of any of the claims 1-33, wherein the lipophilic substituent is an straight-chain or branched alkyl group.

36. The derivative of GLP-1 analog of any of the claims 1-33 wherein the lipophilic substituent is the acyl group of a straight-chain or branched fatty acid.

37. The derivative GLP-1 analog of claim 36 wherein the acyl group is selected from the group comprising CH₃(CH₂)ₙCO-, wherein n is 4 to 38, preferably CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO-and CH₃(CH₂)₂₂CO-.

38. The derivative of GLP-1 analog of any one of the claims 1-33 wherein the lipophilic substituent is an acyl group of a straight-chain or branched alkane (α,ω-dicarboxylic acid.

39. The derivative of GLP-1 analog of claim 38 wherein the acyl group is selected from the group comprising HOOC(CH₂)ₘCO-, wherein m is from 4 to 38, preferably from 4 to 24, more preferred selected from the group comprising HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.

40. The derivative of GLP-1 analog of any one of the claims 1-33, wherein the lipophilic substituent is a group of the formula CH₃(CH₂)ₚ((CH₂)_{q}COOH)CHNH-CO(CH₂)₂CO-, wherein p and q are integers and p+q is an integer of from 8 to 33, preferably from 12 to 28.

41. The derivative of GLP-1 analog of any one of the claims 1-33, wherein the lipophilic substituent is a group of the formula CH₃(CH₂)ᵣCO-NHCH(COOH)(CH₂)₂CO-, wherein r is an integer of from 10 to 24.

42. The derivative of GLP-1 analog of any one of the claims 1-33, wherein the lipophilic substituent is a group of the formula CH₃(CH₂)ₛCO-NHCH((CH₂)₂COOH)CO-, wherein s is an integer of from 8 to 24.

43. The derivative of GLP-1 analog of any one of the claims 1-33, wherein the lipophilic substituent is a group of the formula -NHCH(COOH)(CH₂)₄NH-CO(CH₂)ᵤCH₃, wherein u is an integer of from 8 to 18.

44. The derivative of GLP-1 analog of any one of the claims 1-33, wherein the lipophilic substituent is a group of the formula -NHCH(COOH)(CH₂)₄NH-COCH((CH₂)₂COOH)NH-CO(CH₂)_{w}CH₃, wherein w is an integer of from 10 to 16.

45. The derivative of GLP-1 analog of any one of the claims. 1-33, wherein the lipophilic substituent is a group of the formula -NHCH(COOH)(CH₂)₄NH-CO(CH₂)₂CH(COOH)NH-CO(CH₂)ₓCH₃, wherein x is an integer of from 10 to 16.

46. The derivative of GLP-1 analog of any one of the claims 1-33, wherein the lipophilic substituent is a group of the formula -NHCH(COOH)(CH₂)₄NH-CO(CH₂)₂CH(COOH)NH-CO(CH₂)_{y}CH₃, wherein y is zero or an integer of from 1 to 22.

47. A pharmaceutical composition comprising a derivative of GLP-1 analog of any of claims 1-46 and a pharmaceutically acceptable vehicle or carrier.

48. The pharmaceutical composition of claim 47, further comprising another antidiabetic agent.

49. The pharmaceutical composition of claim 48, wherein the antidiabetic agent is an insulin, more preferably human insulin.

50. The pharmaceutical composition of claim 48, wherein the antidiabetic agent is a hypoglaemic agent.

51. Use of a derivative of GLP-1 analog of any of claims 1-46 for the preparation of a medicament with a protracted profile of action relative to GLP-1 (7-37), for the treatment of non-insulin dependent diabetes mellitus.

52. Use of a derivative of GLP-1 analog of any of claims 1-46 for the preparation of a medicament with a protracted profile of action relative to GLP-1 (7-37), for the treatment of insulin dependent diabetes mellitus.

53. Use of a derivative of GLP-1 analog of any of claims 1-46 for the preparation of a medicament with a protracted profile of action relative to GLP-1 (7-37), for the treatment of obesity.

54. A GLP-1 derivative of any one of claims 1-46 for use as a medicament.

55. A GLP-1 derivative of any one of claims 1-46 for use as a medicament in the treatment of non-insulin dependent diabetes mellitus, insulin dependent diabetes mellitus, and/or obesity.

56. Use of a GLP-1 derivative of any one of claims 1-46 for the preparation of a medicament for the treatment of non-insulin dependent diabetes mellitus, insulin dependent diabetes mellitus, and/or obesity.

## Patentansprüche

1. Derivat eines GLP-1-Analogons, wobei das Derivat ein verzögertes Wirkungsprofil in Bezug auf GLP-1(7-37) aufweist, wobei das GLP-1-Analogon die Formel I aufweist: 
 7   8   9  10  11  12  13  14  15  16  17
His-Xaa-Xaa-Gly-Xaa-Phe-Thr-Xaa-Asp-Xaa-Xaa-

18  19  20  21  22  23  24  25  26  27  28
Xaa-Xaa-Xaa-Xaa-Xaa-Xaa-Xaa-Xaa-Xaa-Xaa-Phe-

29  30  31  32  33  34  35  36  37  38
Ile-Xaa-Xaa-Xaa-Xaa-Xaa-Xaa-Xaa-Xaa-Xaa

39  40  41  42  43  44  45
Xaa-Xaa-Xaa-Xaa-Xaa-Xaa-Xaa
 wobei
Xaa an Position 8 für Ala, Gly, Ser, Thr, Leu, Ile, Val, Glu oder Asp steht,
Xaa an Position 9 für Glu oder Asp steht,
Xaa an Position 11 für Thr, Ala, Gly, Ser, Leu, Ile, Val, Glu oder Asp steht,
Xaa an Position 14 für Ser, Ala, Gly, Thr, Leu, Ile, Val, Glu oder Asp steht,
Xaa an Position 16 für Val, Ala, Gly, Ser, Thr, Leu, Ile, Tyr, Glu oder Asp steht,
Xaa an Position 17 für Ser, Ala, Gly, Thr, Leu, Ile, Val, Glu oder Asp steht,
Xaa an Position 18 für Ser, Ala, Gly, Thr, Leu, Ile, Val, Glu oder Asp steht,
Xaa an Position 19 für Tyr, Phe, Trp, Glu oder Asp steht,
Xaa an Position 20 für Leu, Ala, Gly, Ser, Thr, Leu, Ile, Val, Glu oder Asp steht,
Xaa an Position 21 für Glu oder Asp steht,
Xaa an Position 22 für Gly, Ala, Ser, Thr, Leu, Ile, Val, Glu oder Asp steht,
Xaa an Position 23 für Gln, Asn, Arg, Glu oder Asp steht,
Xaa an Position 24 für Ala, Gly, Ser, Thr, Leu, Ile, Val, Arg, Glu oder Asp steht,
Xaa an Position 25 für Ala, Gly, Ser, Thr, Leu, Ile, Val, Glu oder Asp steht,
Xaa an Position 26 für Arg, Gln, Glu, Asp oder His steht,
Xaa an Position 27 für Glu oder Asp steht,
Xaa an Position 30 für Ala, Gly, Ser, Thr, Leu, Ile, Val, Glu oder Asp steht,
Xaa an Position 31 für Trp, Phe, Tyr, Glu oder Asp steht,
Xaa an Position 32 für Leu, Gly, Ala, Ser, Thr, Ile, Val, Glu oder Asp steht,
Xaa an Position 33 für Val, Gly, Ala, Ser, Thr, Leu, Ile, Glu oder Asp steht,
Xaa an Position 34 für Arg, Glu, Asp oder His steht,
Xaa an Position 35 für Gly, Ala, Ser, Thr, Leu, Ile, Val, Glu oder Asp steht,
Xaa an Position 36 für Arg, Lys, Glu, Asp oder His steht,
Xaa an Position 37 für Gly, Ala, Ser, Thr, Leu, Ile, Val, Glu, Asp oder Lys steht oder entfernt ist,
Xaa an Position 38 für Arg, Lys, Glu, Asp, oder His steht oder entfernt ist,
Xaa an Position 39 für Arg, Lys, Glu, Asp oder His steht oder entfernt ist,
Xaa an Position 40 für Asp, Glu oder Lys steht oder entfernt ist,
Xaa an Position 41 für Phe, Trp, Tyr, Glu, Asp oder Lys steht oder entfernt ist,
Xaa an Position 42 für Pro, Lys, Glu oder Asp steht oder entfernt ist,
Xaa an Position 43 für Glu, Asp oder Lys steht oder entfernt ist,
Xaa an Position 44 für Glu, Asp oder Lys steht oder entfernt ist und
Xaa an Position 45 für Val, Glu, Asp oder Lys oder entfernt ist, oder
(a) ein C-1-6-Ester davon (b) Amid, C-1-6-Alkylamid oder C-1-6-Dialkylamid davon und/oder (c) ein pharmazeutisch verträgliches Salz davon,
mit der Maßgabe, dass
A. falls die Aminosäure an Position 37, 38, 39, 40, 41, 42, 43 oder 44 entfernt ist, jede Aminosäure stromabwärts dann ebenfalls entfernt ist,
B. das Derivat des GLP-1-Analogons nur ein Lys am Carboxyterminus enthält und Glu oder Asp mit Lys benachbart ist,
C. die ε-Aminogruppe von Lys mit einem lipophilen Substituenten über einen Abstandhalter substituiert ist,
D. die Gesamtanzahl von verschiedenen Aminosäuren zwischen dem Derivat des GLP-1-Analogons und der entsprechenden nativen Form nicht sechs übersteigt,
E. das Derivat eines GLP-1-Analogons der Formel I nicht ausgewählt ist aus:
Glu^{22,23,30}Arg^{26,34}Lys³⁸(N^{ε}-(γ-Glutamyl(N^{α}-tetradecanoyl)))-GLP-1 (7-38)-OH,
Glu^{23,26}Arg³⁴Lys³⁸(N^{ε}-(γ-Glutamyl(N^{α}-tetradecanoyl)))-GLP-1 (7-38)-OH,
Arg^{26,34}Lys³⁸(N^{ε}-(γ-Glutamyl(N^{α}-tetradecanoyl)))-GLP-1(7-38)-OH,
Arg^{26,34}Lys³⁸(N^{ε}-(γ-Glutamyl(N^{α}-hexadecanoyl)))-GLP-1(7-38)-OH,
Arg^{26,34}Lys³⁸(N^{ε}-(γ-Glutamyl(N^{α}-octadecanoyl)))-GLP-1(7-38)-OH.

2. Derivat eines GLP-1-Analogons nach Anspruch 1, wobei die Aminosäuren an den Positionen 37-45 fehlen.

3. Derivat eines GLP-1-Analogons nach Anspruch 1, wobei die Aminosäuren an den Positionen 38-45 fehlen.

4. Derivat eines GLP-1-Analogons nach Anspruch 1, wobei die Aminosäuren an den Positionen 39-45 fehlen.

5. Derivat eines GLP-1-Analogons nach einem der Ansprüche 1-4, wobei Xaa an Position 8 für Ala, Gly, Ser, Thr oder Val steht.

6. Derivat eines GLP-1-Analogons nach einem der Ansprüche 1-5, wobei Xaa an Position 9 für Glu steht.

7. Derivat eines GLP-1-Analogons nach einem der Ansprüche 1-6, wobei Xaa an Position 11 für Thr steht.

8. Derivat eines GLP-1-Analogons nach einem der Ansprüche 1-7, wobei Xaa an Position 14 für Ser steht.

9. Derivat eines GLP-1-Analogons nach einem der Ansprüche 1-8, wobei Xaa an Position 16 für Val steht.

10. Derivat eines GLP-1-Analogons nach einem der Ansprüche 1-9, wobei Xaa an Position 17 für Ser steht.

11. Derivat eines GLP-1-Analogons nach einem der Ansprüche 1-10, wobei Xaa an Position 18 für Ser, Glu oder Asp steht.

12. Derivat eines GLP-1-Analogons nach einem der Ansprüche 1-11, wobei Xaa an Position 19 für Tyr, Glu oder Asp steht.

13. Derivat eines GLP-1-Analogons nach einem der Ansprüche 1-12, wobei Xaa an Position 20 für Leu, Glu oder Asp steht.

14. Derivat eines GLP-1-Analogons nach einem der Ansprüche 1-13, wobei Xaa an Position 21 für Glu oder Asp steht.

15. Derivat eines GLP-1-Analogons nach einem der Ansprüche 1-14, wobei Xaa an Position 22 für Gly, Glu oder Asp steht.

16. Derivat eines GLP-1-Analogons nach einem der Ansprüche 1-15, wobei Xaa an Position 23 für Gln, Glu oder Asp steht.

17. Derivat eines GLP-1-Analogons nach einem der Ansprüche 1-16, wobei Xaa an Position 24 für Ala, Glu oder Asp steht.

18. Derivat eines GLP-1-Analogons nach einem der Ansprüche 1-17, wobei Xaa an Position 25 für Ala, Glu oder Asp steht.

19. Derivat eines GLP-1-Analogons nach einem der Ansprüche 1-18, wobei Xaa an Position 26 für Glu, Asp oder Arg steht.

20. Derivat eines GLP-1-Analogons nach einem der Ansprüche 1-19, wobei Xaa an Position 27 für Glu oder Asp steht.

21. Derivat eines GLP-1-Analogons nach einem der Ansprüche 1-20, wobei Xaa an Position 30 für Ala, Glu oder Asp steht.

22. Derivat eines GLP-1-Analogons nach einem der Ansprüche 1-21, wobei Xaa an Position 31 für Trp, Glu oder Asp steht.

23. Derivat eines GLP-1-Analogons nach einem der Ansprüche 1-22, wobei Xaa an Position 32 für Leu, Glu oder Asp steht.

24. Derivat eines GLP-1-Analogons nach einem der Ansprüche 1-23, wobei Xaa an Position 33 für Val, Glu oder Asp steht.

25. Derivat eines GLP-1-Analogons nach einem der Ansprüche 1-24, wobei Xaa an Position 34 für Arg, Glu oder Asp steht.

26. Derivat eines GLP-1-Analogons nach einem der Ansprüche 1-25, wobei Xaa an Position 35 für Gly, Glu oder Asp steht.

27. Derivat eines GLP-1-Analogons nach einem der Ansprüche 1-26, wobei Xaa an Position 36 für Arg, Lys, Glu oder Asp steht.

28. Derivat eines GLP-1-Analogons nach einem der Ansprüche 1-27, wobei Xaa an Position 37 für Gly, Glu, Asp oder Lys steht.

29. Derivat eines GLP-1-Analogons nach einem der Ansprüche 1-31, wobei Xaa an Position 38 für Arg oder Lys steht.

30. Derivat eines GLP-1-Analogons nach einem der vorangehenden Ansprüche, wobei der lipophile Substituent 4 bis 40 Kohlenstoffatome, stärker bevorzugt 8 bis 25 Kohlenstoffatome umfasst.

31. Derivat eines GLP-1-Analogons nach einem der vorangehenden Ansprüche, wobei es sich bei dem Abstandhalter um Glu oder Asp handelt, wobei dessen Carboxygruppe mit einer Aminogruppe des Aminosäurerests eine Amidbindung bildet und die Aminogruppe mit einer Carboxygruppe des lipophilen Substituenten eine Amidbindung bildet.

32. Derivat eines GLP-1-Analogons nach einem der vorangehenden Ansprüche, wobei es sich bei dem Abstandhalter um N-epsilon-(gamma-L-glutamyl) oder N-epsilon-(beta-L-asparagyl)) handelt.

33. Derivat eines GLP-1-Analogons nach Anspruch 34, wobei die ε-Aminogruppe von Lys mit einer Carboxygruppe des Aminosäurerestabstandhalters eine Amidbindung bildet und eine Aminogruppe des Aminosäurerestabstandhalters mit einer Carboxygruppe des lipophilen Substituenten eine Amidbindung bildet.

34. Derivat eines GLP-1-Analogons nach einem der vorangehenden Ansprüche, wobei der lipophile Substituent ein teilweise oder vollständig hydriertes Cyclopentanophenathrengerüst umfasst.

35. Derivat eines GLP-1-Analogons nach einem der Ansprüche 1-33, wobei es sich bei dem lipophilen Substituenten um eine geradkettige oder verzweigte Alkylgruppe handelt.

36. Derivat eines GLP-1-Analogons nach einem der Ansprüche 1-33, wobei es sich bei dem lipophilen Substituenten um die Acylgruppe einer geradkettigen oder verzweigten Fettsäure handelt.

37. Derivat eines GLP-1-Analogons nach Anspruch 36, wobei die Acylgruppe ausgewählt ist aus der Gruppe, umfassend CH₃(CH₂)ₙCO-, wobei n für 4 bis 38 steht, vorzugsweise CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- und CH₃(CH₂)₂₂CO-.

38. Derivat eines GLP-1-Analogons nach einem der Ansprüche 1-33, wobei es sich bei dem lipophilen Substituenten um eine Acylgruppe einer geradkettigen oder verzweigten Alkan-α,ω-dicarbonsäure handelt.

39. Derivat eines GLP-1-Analogons nach Anspruch 38, wobei die Acylgruppe ausgewählt ist aus der Gruppe, umfassend HOOC(CH₂)ₘCO-, wobei m für 4 bis 38, vorzugsweise für 4 bis 24 steht, stärker bevorzugt ausgewählt ist aus der Gruppe, umfassend HOOC(CH₂)₁₄C0-, HOOC(CH₂)₁₆CO-, HOOC(CH₂)₁₈CO-, HOOC(CH₂)₂₀CO- und HOOC(CH₂)₂₂CO-.

40. Derivat eines GLP-1-Analogons nach einem der Ansprüche 1-33, wobei es sich bei dem lipophilen Substituenten um eine Gruppe der Formel CH₃(CH₂)ₚ((CH₂)_{q}COOH)CHNH-CO(CH₂)₂CO- handelt, wobei p und q für ganze Zahlen stehen und p+q für eine ganze Zahl von 8 bis 33, vorzugsweise von 12 bis 28 steht.

41. Derivat eines GLP-1-Analogons nach einem der Ansprüche 1-33, wobei es sich bei dem lipophilen Substituenten um eine Gruppe der Formel CH₃(CH₂)ᵣCO-NHCH(COOH)(CH₂)₂CO- handelt, wobei r für eine ganze Zahl von 10 bis 24 steht.

42. Derivat eines GLP-1-Analogons nach einem der Ansprüche 1-33, wobei es sich bei dem lipophilen Substituenten um eine Gruppe der Formel CH₃(CH₂)ₛCO-NHCH((CH₂)₂COOH)CO- handelt, wobei s für eine ganze Zahl von 8 bis 24 steht.

43. Derivat eines GLP-1-Analogons nach einem der Ansprüche 1-33, wobei es sich bei dem lipophilen Substituenten um eine Gruppe der Formel -NHCH(COOH)(CH₂)₄NH-CO(CH₂)ᵤCH₃ handelt, wobei u für eine ganze Zahl von 8 bis 18 steht.

44. Derivat eines GLP-1-Analogons nach einem der Ansprüche 1-33, wobei es sich bei dem lipophilen Substituenten um eine Gruppe der Formel -NHCH(COOH)(CH₂)₄NH-COCH((CH₂)₂COOH)NH-CO(CH₂)_{w}CH₃ handelt, wobei w für eine ganze Zahl von 10 bis 16 steht.

45. Derivat eines GLP-1-Analogons nach einem der Ansprüche 1-33, wobei es sich bei dem lipophilen Substituenten um eine Gruppe der Formel -NHCH(COOH)(CH₂)₄NH-CO(CH₂)₂CH(COOH)NH-CO(CH₂)ₓCH₃ handelt, wobei x für eine ganze Zahl von 10 bis 16 steht.

46. Derivat eines GLP-1-Analogons nach einem der Ansprüche 1-33, wobei es sich bei dem lipophilen Substituenten um eine Gruppe der Formel -NHCH(COOH)(CH₂)₄NH-CO(CH₂)₂CH(COOH)NH-CO(CH₂)_{y}CH₃ handelt, wobei y für Null oder eine ganze Zahl von 1 bis 22 steht.

47. Arzneimittel, umfassend ein Derivat eines GLP-1-Analogons nach einem der Ansprüche 1-46 und ein pharmazeutisch verträgliches Vehikulum oder einen pharmazeutisch verträglichen Träger.

48. Arzneimittel nach Anspruch 47, des Weiteren umfassend ein weiteres Antidiabetikum.

49. Arzneimittel nach Anspruch 48, wobei es sich bei dem Antidiabetikum um Insulin, stärker bevorzugt Humaninsulin handelt.

50. Arzneimittel nach Anspruch 48, wobei es sich bei dem Antidiabetikum um ein hypoglykämisches Mittel handelt.

51. Verwendung eines Derivats eines GLP-1-Analogons nach Ansprüche 1-46 zur Herstellung eines Medikaments mit einem verzögerten Wirkungsprofil in Bezug auf GLP-1(7-37) zur Behandlung von nicht-insulinabhängiger Diabetes mellitus.

52. Verwendung eines Derivats eines GLP-1-Analogons nach einem der Ansprüche 1-46 zur Herstellung eines Medikaments mit einem verzögerten Wirkungsprofil in Bezug auf GLP-1(7-37) zur Behandlung von insulinabhängiger Diabetes mellitus.

53. Verwendung eines Derivats eines GLP-1-Analogons nach einem der Ansprüche 1-46 zur Herstellung eines Medikaments mit einem verzögerten Wirkungsprofil in Bezug auf GLP-1(7-37) zur Behandlung von Fettsucht.

54. Derivat eines GLP-1-Analogons nach einem der Ansprüche 1-46 zur Verwendung als Medikament.

55. Derivat eines GLP-1-Analogons nach einem der Ansprüche 1-46 zur Herstellung eines Medikaments zur Behandlung von nicht-insulinabhängiger Diabetes mellitus, insulinabhängiger Diabetes mellitus und/oder Fettsucht.

56. Verwendung eines Derivat eines GLP-1-Analogons nach einem der Ansprüche 1-46 zur Herstellung eines Medikaments zur Behandlung von nicht-insulinabhängiger Diabetes mellitus, insulinabhängiger Diabetes mellitus und/oder Fettsucht.

## Revendications

1. Dérivé d'un analogue du GLP-1, lequel dérivé a un profil d'action prolongée par rapport à celui du GLP-1(7-37), lequel analogue du GLP-1 a la formule I : 
7 8 9 10 11 12 13 14 15 16 17
His-Xaa-Xaa-Gly-Xaa-Phe-Thr-Xaa-Asp-Xaa-Xaa-

18 19 20 21 22 23 24 25 26 27 28
Xaa-Xaa-Xaa-Xaa-Xaa-Xaa-Xaa-Xaa-Xaa-Xaa-Phe-

29 30 31 32 33 34 35 36 37 38
Ile-Xaa-Xaa-Xaa-Xaa-Xaa-Xaa-Xaa-Xaa-Xaa

39 40 41 42 43 44 45
Xaa-Xaa-Xaa-Xaa-Xaa-Xaa-Xaa
 dans laquelle
Xaa en position 8 est Ala, Gly, Ser, Thr, Leu, Ile, Val, Glu ou Asp,
Xaa en position 9 est Glu ou Asp,
Xaa en position 11 est Thr, Ala, Gly, Ser, Leu, Ile, Val, Glu ou Asp,
Xaa en position 14 est Ser, Ala, Gly, Thr, Leu, Ile, Val, Glu ou Asp,
Xaa en position 16 est Val, Ala, Gly, Ser, Thr, Leu, Ile, Tyr, Glu ou Asp,
Xaa en position 17 est Ser, Ala, Gly, Thr, Leu, Ile, Val, Glu ou Asp,
Xaa en position 18 est Ser, Ala, Gly, Thr, Leu, Ile, Val, Glu ou Asp,
Xaa en position 19 est Tyr, Phe, Trp, Glu ou Asp,
Xaa en position 20 est Leu, Ala, Gly, Ser, Thr, Leu, Ile, Val, Glu ou Asp,
Xaa en position 21 est Glu ou Asp,
Xaa en position 22 est Gly, Ala, Ser, Thr, Leu, Ile, Val, Glu ou Asp,
Xaa en position 23 est Gln, Asn, Arg, Glu ou Asp,
Xaa en position 24 est Ala, Gly, Ser, Thr, Leu, Ile, Val, Arg, Glu ou Asp,
Xaa en position 25 est Ala, Gly, Ser, Thr, Leu, Ile, Val, Glu ou Asp,
Xaa en position 26 est Arg, Gln, Glu, Asp ou His,
Xaa en position 27 est Glu ou Asp,
Xaa en position 30 est Ala, Gly, Ser, Thr, Leu, Ile, Val, Glu ou Asp,
Xaa en position 31 est Trp, Phe, Tyr, Glu ou Asp,
Xaa en position 32 est Leu, Gly, Ala, Ser, Thr, Ile, Val, Glu ou Asp,
Xaa en position 33 est Val, Gly, Ala, Ser, Thr, Leu, Ile, Glu ou Asp,
Xaa en position 34 est Arg, Glu, Asp ou His,
Xaa en position 35 est Gly, Ala, Ser, Thr, Leu, Ile, Val, Glu ou Asp,
Xaa en position 36 est Arg, Lys, Glu, Asp ou His,
Xaa en position 37 est Gly, Ala, Ser, Thr, Leu, Ile, Val, Glu, Asp ou Lys, ou est délété,
Xaa en position 38 est Arg, Lys, Glu, Asp ou His, ou est délété,
Xaa en position 39 est Arg, Lys, Glu, Asp ou His, ou est délété,
Xaa en position 40 est Asp, Glu ou Lys, ou est délété,
Xaa en position 41 est Phe, Trp, Tyr, Glu, Asp ou Lys, ou est délété,
Xaa en position 42 est Pro, Lys, Glu ou Asp, ou est délété,
Xaa en position 43 est Glu, Asp ou Lys, ou est délété,
Xaa en position 44 est Glu, Asp ou Lys, ou est délété, et
Xaa en position 45 est Val, Glu, Asp ou Lys, ou est délété, ou
(a) l'un de ses esters en C₁₋₆,
(b) l' un de ses amides, alkylamides en C₁₋₆ ou di (alkyle en C₁₋₆) amides, et/ou
(c) l'un de ses sels acceptables d'un point de vue pharmaceutique,
à la condition que :
A. quand l'acide aminé en position 37, 38, 39, 40, 41, 42, 43 ou 44 est délété, alors chaque acide aminé en aval de l'acide aminé soit lui aussi délété,
B. le dérivé de l'analogue du GLP-1 ne contienne qu'un Lys sur le site carboxy-terminal, et que Glu ou Asp soit adjacent à Lys,
C. le groupe ε-amino de Lys soit substitué par un substituant lipophile par l'intermédiaire d'un espaceur,
D. le nombre total d'acides aminés différents entre le dérivé de l'analogue du GLP-1 et la forme native correspondante du GLP-1 ne dépasse pas six,
E. le dérivé de l'analogue du GLP-1 de formule I ne soit pas choisi parmI :
Glu^{22,23,30}Arg^{26,34}Lys³⁸(N^{ε}-(γ-glutamyl(N^{α}-tétradécanoyl)))-GLP-1(7-38)-OH,
Glu^{23,26}Arg³⁴Lys³⁸(N^{ε}-(γ-glutamyl(N^{α}-tétradécanoyl)))-GLP-1(7-38)-OH,
Arg^{26,34}Lys³⁸(N^{ε}-(γ-glutamyl(N^{α}-tétradécanoyl)))-GLP-1(7-38)-OH,
Arg^{26,34}Lys³⁸(N^{ε}-(γ-glutamyl(N^{α}-hexadécanoyl)))-GLP-1(7-38)-OH,
Arg^{26,34}Lys³⁸(N^{ε}-(γ-glutamyl(N^{α}-octadécanoyl)))-GLP-1(7-38)-OH.

2. Dérivé de l'analogue du GLP-1 selon la revendication 1, dans lequel les acides aminés sur les positions 37-35 sont absents.

3. Dérivé de l'analogue du GLP-1 selon la revendication 1, dans lequel les acides aminés sur les positions 38-45 sont absents.

4. Dérivé de l'analogue du GLP-1 selon la revendication 1, dans lequel les acides aminés sur les positions 39-45 sont absents.

5. Dérivé de l'analogue du GLP-1 selon l'une quelconque des revendications 1 à 4, dans lequel Xaa en position 8 est Ala, Gly, Ser, Thr ou Val.

6. Dérivé de l'analogue du GLP-1 selon l'une quelconque des revendications 1 à 5, dans lequel Xaa en position 9 est Glu.

7. Dérivé de l'analogue du GLP-1 selon l'une quelconque des revendications 1 à 6, dans lequel Xaa en position 11 est Thr.

8. Dérivé de l'analogue du GLP-1 selon l'une quelconque des revendications 1 à 7, dans lequel Xaa en position 14 est Ser.

9. Dérivé de l'analogue du GLP-1 selon l'une quelconque des revendications 1 à 8, dans lequel Xaa en position 16 est Val.

10. Dérivé de l'analogue du GLP-1 selon l'une quelconque des revendications 1 à 9, dans lequel Xaa en position 17 est Ser.

11. Dérivé de l'analogue du GLP-1 selon l'une quelconque des revendications 1 à 10, dans lequel Xaa en position 18 est Ser, Glu ou Asp.

12. Dérivé de l'analogue du GLP-1 selon l'une quelconque des revendications 1 à 11, dans lequel Xaa en position 19 est Tyr, Glu ou Asp.

13. Dérivé de l'analogue du GLP-1 selon l'une quelconque des revendications 1 à 12, dans lequel Xaa en position 20 est Leu, Glu ou Asp.

14. Dérivé de l'analogue du GLP-1 selon l'une quelconque des revendications 1 à 13, dans lequel Xaa en position 21 est Glu ou Asp.

15. Dérivé de l'analogue du GLP-1 selon l'une quelconque des revendications 1 à 14, dans lequel Xaa en position 22 est Gly, Glu ou Asp.

16. Dérivé de l'analogue du GLP-1 selon l'une quelconque des revendications 1 à 15, dans lequel Xaa en position 23 est Gln, Glu ou Asp.

17. Dérivé de l'analogue du GLP-1 selon l'une quelconque des revendications 1 à 16, dans lequel Xaa en position 24 est Ala, Glu ou Asp.

18. Dérivé de l'analogue du GLP-1 selon l'une quelconque des revendications 1 à 17, dans lequel Xaa en position 25 est Ala, Glu ou Asp.

19. Dérivé de l'analogue du GLP-1 selon l'une quelconque des revendications 1 à 18, dans lequel Xaa en position 26 est Glu, Asp ou Arg.

20. Dérivé de l'analogue du GLP-1 selon l'une quelconque des revendications 1 à 19, dans lequel Xaa en position 27 est Glu ou Asp.

21. Dérivé de l'analogue du GLP-1 selon l'une quelconque des revendications 1 à 20, dans lequel Xaa en position 30 est Ala, Glu ou Asp.

22. Dérivé de l'analogue du GLP-1 selon l'une quelconque des revendications 1 à 21, dans lequel Xaa en position 31 est Trp, Glu ou Asp.

23. Dérivé de l'analogue du GLP-1 selon l'une quelconque des revendications 1 à 22, dans lequel Xaa en position 32 est Leu, Glu ou Asp.

24. Dérivé de l'analogue du GLP-1 selon l'une quelconque des revendications 1 à 23, dans lequel Xaa en position 33 est Val, Glu ou Asp.

25. Dérivé de l'analogue du GLP-1 selon l'une quelconque des revendications 1 à 24, dans lequel Xaa en position 34 est Arg, Glu ou Asp.

26. Dérivé de l'analogue du GLP-1 selon l'une quelconque des revendications 1 à 25, dans lequel Xaa en position 35 est Gly, Glu ou Asp.

27. Dérivé de l'analogue du GLP-1 selon l'une quelconque des revendications 1 à 26, dans lequel Xaa en position 36 est Arg, Lys, Glu ou Asp.

28. Dérivé de l'analogue du GLP-1 selon l'une quelconque des revendications 1 à 27, dans lequel Xaa en position 37 est Gly, Glu, Asp ou Lys.

29. Dérivé de l'analogue du GLP-1 selon l'une quelconque des revendications 1 à 31, dans lequel Xaa en position 38 est Arg ou Lys.

30. Dérivé de l'analogue du GLP-1 selon l'une quelconque des revendications précédentes, dans lequel le substituant lipophile comprend de 4 à 40 atomes de carbone, d'une manière plus préférée de 8 à 25 atomes de carbone.

31. Dérivé de l'analogue du GLP-1 selon l'une quelconque des revendications précédentes, dans lequel l'espaceur est Glu ou Asp, dont le groupe carboxyle forme une liaison amide avec un groupe amino du résidu d'acide aminé, et dont le groupe amino forme une liaison amide avec un groupe carboxyle du substituant lipophile.

32. Dérivé de l'analogue du GLP-1 selon l'une quelconque des revendications précédentes, dans lequel l'espaceur est le groupe N-epsilon-(gamma-L-glutamyle) ou N-epsilon-(bêta-L-asparagyle).

33. Dérivé de l'analogue du GLP-1 selon la revendication 34, dans lequel le groupe ε-amino de Lys forme une liaison amide avec un groupe carboxylique de l'espaceur résidu d'acide aminé, et un groupe amino de l'espaceur résidu d'acide aminé forme une liaison amide avec un groupe carboxyle du substituant lipophile.

34. Dérivé de l'analogue du GLP-1 selon l'une quelconque des revendications précédentes, dans lequel le substituant lipophile comprend un squelette cyclopentanophénanthrène partiellement ou entièrement hydrogéné.

35. Dérivé de l'analogue du GLP-1 selon l'une quelconque des revendications 1 à 33, dans lequel le substituant lipophile est un groupe alkyle à chaîne droite ou ramifiée.

36. Dérivé de l'analogue du GLP-1 selon l'une quelconque des revendications 1 à 33, dans lequel le substituant lipophile est le groupe acyle d'un acide gras à chaîne droite ou ramifiée.

37. Dérivé de l'analogue du GLP-1 selon la revendication 36, dans lequel le groupe acyle est choisi dans le groupe comprenant CH₃(CH₂)ₙCO-, où n vaut 4 à 38, de préférence CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- et CH₃(CH₂)₂₂CO-.

38. Dérivé de l'analogue du GLP-1 selon l'une quelconque des revendications 1 à 33, dans lequel le substituant lipophile est un groupe acyle d'un acide (alcane à chaîne droite ou ramifiée)-α,ω-dicarboxylique.

39. Dérivé de l'analogue du GLP-1 selon la revendication 38, dans lequel le groupe acyle est choisi dans le groupe comprenant HOOC(CH₂)ₘCO-, dans lequel m vaut 4 à 38, de préférence 4 à 24, d'une manière plus préférée choisi dans le groupe comprenant HOOC(CH₂)₁₄CO-, HOOC(CH₂)₁₆CO-, HOOC(CH₂)₁₈CO-, HOOC (CH₂)₂₀CO- et HOOC(CH₂)₂₂CO-.

40. Dérivé de l'analogue du GLP-1 selon l'une quelconque des revendications 1 à 33, dans lequel le substituant lipophile est un groupe de formule CH₃(CH₂)ₚ((CH₂)_{q}COOH)CHNH-CO(CH₂)₂CO-, où p et q sont des entiers et p+q est un entier de 8 à 33, de préférence de 12 à 28.

41. Dérivé de l'analogue du GLP-1 selon l'une quelconque des revendications 1 à 33, dans lequel le substituant lipophile est un groupe de formule CH₃(CH₂)ᵣCO-NHCH(COOH)(CH₂)₂CO-, où r est un entier de 10 à 24.

42. Dérivé de l'analogue du GLP-1 selon l'une quelconque des revendications 1 à 33, dans lequel le substituant lipophile est un groupe de formule CH₃(CH₂)ₛCO-NHCH((CH₂)₂COOH)CO-, où s est un entier de 8 à 24.

43. Dérivé de l'analogue du GLP-1 selon l'une quelconque des revendications 1 à 33, dans lequel le substituant lipophile est un groupe de formule -NHCH(COOH)(CH₂)₄NH-CO(CH₂)ᵤCH₃, où u est un entier de 8 à 18.

44. Dérivé de l'analogue du GLP-1 selon l'une quelconque des revendications 1 à 33, dans lequel le substituant lipophile est un groupe de formule -NHCH(COOH)(CH₂)₄NH-COCH((CH₂)₂COOH)NH-CO(CH₂)_{w}CH₃, où w est un entier de 10 à 16.

45. Dérivé de l'analogue du GLP-1 selon l'une quelconque des revendications 1 à 33, dans lequel le substituant lipophile est un groupe de formule -NHCH(COOH) (CH₂)₄NH-CO(CH₂)₂CH(COOH)NH-CO(CH₂)ₓCH₃, où x est un entier de 10 à 16.

46. Dérivé de l'analogue du GLP-1 selon l'une quelconque des revendications 1 à 33, dans lequel le substituant lipophile est un groupe de formule -NHCH (COOH) (CH₂) ₄NH-CO (CH₂) ₂CH (COOH) NH-CO (CH₂) _{y}CH₃, où y vaut zéro ou est un entier de 1 à 22.

47. Composition pharmaceutique comprenant un dérivé de l'analogue du GLP-1 selon l'une quelconque des revendications 1 à 46 et un véhicule ou un excipient acceptable d'un point de vue pharmaceutique.

48. Composition pharmaceutique selon la revendication 47, qui comprend en outre un autre agent anti-diabétique.

49. Composition pharmaceutique selon la revendication 48, dans laquelle l'agent anti-diabétique est une insuline, d'une manière plus préférée l'insuline humaine.

50. Composition pharmaceutique selon la revendication 48, dans laquelle l'agent anti-diabétique est un agent hypoglycémiant.

51. Utilisation d'un dérivé de l'analogue du GLP-1 selon l'une quelconque des revendications 1 à 46 pour la préparation d'un médicament ayant un profil d'action prolongé par rapport à celui du GLP-1(7-37), pour le traitement du diabète sucré non insulino-dépendant.

52. Utilisation d'un dérivé de l'analogue du GLP-1 selon l'une quelconque des revendications 1 à 46 pour la préparation d'un médicament ayant un profil d'action prolongé par rapport à celui du GLP-1(7-37), pour le traitement du diabète sucré insulino-dépendant.

53. Utilisation d'un dérivé de l'analogue du GLP-1 selon l'une quelconque des revendications 1 à 34 pour la préparation d'un médicament ayant un profil d'action prolongé par rapport à celui du GLP-1(7-37), pour le traitement de l'obésité.

54. Dérivé du GLP-1 selon l'une quelconque des revendications 1 à 46 pour utilisation en tant que médicament.

55. Dérivé du GLP-1 selon l'une quelconque des revendications 1 à 46 pour utilisation en tant que médicament dans le traitement du diabète sucré non insulino-dépendant, du diabète sucré insulino-dépendant et/ou de l'obésité.

56. Utilisation d'un dérivé du GLP-1 selon l'une quelconque des revendications 1 à 46 pour la préparation d'un médicament pour le traitement du diabète sucré non insulino-dépendant, du diabète sucré insulino-dépendant et/ou de l'obésité.
